# EUROPEAN PATENT SPECIFICATION

(11) **EP 2 470 204 B1**
(45) Date of publication and mention of the grant of the patent: **16.12.2015**
(21) Application number: 10765842.9
(22) Date of filing: 27.08.2010
(51) Int. Cl.: A61K 39/095

(54) **HYBRID POLYPEPTIDES INCLUDING MENINGOCOCCAL FHBP SEQUENCES**
HYBRIDPOLYPEPTIDE MIT MENINGOKOKKEN-FHBP-SEQUENZEN
POLYPEPTIDES HYBRIDES CONTENANT DES SÉQUENCES FHBP À MÉNINGOCOQUES

(30) Priority: 27.08.2009 US 237576 P
(43) Date of publication of application: 04.07.2012
(60) Divisional application: 15193267.0
(73) Proprietor: GlaxoSmithKline Biologicals SA, 1330 Rixensart (BE)
(72) Inventor: PIZZA, Mariagrazia, 53100 Siena (IT); SCARSELLI, Maria, 53100 Siena (IT); RAPPUOLI, Rino, 53100 Siena (IT); GIULIANI, Marzia Monica, 53100 Siena (IT); ARICO, Maria, 53100 Siena (IT)
(74) Representative: Evans, Stephen John Eves
(86) International application number: PCT/IB2010/002260
(87) International publication number: WO 2011/024072

(56) References cited:
- WO-A1-2004/032958
- WO-A2-2004/048404
- WO-A2-2007/060548
- BEERNINK PETER T ET AL: "Bactericidal antibody responses induced by meningococcal recombinant chimeric factor H-binding protein vaccines.", INFECTION AND IMMUNITY JUN 2008 LNKD- PUBMED:18362128, vol. 76, no. 6, June 2008 (2008-06), pages 2568-2575, XP002617167, ISSN: 1098-5522

## Description

### TECHNICAL FIELD

This invention is in the field of immunisation and, in particular, immunisation against diseases caused by pathogenic bacteria in the genus *Neisseria,* such as *N.meningitidis* (meningococcus).

### BACKGROUND ART

*Neisseria meningitidis* is a Gram-negative encapsulated bacterial pathogen. Although polysaccharide and conjugate vaccines are available against serogroups A, C, W 135 and Y, this approach cannot be applied to serogroup B because the capsular polysaccharide is a polymer of polysialic acid, which is a self antigen in humans. To develop a vaccine against serogroup B, outer membrane vesicles (OMVs) have been used. These vaccines elicit serum bactericidal antibody responses and protect against disease, but they fail to induce cross-strain protection [1]. Some workers are therefore focusing on specific meningococcal antigens for use in vaccines [2].

One such antigen is the meningococcal factor H binding protein (fHBP), also known as protein '741' [SEQ IDs 2535 & 2536 in ref. 3; SEQ ID 1 herein], 'NMB1870', 'GNA1870' [refs. 4-6, following ref. 2], 'P2086', 'LP2086' or 'ORF2086' [7-9]. This lipoprotein is expressed across all meningococcal serogroups and has been found in multiple strains. fHBP sequences have been grouped into three families [4] (referred to herein as families I, II & III), and serum raised against a given family is bactericidal within the same family, but is not active against strains which express one of the other families *i.e.* there is intra-family, but not inter-family, cross-protection.

To achieve cross-strain protection using fHBP, therefore, more than one family is used. To avoid the need to express and purify separate proteins, it has been proposed to express different families as hybrid proteins [10-12], including two or three of the families in a single polypeptide chain. References 13 and 14 describe various mutagenesis-based approaches for modifying fHBP sequences to increase their coverage across families I, II and III. Reference 15 describes various further forms of fHBP which are modified to improve their inter-family coverage.

It is an object of the invention to provide further and improved approaches for overcoming the family specificity of protection afforded by fHBP, and to use these approaches for providing immunity against meningococcal disease and/or infection, particularly for serogroup B.

### DISCLOSURE OF THE INVENTION

Full-length fHBP has the following amino acid sequence (SEQ ID NO: 1) in strain MC58:

MNRTAFCCLSLTTALILTACSSGGGGVAADIGAGLADALTAPLDHKDKGLQSLTLDQSVRKNEKLK
LAAQGAEKTYGNGDSLNTGKLKNDKVSRFDFIRQIEVDGQLITLESGEFQVYKQSHSALTAFQTEQ
IQDSEHSGKMVAKRQFRIGDIAGEHTSFDKLPEGGRATYRGTAFGSDDAGGKLTYTIDFAAKQGNG
KIEHLKSPELNVDLAAADIKPDGKRHAVISGSVLYNQAEKGSYSLGIFGGKAQEVAGSAEVKTVNG
IRHIGLAAKQ

The mature lipoprotein lacks the first 19 amino acids of SEQ ID NO: 1, and the ΔG form of fHBP lacks the first 26 amino acids.

The MC58 sequence (SEQ ID NO: 1) is in fHBP family I. Antibodies elicited using the MC58 sequence have high bactericidal activity against the MC58 strain, but much lower activity against strains that express a family II or III fHBP. In some embodiments the invention relates to modified forms of fHBP, wherein the modification(s) improve the ability of the protein to elicit cross-family bactericidal antibodies. In other embodiments the invention relates to fusion proteins in which a modified form of fHBP is fused to a second amino acid sequence *e.g.* to another meningococcal immunogen or to another fHBP (including a modified fHBP).

The invention provides a polypeptide comprising a first immunogenic amino acid sequence and a second immunogenic amino acid sequence, wherein the first amino acid sequence is SEQ ID NO 23. In some embodiments, first and second amino acid sequences may be the same; in other embodiments, they are different from each other. Suitable second immunogenic amino acid sequences are described in more detail below and include, but are not limited to: (a) non-meningococcal antigens; (b) meningococcal non-fHBP antigens; (c) wild-type meningococcal fHBP antigens; and (d) modified meningococcal fHBP antigens, which may be the same as or different from the first immunogenic amino acid sequence. The first and second sequences may be arranged in either order from N-terminus to C-terminus.

Thus the invention provides a polypeptide comprising an amino acid sequence selected from the group consisting of SEQ ID NOs: 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137 and 138.

These various polypeptides have the ability to induce bactericidal anti-meningococcal antibodies after administration to a host animal, and in preferred embodiments can induce antibodies that are bactericidal against strains in each of the three fHBP families I to III. Further information on bactericidal responses is given below.

### Second immunogenic amino acid sequences

A polypeptide of the invention includes a second immunogenic amino acid sequence. Various such second sequences can be used.

The second immunogenic amino acid sequence may comprise a non-meningococcal antigen. This will preferably be from a non-meningococcal pathogen, such as a bacterium or virus. For instance, the second sequence might comprise an immunogenic pneumococcal amino acid sequence or an immunogenic hepatitis virus amino acid sequence.

The second immunogenic amino acid sequence may comprise a meningococcal antigen, other than a fHBP antigen. For instance, the second sequence might comprise a sequence for meningococcal antigen 287, NadA, NspA, HmbR, NhhA, App, 936 or Omp85. Further details of these second sequences are given below. Examples of polypeptide sequences including such second immunogenic sequences are SEQ ID NOs: 102, 124, 125, 126, 127, 128 and 129.

The second immunogenic amino acid sequence may comprise a wild-type or modified fHBP sequence. This second amino acid sequence can preferably elicit, when administered to a subject as part of a polypeptide of the invention, antibody response comprising antibodies that bind to the wild-type meningococcus protein having one of amino acid sequences SEQ ID NOs: 1, 2 or 3. For instance, the second amino acid sequence may comprise any of:
- A sequence selected from SEQ ID NOs: 1, 2, and 3 (wild-type fHBP sequences).
- A sequence selected from SEQ ID NOs 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77 and 78 (modified fHBP sequences). In some such embodiments, the second immunogenic sequence is identical to the first immunogenic sequence.
- An amino acid sequence having at least x% sequence identity to any one of SEQ ID NOs: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77 and 78.

The value of x is at least 80 *e.g.* 82, 84, 86, 88, 90, 92, 94, 95, 96, 97, 98, 99 or more.

Examples of polypeptides including such fHBP sequences as the second immunogenic sequence are SEQ ID NOs: 99, 100, 101, 103, 104, 105, 106, 107, 108, 109, 110, 111, 112, 113, 114, 115, 116, 117, 118, 119, 120, 121, 122, 123, 130, 131, 132, 133, 134, 135, 136, 137 and 138.

The first and second immunogenic amino acid sequences may be joined directly via a peptide bond, such that (i) the C-terminus amino acid of the first immunogenic amino acid sequence is directly upstream of the N-terminus amino acid of the second immunogenic amino acid sequence, or (ii) the C-terminus amino acid of the second immunogenic amino acid sequence is directly upstream of the N-terminus amino acid of the first immunogenic amino acid sequence, In other embodiments, however, the first and second immunogenic amino acid sequences are separated by a linker amino acid sequence, while still forming a single translated polypeptide chain. Such linker amino acid sequence(s) -L- will typically be short *(e.g.* 20 or fewer amino acids *i.e.* 20, 19, 18, 17, 16, 15, 14, 13, 12, 11, 10, 9, 8, 7, 6, 5, 4, 3, 2, 1). Examples comprise short peptide sequences which facilitate cloning, poly-glycine linkers *(i.e.* comprising Gly*ₙ* where *n* = 2, 3, 4, 5, 6, 7, 8, 9, 10 or more), and histidine tags (*i.e.* His*ₙ* where *n* = 3, 4, 5, 6, 7, 8, 9, 10 or more e.g. SEQ ID NO: 95). Other suitable linker amino acid sequences will be apparent to those skilled in the art. A useful linker is GSGGGG (SEQ ID NO: 81) or GSGSGGGG (SEQ ID NO: 82), with the Gly-Ser dipeptide being formed from a *Bam*HI restriction site, thus aiding cloning and manipulation, and the (Gly)₄ tetrapeptide being a typical poly-glycine linker. Other suitable linkers, are ASGGGS (SEQ ID NO: 93 *e.g.* encoded by SEQ ID NO: 94) or a Leu-Glu dipeptide.

More than one of these second sequences may be present, thereby providing third, fourth, fifth, *etc.,* immunogenic sequences in the polypeptide. Such polypeptides include those comprising a first immunogenic amino acid sequence, a second immunogenic amino acid sequence and a third immunogenic amino acid sequence, wherein: the first and second amino acid sequences are as defined above; and the third amino acid sequence is selected from the group consisting of SEQ ID NOs 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77 and 78. The first and third sequences may be the same as or different from each other; the second sequence may be the same as the first or as the third, or may differ from both. Examples of polypeptides including first, second and third sequences are SEQ ID NOs: 99, 100, 101, 103, 104, 105, 106, 107, 108, 109, 110, 111, 112, 113, 114, 115, 116, 117, 118, 119, 120, 121, 122, 123, 124, 125, 126 and 127.

One useful group of polypeptides comprises (i) two amino acid sequences independently selected from the group consisting of SEQ ID NOs 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77 and 78, and (ii) a meningococcal non-fHBP antigen. Examples of such polypeptides include SEQ ID NOs: 124, 125, 126, 127, 140, 141 and 142. The two amino acid sequences of part (i) may be the same or different. The non-fHBP antigen may be between the two amino acid sequences of part (i), to the C-terminus of the two amino acid sequences of part (i), or to the N-terminus of the two amino acid sequences of part (i).

### Non-fHBP meningococcal antigens

A composition of the invention may include a 287 antigen. The 287 antigen was included in the published genome sequence for meningococcal serogroup B strain MC58 [16] as gene NMB2132 (GenBank accession number GI:7227388; SEQ ID NO: 83 herein). The sequences of 287 antigen from many strains have been published since then. For example, allelic forms of 287 can be seen in Figures 5 and 15 of reference 17, and in example 13 and figure 21 of reference 18 (SEQ IDs 3179 to 3184 therein). Various immunogenic fragments of the 287 antigen have also been reported. Preferred 287 antigens for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 83; and/or (b) comprising a fragment of at least 'n' consecutive amino acids of SEQ ID NO: 83, wherein 'n' is 7 or more (*e.g.* 8, 10, 12, 14, 16, 18, 20, 25, 30, 35, 40, 50, 60, 70, 80, 90, 100, 150, 200, 250 or more). Preferred fragments of (b) comprise an epitope from SEQ ID NO: 83. The most useful 287 antigens can elicit antibodies which, after administration to a subject, can bind to a meningococcal polypeptide consisting of amino acid sequence SEQ ID NO: 83. Advantageous 287 antigens for use with the invention can elicit bactericidal anti-meningococcal antibodies after administration to a subject.

A composition of the invention may include a NadA antigen. The NadA antigen was included in the published genome sequence for meningococcal serogroup B strain MC58 [16] as gene NMB1994 (GenBank accession number GI:7227256; SEQ ID NO: 84 herein). The sequences of NadA antigen from many strains have been published since then, and the protein's activity as a Neisserial adhesin has been well documented. Various immunogenic fragments of NadA have also been reported. Preferred NadA antigens for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 84; and/or (b) comprising a fragment of at least 'n' consecutive amino acids of SEQ ID NO: 84, wherein 'n' is 7 or more (*e.g.* 8, 10, 12, 14, 16, 18, 20, 25, 30, 35, 40, 50, 60, 70, 80, 90, 100, 150, 200, 250 or more). Preferred fragments of (b) comprise an epitope from SEQ ID NO: 84. The most useful NadA antigens can elicit antibodies which, after administration to a subject, can bind to a meningococcal polypeptide consisting of amino acid sequence SEQ ID NO: 84. Advantageous NadA antigens for use with the invention can elicit bactericidal anti-meningococcal antibodies after administration to a subject. SEQ ID NO: 6 is one such fragment.

A composition of the invention may include a NspA antigen. The NspA antigen was included in the published genome sequence for meningococcal serogroup B strain MC58 [16] as gene NMB0663 (GenBank accession number GI:7225888; SEQ ID NO: 85 herein). The antigen was previously known from references 19 & 20. The sequences of NspA antigen from many strains have been published since then. Various immunogenic fragments of NspA have also been reported. Preferred NspA antigens for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 85; and/or (b) comprising a fragment of at least 'n' consecutive amino acids of SEQ ID NO: 85, wherein 'n' is 7 or more (*e.g.* 8, 10, 12, 14, 16, 18, 20, 25, 30, 35, 40, 50, 60, 70, 80, 90, 100, 150, 200, 250 or more). Preferred fragments of (b) comprise an epitope from SEQ ID NO: 85. The most useful NspA antigens can elicit antibodies which, after administration to a subject, can bind to a meningococcal polypeptide consisting of amino acid sequence SEQ ID NO: 85. Advantageous NspA antigens for use with the invention can elicit bactericidal anti-meningococcal antibodies after administration to a subject.

Compositions of the invention may include a meningococcal HmbR antigen. The full-length HmbR sequence was included in the published genome sequence for meningococcal serogroup B strain MC58 [16] as gene NMB1668 (SEQ ID NO: 86 herein). The invention can use a polypeptide that comprises a full-length HmbR sequence, but it will often use a polypeptide that comprises a partial HmbR sequence. Thus in some embodiments a HmbR sequence used according to the invention may comprise an amino acid sequence having at least *i*% sequence identity to SEQ ID NO: 86, where the value of *i* is 50, 60, 70, 80, 90, 95, 99 or more. In other embodiments a HmbR sequence used according to the invention may comprise a fragment of at least *j* consecutive amino acids from SEQ ID NO: 86, where the value of *j* is 7, 8, 10, 12, 14, 16, 18, 20, 25, 30, 35, 40, 50, 60, 70, 80, 90, 100, 150, 200, 250 or more. In other embodiments a HmbR sequence used according to the invention may comprise an amino acid sequence (i) having at least *i*% sequence identity to SEQ ID NO: 86 and/or (ii) comprising a fragment of at least *j* consecutive amino acids from SEQ ID NO: 86. Preferred fragments of *j* amino acids comprise an epitope from SEQ ID NO: 86. Such epitopes will usually comprise amino acids that are located on the surface of HmbR. Useful epitopes include those with amino acids involved in HmbR's binding to haemoglobin, as antibodies that bind to these epitopes can block the ability of a bacterium to bind to host haemoglobin. The topology of HmbR, and its critical functional residues, were investigated in reference 21. The most useful HmbR antigens can elicit antibodies which, after administration to a subject, can bind to a meningococcal polypeptide consisting of amino acid sequence SEQ ID NO: 86. Advantageous HmbR antigens for use with the invention can elicit bactericidal anti-meningococcal antibodies after administration to a subject.

A composition of the invention may include a NhhA antigen. The NhhA antigen was included in the published genome sequence for meningococcal serogroup B strain MC58 [16] as gene NMB0992 (GenBank accession number GI:7226232; SEQ ID NO: 87 herein). The sequences of NhhA antigen from many strains have been published since *e.g.* refs 17 & 22, and various immunogenic fragments of NhhA have been reported. It is also known as Hsf. Preferred NhhA antigens for use with the invention comprise an amino acid sequence: (a) having 50% or more identity *(e.g.* 60%, 65%, 70%, 75%, 80%, 85%, 90%, 91%, 92%, 93%, 94%, 95%, 96%, 97%, 98%, 99%, 99.5% or more) to SEQ ID NO: 87; and/or (b) comprising a fragment of at least 'n' consecutive amino acids of SEQ ID NO: 87, wherein 'n' is 7 or more (*e.g*. 8, 10, 12, 14, 16, 18, 20, 25, 30, 35, 40, 50, 60, 70, 80, 90, 100, 150, 200, 250 or more). Preferred fragments of (b) comprise an epitope from SEQ ID NO: 87. The most useful NhhA antigens can elicit antibodies which, after administration to a subject, can bind to a meningococcal polypeptide consisting of amino acid sequence SEQ ID NO: 87. Advantageous NhhA antigens for use with the invention can elicit bactericidal anti-meningococcal antibodies after administration to a subject.

A composition of the invention may include an App antigen. The App antigen was included in the published genome sequence for meningococcal serogroup B strain MC58 [16] as gene NMB1985 (GenBank accession number GI:7227246; SEQ ID NO: 88 herein). The sequences of App antigen from many strains have been published since then. Various immunogenic fragments of App have also been reported. Preferred App antigens for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 88; and/or (b) comprising a fragment of at least 'n' consecutive amino acids of SEQ ID NO: 88, wherein 'n' is 7 or more (*e.g.* 8, 10, 12, 14, 16, 18, 20, 25, 30, 35, 40, 50, 60, 70, 80, 90, 100, 150, 200, 250 or more). Preferred fragments of (b) comprise an epitope from SEQ ID NO: 88. The most useful App antigens can elicit antibodies which, after administration to a subject, can bind to a meningococcal polypeptide consisting of amino acid sequence SEQ ID NO: 88. Advantageous App antigens for use with the invention can elicit bactericidal anti-meningococcal antibodies after administration to a subject.

A composition of the invention may include an Omp85 antigen. The Omp85 antigen was included in the published genome sequence for meningococcal serogroup B strain MC58 [16] as gene NMB0182 (GenBank accession number GI:7225401; SEQ ID NO: 89 herein). The sequences of Omp85 antigen from many strains have been published since then. Further information on Omp85 can be found in references 23 and 24. Various immunogenic fragments of Omp85 have also been reported. Preferred Omp85 antigens for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 89; and/or (b) comprising a fragment of at least 'n' consecutive amino acids of SEQ ID NO: 89, wherein 'n' is 7 or more (*e.g.* 8, 10, 12, 14, 16, 18, 20, 25, 30, 35, 40, 50, 60, 70, 80, 90, 100, 150, 200, 250 or more). Preferred fragments of (b) comprise an epitope from SEQ ID NO: 89. The most useful Omp85 antigens can elicit antibodies which, after administration to a subject, can bind to a meningococcal polypeptide consisting of amino acid sequence SEQ ID NO: 89. Advantageous Omp85 antigens for use with the invention can elicit bactericidal anti-meningococcal antibodies after administration to a subject.

A composition of the invention may include a 936 antigen. The 936 antigen was included in the published genome sequence for meningococcal serogroup B strain MC58 [25] as gene NMB2091 (SEQ ID NO: 98 herein). Preferred 936 antigens for use with the invention comprise an amino acid sequence: (a) having 50% or more identity (*e.g.* 60%, 65%, 70%, 75%, 80%, 85%, 90%, 91%, 92%, 93%, 94%, 95%, 96%, 97%, 98%, 99%, 99.5% or more) to SEQ ID NO: 98; and/or (b) comprising a fragment of at least 'n' consecutive amino acids of SEQ ID NO: 98, wherein 'n' is 7 or more (*e.g.* 8, 10, 12, 14, 16, 18, 20, 25, 30, 35, 40, 50, 60, 70, 80, 90, 100, 150, 200, 250 or more). Preferred fragments of (b) comprise an epitope from SEQ ID NO: 98. The most useful 936 antigens can elicit antibodies which, after administration to a subject, can bind to a meningococcal polypeptide consisting of amino acid sequence SEQ ID NO: 98. The 936 antigen is a good fusion partner for fHBP *(e.g.* see references 108 & 109).

### Polypeptides

Polypeptides of the invention can be prepared by various means e.g. by chemical synthesis (at least in part), by digesting longer polypeptides using proteases, by translation from RNA, by purification from cell culture *(e.g.* from recombinant expression or from *N.meningitidis* culture). *etc.* Heterologous expression in an *E.coli* host is a preferred expression route.

fHBP is naturally a lipoprotein in *N.meningitidis.* It has also been found to be lipidated when expressed in *E.coli* with its native leader sequence. Polypeptides of the invention may have an N-terminus cysteine residue, which may be lipidated *e.g.* comprising a palmitoyl group, usually forming tripalmitoyl-S-glyceryl-cysteine. In other embodiments the polypeptides are not lipidated.

A characteristic of preferred polypeptides of the invention is the ability to induce bactericidal anti-meningococcal antibodies after administration to a host animal.

Polypeptides are preferably prepared in substantially pure or substantially isolated form (*i.e.* substantially free from other Neisserial or host cell polypeptides) or substantially isolated form. In general, the polypeptides are provided in a non-naturally occurring environment *e.g.* they are separated from their naturally-occurring environment. In certain embodiments, the subject polypeptide is present in a composition that is enriched for the polypeptide as compared to a control. As such, purified polypeptide is provided, whereby purified is meant that the polypeptide is present in a composition that is substantially free of other expressed polypeptides, where by substantially free is meant that less than 90%, usually less than 60% and more usually less than 50% of the composition is made up of other expressed polypeptides.

Polypeptides can take various forms (*e.g.* native, fusions, glycosylated, non-glycosylated, lipidated, disulfide bridges, *etc.*).

SEQ ID NOs 4 to 78 do not include an N-terminus methionine. If a polypeptide of the invention is produced by translation in a biological host then a start codon is required, which will provide an N-terminus methionine in most hosts. Thus a polypeptide of the invention will, at least at a nascent stage, include a methionine residue upstream of said SEQ ID NO sequence.

In some embodiments the polypeptide has a single methionine at the N-terminus immediately followed by the SEQ ID NO sequence; in other embodiments a longer upstream sequence may be used. Such an upstream sequence may be short *(e.g.* 40 or fewer amino acids *i.e.* 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1). Examples include leader sequences to direct protein trafficking, or short peptide sequences which facilitate cloning or purification *(e.g.* histidine tags *i.e.* His*ₙ* where *n* = 3, 4, 5, 6, 7, 8, 9, 10 or more *e.g.* SEQ ID NO: 95). Other suitable N-terminal amino acid sequences will be apparent to those skilled in the art.

A polypeptide of the invention may also include amino acids downstream of the final amino acid of the SEQ ID NO sequences. Such C-terminal extensions may be short (*e.g.* 40 or fewer amino acids *i.e.* 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1). Examples include sequences to direct protein trafficking, short peptide sequences which facilitate cloning *(e.g.* a Leu-Glu dipeptide) or purification (*e.g.* comprising histidine tags *i.e.* His*ₙ* where *n =* 3, 4, 5, 6, 7, 8, 9, 10 or more *e.g.* SEQ ID NO: 95), or sequences which enhance polypeptide stability. Combinations of these may be used *e.g.* SEQ ID NO: 96, providing a Leu-Glu dipeptide and a hexa-histidine tag. Other suitable C-terminal amino acid sequences will be apparent to those skilled in the art.

The term "polypeptide" refers to amino acid polymers of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, *etc.),* as well as other modifications known in the art. Polypeptides can occur as single chains or associated chains.

Polypeptides of the invention may be attached or immobilised to a solid support.

Polypeptides of the invention may comprise a detectable label *e.g.* a radioactive label, a fluorescent label, or a biotin label. This is particularly useful in immunoassay techniques.

As disclosed in reference 13, fHBP can be split into three domains, referred to as A, B and C. Taking SEQ ID NO: 1, the three domains are (A) 1-119, (B) 120-183 and (C) 184-274:

MNRTAFCCLSLTTALILTACSSGGGGVAADIGAGLADALTAPLDHKDKGLQSLTLDQSVRKNEKLK
LAAQGAEKTYGNGDSLNTGKLKNDKVSRFDFIRQIEVDGQLITLESGEFQVYKQSHSALTAFQTEQ
IQDSEHSGKMVAKRQFRIGDIAGEHTSFDKLPEGGRATYRGTAFGSDDAGGKLTYTIDFAAKQGNG
KIEHLKSPELNVDLAAADIKPDGKRHAVISGSVLYNQAEKGSYSLGIFGGKAQEVAGSAEVKTVNG
IRHIGLAAKQ

The mature form of domain 'A', from Cys-20 at its N-terminus to Lys-119, is called 'Aₘₐₜᵤᵣₑ'.

Multiple fHBP sequences are known and these can readily be aligned using standard methods. By such alignments the skilled person can identify (a) domains 'A' (and 'Aₘₐₜᵤᵣₑ'), 'B' and 'C' in any given fHBP sequence by comparison to the coordinates in the MC58 sequence, and (b) single residues in multiple fHBP sequences e.g. for identifying substitutions. For ease of reference, however, the domains are defined below:
- Domain 'A' in a given fHBP sequence is the fragment of that sequence which, when aligned to SEQ ID NO: 1 using a pairwise alignment algorithm, starts with the amino acid aligned to Met-1 of SEQ ID NO: 1 and ends with the amino acid aligned to Lys-119 of SEQ ID NO: 1.
- Domain 'Aₘₐₜᵤᵣₑ' in a given fHBP sequence is the fragment of that sequence which, when aligned to SEQ ID NO: 1 using a pairwise alignment algorithm, starts with the amino acid aligned to Cys-20 of SEQ ID NO: 1 and ends with the amino acid aligned to Lys-119 of SEQ ID NO: 1.
- Domain 'B' in a given fHBP sequence is the fragment of that sequence which, when aligned to SEQ ID NO: 1 using a pairwise alignment algorithm, starts with the amino acid aligned to Gln-120 of SEQ ID NO: 1 and ends with the amino acid aligned to Gly-183 of SEQ ID NO: 1.
- Domain 'C' in a given fHBP sequence is the fragment of that sequence which, when aligned to SEQ ID NO: 1 using a pairwise alignment algorithm, starts with the amino acid aligned to Lys-184 of SEQ ID NO: 1 and ends with the amino acid aligned to Gln-274 of SEQ ID NO: 1.

The preferred pairwise alignment algorithm for defining the domains is the Needleman-Wunsch global alignment algorithm [26], using default parameters (*e.g.* with Gap opening penalty = 10.0, and with Gap extension penalty = 0.5, using the EBLOSUM62 scoring matrix). This algorithm is conveniently implemented in the *needle* tool in the EMBOSS package [27].

In some embodiments, a fHBP amino acid sequence in a polypeptide of the invention is truncated to remove its domain A *i.e.* domain A is omitted from a SEQ ID.

In some embodiments, a polypeptide comprises an amino acid sequence as described above, except that up to 10 amino acids (*i.e.* 1, 2, 3, 4, 5, 6, 7, 8, 9 or 10) at the N-terminus and/or up to 10 amino acids (*i.e.* 1, 2, 3, 4, 5, 6, 7, 8, 9 or 10) at the C-terminus are deleted. Thus the polypeptide may comprise an amino acid sequence comprising a fragment of an amino acid sequence selected from the group consisting of SEQ ID NOs 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76 & 77, wherein said fragment is amino acids *a* to *b* of said SEQ ID, wherein *a* is 1, 2, 3, 4, 5, 6, 7, 8, 9, 10 or 11, and wherein *b* is *j, j-1, j-2, j-3, j-4, j-5, j-6, j-7, j-8, j-9* or *j-10* where *j* is the length of said SEQ ID. Longer truncations *(e.g.* up to 15 amino acids, up to 20 amino acids, *etc.)* may also be used.

### Nucleic acids

The invention provides nucleic acid encoding a polypeptide of the invention as defined above.

Nucleic acids of the invention may be prepared in many ways *e.g.* by chemical synthesis *(e.g.* phosphoramidite synthesis of DNA) in whole or in part, by digesting longer nucleic acids using nucleases *(e.g.* restriction enzymes), by joining shorter nucleic acids or nucleotides *(e.g.* using ligases or polymerases), from genomic or cDNA libraries, *etc.*

Nucleic acids of the invention can take various forms *e.g.* single-stranded, double-stranded, vectors, primers, probes, labelled, unlabelled, *etc.*

Nucleic acids of the invention are preferably in isolated or substantially isolated form.

The term "nucleic acid" includes DNA and RNA, and also their analogues, such as those containing modified backbones, and also peptide nucleic acids (PNA), *etc.*

Nucleic acid according to the invention may be labelled *e.g.* with a radioactive or fluorescent label.

The invention also provides vectors (such as plasmids) comprising nucleotide sequences of the invention (*e.g.* cloning or expression vectors, such as those suitable for nucleic acid immunisation) and non-human host cells transformed with such vectors.

### Bactericidal responses

Preferred polypeptides of the invention can elicit antibody responses that are bactericidal against meningococci. Bactericidal antibody responses are conveniently measured in mice and are a standard indicator of vaccine efficacy [*e.g*. see end-note 14 of reference 2]. Polypeptides of the invention can preferably elicit an antibody response which is bactericidal against at least one *N.meningitidis* strain from each of at least two of the following three groups of strains:
(I) MC58, gb185 (=M01-240185), m4030, m2197, m2937, iss1001, NZ394/98, 67/00, 93/114, bzl98, m1390, nge28, lnp17592, 00-241341, f6124, 205900, m198/172, bz133, gb149 (=M01-240149), nm008, nm092, 30/00, 39/99, 72/00, 95330, bz169, bz83, cu385, h44/76, m1590, m2934, m2969, m3370, m4215, m4318, n44/89, 14847.
(II) 961-5945, 2996, 96217, 312294, 11327, a22, gb013 (=M01-240013), e32, m1090, m4287, 860800, 599, 95N477, 90-18311, c11, m986, m2671, 1000, m1096, m3279, bz232, dk353, m3697, ngh38, L93/4286.
(III) M1239, 16889, gb355 (=M01-240355), m3369, m3813, ngp165.

For example, a polypeptide may elicit a bactericidal response effective against two or three of serogroup B *N.meningitidis* strains MC58, 961-5945 and M1239.

The polypeptide can preferably elicit an antibody response which is bactericidal against at least 50% of clinically-relevant meningococcal serogroup B strains (*e.g.* 60%, 70%, 80%, 90%, 95% or more). The polypeptide may elicit an antibody response which is bactericidal against strains of serogroup B *N*.*meningitidis* and strains of at least one *(e.g.* 1, 2, 3, 4) of serogroups A, C, W135 and Y. The polypeptide may elicit an antibody response which is bactericidal against strains of *N.gonorrhoeae* and/or *N.cinerea.* The polypeptide may elicit a response which is bactericidal against strains from at least two of the three main branches of the dendrogram shown in Figure 5 of reference 4.

The polypeptide may elicit an antibody response which is bactericidal against *N*.*meningitidis* strains in at least 2 *(e.g.* 2, 3, 4, 5, 6, 7) of hypervirulent lineages ET-37, ET-5, cluster A4, lineage 3, subgroup I, subgroup III, and subgroup IV-1 [28,29]. Polypeptides may additionally induce bactericidal antibody responses against one or more hyperinvasive lineages.

Polypeptides may elicit an antibody response which is bactericidal against *N*.*meningitidis* strains in at least 2 *(e.g.* 2, 3, 4, 5, 6, 7) of the following multilocus sequence types: ST1, ST4, ST5, ST8, ST11, ST32 and ST41 [30]. The polypeptide may also elicit an antibody response which is bactericidal against ST44 strains.

The polypeptide need not induce bactericidal antibodies against each and every MenB strain within the specified lineages or MLST; rather, for any given group of four or more strains of serogroup B meningococcus within a particular hypervirulent lineage or MLST, the antibodies induced by the composition are preferably bactericidal against at least 50% *(e.g.* 60%, 70%, 80%, 90% or more) of the group. Preferred groups of strains will include strains isolated in at least four of the following countries: GB, AU, CA, NO, IT, US, NZ, NL, BR, and CU. The serum preferably has a bactericidal titre of at least 1024 (*e.g.* 2¹⁰, 2¹¹, 2¹², 2¹³, 2¹⁴, 2¹⁵, 2¹⁶, 2¹⁷, 2¹⁸ or higher, preferably at least 2¹⁴) *i.e.* the serum is able to kill at least 50% of test bacteria of a particular strain when diluted 1:1024 *e.g.* as described in end-note 14 of reference 2. Preferred chimeric polypeptides can elicit an antibody response in mice that remains bactericidal even when the serum is diluted 1:4096 or further.

### Immunisation

A polypeptide of the invention may be used as an active component of an immunogenic composition, and so the invention provides an immunogenic composition comprising a polypeptide of the invention.

The disclosure also provides a method for raising an antibody response in a mammal, comprising administering an immunogenic composition of the invention to the mammal. The antibody response is preferably a protective and/or bactericidal antibody response. The invention also provides polypeptides of the invention for use in such methods.

The disclosure also provides a method for protecting a mammal against a Neisserial (*e.g.* meningococcal) infection and/or disease (*e.g.* against meningococcal meningitis), comprising administering to the mammal an immunogenic composition of the invention.

The disclosure provides polypeptides of the invention for use as medicaments *(e.g.* as immunogenic compositions or as vaccines) or as diagnostic reagents. It also provides the use of nucleic acid, polypeptide, or antibody disclosed herein in the manufacture of a medicament for preventing Neisserial (*e.g.* meningococcal) infection in a mammal.

The mammal is preferably a human. The human may be an adult or, preferably, a child. Where the vaccine is for prophylactic use, the human is preferably a child *(e.g.* a toddler or infant); where the vaccine is for therapeutic use, the human is preferably an adult. A vaccine intended for children may also be administered to adults *e.g.* to assess safety, dosage, immunogenicity, *etc.*

The uses and methods are particularly useful for preventing/treating diseases including, but not limited to, meningitis (particularly bacterial, such as meningococcal, meningitis) and bacteremia.

Efficacy of therapeutic treatment can be tested by monitoring Neisserial infection after administration of the composition of the invention. Efficacy of prophylactic treatment can be tested by monitoring immune responses against fHBP after administration of the composition. Immunogenicity of compositions of the invention can be determined by administering them to test subjects (*e.g.* children 12-16 months age, or animal models [31]) and then determining standard parameters including serum bactericidal antibodies (SBA) and ELISA titres (GMT). These immune responses will generally be determined around 4 weeks after administration of the composition, and compared to values determined before administration of the composition. A SBA increase of at least 4-fold or 8-fold is preferred. Where more than one dose of the composition is administered, more than one post-administration determination may be made.

Preferred compositions of the invention can confer an antibody titre in a patient that is superior to the criterion for seroprotection for each antigenic component for an acceptable percentage of human subjects. Antigens with an associated antibody titre above which a host is considered to be seroconverted against the antigen are well known, and such titres are published by organisations such as WHO. Preferably more than 80% of a statistically significant sample of subjects is seroconverted, more preferably more than 90%, still more preferably more than 93% and most preferably 96-100%.

Compositions of the invention will generally be administered directly to a patient. Direct delivery may be accomplished by parenteral injection (*e.g.* subcutaneously, intraperitoneally, intravenously, intramuscularly, or to the interstitial space of a tissue), or by rectal, oral, vaginal, topical, transdermal, intranasal, ocular, aural, pulmonary or other mucosal administration. Intramuscular administration to the thigh or the upper arm is preferred. Injection may be via a needle *(e.g.* a hypodermic needle), but needle-free injection may alternatively be used. A typical intramuscular dose is about 0.5 ml.

The invention may be used to elicit systemic and/or mucosal immunity.

Dosage treatment can be a single dose schedule or a multiple dose schedule. Multiple doses may be used in a primary immunisation schedule and/or in a booster immunisation schedule. A primary dose schedule may be followed by a booster dose schedule. Suitable timing between priming doses (*e.g.* between 4-16 weeks), and between priming and boosting, can be routinely determined.

The immunogenic composition of the invention will generally include a pharmaceutically acceptable carrier, which can be any substance that does not itself induce the production of antibodies harmful to the patient receiving the composition, and which can be administered without undue toxicity. Pharmaceutically acceptable carriers can include liquids such as water, saline, glycerol and ethanol. Auxiliary substances, such as wetting or emulsifying agents, pH buffering substances, and the like, can also be present in such vehicles. A thorough discussion of suitable carriers is available in ref. 32.

Neisserial infections affect various areas of the body and so the compositions of the invention may be prepared in various forms. For example, the compositions may be prepared as injectables, either as liquid solutions or suspensions. Solid forms suitable for solution in, or suspension in, liquid vehicles prior to injection can also be prepared. The composition may be prepared for topical administration *e.g.* as an ointment, cream or powder. The composition may be prepared for oral administration *e.g.* as a tablet or capsule, or as a syrup (optionally flavoured). The composition may be prepared for pulmonary administration *e.g.* as an inhaler, using a fine powder or a spray. The composition may be prepared as a suppository or pessary. The composition may be prepared for nasal, aural or ocular administration *e.g.* as drops.

The composition is preferably sterile. It is preferably pyrogen-free. It is preferably buffered *e.g.* at between pH 6 and pH 8, generally around pH 7. Where a composition comprises an aluminium hydroxide salt, it is preferred to use a histidine buffer [33]. Compositions of the invention may be isotonic with respect to humans.

Immunogenic compositions comprise an immunologically effective amount of immunogen, as well as any other of other specified components, as needed. By 'immunologically effective amount', it is meant that the administration of that amount to an individual, either in a single dose or as part of a series, is effective for treatment or prevention. This amount varies depending upon the health and physical condition of the individual to be treated, age, the taxonomic group of individual to be treated *(e.g.* non-human primate, primate, *etc.),* the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials. Dosage treatment may be a single dose schedule or a multiple dose schedule (*e.g.* including booster doses). The composition may be administered in conjunction with other immunoregulatory agents.

Adjuvants which may be used in compositions of the invention include, but are not limited to:

### A. Mineral-containing compositions

Mineral containing compositions suitable for use as adjuvants in the invention include mineral salts, such as aluminium salts and calcium salts. The invention may include mineral salts such as hydroxides (*e.g.* oxyhydroxides), phosphates (*e.g.* hydroxyphosphates, orthophosphates), sulphates, *etc.* [*e.g.* see chapters 8 & 9 of ref. 34], or mixtures of different mineral compounds, with the compounds taking any suitable form *(e.g.* gel, crystalline, amorphous, *etc.),* and with adsorption being preferred. The mineral containing compositions may also be formulated as a particle of metal salt [35].

A useful aluminium phosphate adjuvant is amorphous aluminium hydroxyphosphate with PO₄/Al molar ratio between 0.84 and 0.92, included at 0.6mg Al³⁺/ml.

### B. Oil Emulsions

Oil emulsion compositions suitable for use as adjuvants in the invention include squalene-in-water emulsions, such as MF59 [Chapter 10 of ref. 34; see also ref. 36] (5% Squalene, 0.5% Tween 80, and 0.5% Span 85, formulated into submicron particles using a microfluidizer). Complete Freund's adjuvant (CFA) and incomplete Freund's adjuvant (IFA) may also be used.

Useful oil-in-water emulsions typically include at least one oil and at least one surfactant, with the oil(s) and surfactant(s) being biodegradable (metabolisable) and biocompatible. The oil droplets in the emulsion are generally less than 1µm in diameter, with these small sizes being achieved with a microfluidiser to provide stable emulsions. Droplets with a size less than 220nm are preferred as they can be subjected to filter sterilization.

The emulsion can comprise oils such as those from an animal (such as fish) or vegetable source. Sources for vegetable oils include nuts, seeds and grains. Peanut oil, soybean oil, coconut oil, and olive oil, the most commonly available, exemplify the nut oils. Jojoba oil can be used *e.g.* obtained from the jojoba bean. Seed oils include safflower oil, cottonseed oil, sunflower seed oil, sesame seed oil and the like. In the grain group, corn oil is the most readily available, but the oil of other cereal grains such as wheat, oats, rye, rice, teff, triticale and the like may also be used. 6-10 carbon fatty acid esters of glycerol and 1,2-propanediol, while not occurring naturally in seed oils, may be prepared by hydrolysis, separation and esterification of the appropriate materials starting from the nut and seed oils. Fats and oils from mammalian milk are metabolizable and may therefore be used in the practice of this invention. The procedures for separation, purification, saponification and other means necessary for obtaining pure oils from animal sources are well known in the art. Most fish contain metabolizable oils which may be readily recovered. For example, cod liver oil, shark liver oils, and whale oil such as spermaceti exemplify several of the fish oils which may be used herein. A number of branched chain oils are synthesized biochemically in 5-carbon isoprene units and are generally referred to as terpenoids. Shark liver oil contains a branched, unsaturated terpenoids known as squalene, 2,6,10,15,19,23-hexamethyl-2,6,10,14,18,22-tetracosahexaene, which is particularly preferred herein. Squalane, the saturated analog to squalene, is also a preferred oil. Fish oils, including squalene and squalane, are readily available from commercial sources or may be obtained by methods known in the art. Other preferred oils are the tocopherols (see below). Mixtures of oils can be used.

Surfactants can be classified by their 'HLB' (hydrophile/lipophile balance). Preferred surfactants have a HLB of at least 10, preferably at least 15, and more preferably at least 16. The invention can be used with surfactants including, but not limited to: the polyoxyethylene sorbitan esters surfactants (commonly referred to as the Tweens), especially polysorbate 20 and polysorbate 80; copolymers of ethylene oxide (EO), propylene oxide (PO), and/or butylene oxide (BO), sold under the DOWFAX™ tradename, such as linear EO/PO block copolymers; octoxynols, which can vary in the number of repeating ethoxy (oxy-1,2-ethanediyl) groups, with octoxynol-9 (Triton X-100, or t-octylphenoxypolyethoxyethanol) being of particular interest; (octylphenoxy)polyethoxyethanol (IGEPAL CA-630/NP-40); phospholipids such as phosphatidylcholine (lecithin); nonylphenol ethoxylates, such as the Tergitol™ NP series; polyoxyethylene fatty ethers derived from lauryl, cetyl, stearyl and oleyl alcohols (known as Brij surfactants), such as triethyleneglycol monolauryl ether (Brij 30); and sorbitan esters (commonly known as the SPANs), such as sorbitan trioleate (Span 85) and sorbitan monolaurate. Non-ionic surfactants are preferred. Preferred surfactants for including in the emulsion are Tween 80 (polyoxyethylene sorbitan monooleate), Span 85 (sorbitan trioleate), lecithin and Triton X-100.

Mixtures of surfactants can be used e.g. Tween 80/Span 85 mixtures. A combination of a polyoxyethylene sorbitan ester such as polyoxyethylene sorbitan monooleate (Tween 80) and an octoxynol such as t-octylphenoxypolyethoxyethanol (Triton X-100) is also suitable. Another useful combination comprises laureth 9 plus a polyoxyethylene sorbitan ester and/or an octoxynol.

Preferred amounts of surfactants (% by weight) are: polyoxyethylene sorbitan esters (such as Tween 80) 0.01 to 1%, in particular about 0.1 %; octyl- or nonylphenoxy polyoxyethanols (such as Triton X-100, or other detergents in the Triton series) 0.001 to 0.1 %, in particular 0.005 to 0.02%; polyoxyethylene ethers (such as laureth 9) 0.1 to 20 %, preferably 0.1 to 10 % and in particular 0.1 to 1 % or about 0.5%.

Preferably, substantially all *(e.g.* at least 90% by number) of the oil droplets have a diameter of less than 1µm, e.g. ≤750nm, ≤500nm, ≤400nm, ≤300nm, ≤250nm, ≤220nm, ≤200nm, or smaller.

One specific useful submicron emulsion of squalene, Tween 80, and Span 85. The composition of the emulsion by volume can be about 5% squalene, about 0.5% polysorbate 80 and about 0.5% Span 85. In weight terms, these ratios become 4.3% squalene, 0.5% polysorbate 80 and 0.48% Span 85. This adjuvant is known as 'MF59' [37-39], as described in more detail in Chapter 10 of ref. 40 and chapter 12 of ref. 41. The MF59 emulsion advantageously includes citrate ions e.g. 10mM sodium citrate buffer.

### C. Saponin formulations [chapter 22 of ref. 341

Saponin formulations may also be used as adjuvants in the invention. Saponins are a heterogeneous group of sterol glycosides and triterpenoid glycosides that are found in the bark, leaves, stems, roots and even flowers of a wide range of plant species. Saponin from the bark of the *Quillaia saponaria* Molina tree have been widely studied as adjuvants. Saponin can also be commercially obtained from *Smilax ornata* (sarsaprilla), *Gypsophilla paniculata* (brides veil), and *Saponaria officianalis* (soap root). Saponin adjuvant formulations include purified formulations, such as QS21, as well as lipid formulations, such as ISCOMs. QS21 is marketed as Stimulon™.

Saponin compositions have been purified using HPLC and RP-HPLC. Specific purified fractions using these techniques have been identified, including QS7, QS17, QS18, QS21, QH-A, QH-B and QH-C. Preferably, the saponin is QS21. A method of production of QS21 is disclosed in ref. 42. Saponin formulations may also comprise a sterol, such as cholesterol [43].

Combinations of saponins and cholesterols can be used to form unique particles called immunostimulating complexs (ISCOMs) [chapter 23 of ref. 34]. ISCOMs typically also include a phospholipid such as phosphatidylethanolamine or phosphatidylcholine. Any known saponin can be used in ISCOMs. Preferably, the ISCOM includes one or more of QuilA, QHA & QHC. ISCOMs are further described in refs. 43-45. Optionally, the ISCOMS may be devoid of additional detergent [46].

A review of the development of saponin based adjuvants can be found in refs. 47 & 48.

### D. Virosomes and virus-like particles

Virosomes and virus-like particles (VLPs) can also be used as adjuvants in the invention. These structures generally contain one or more proteins from a virus optionally combined or formulated with a phospholipid. They are generally non-pathogenic, non-replicating and generally do not contain any of the native viral genome. The viral proteins may be recombinantly produced or isolated from whole viruses. These viral proteins suitable for use in virosomes or VLPs include proteins derived from influenza virus (such as HA or NA), Hepatitis B virus (such as core or capsid proteins), Hepatitis E virus, measles virus, Sindbis virus, Rotavirus, Foot-and-Mouth Disease virus, Retrovirus, Norwalk virus, human Papilloma virus, HIV, RNA-phages, Qß-phage (such as coat proteins), GA-phage, fr-phage, AP205 phage, and Ty (such as retrotransposon Ty protein pl). VLPs are discussed further in refs. 49-54. Virosomes are discussed further in, for example, ref. 55

### E. Bacterial or microbial derivatives

Adjuvants suitable for use in the invention include bacterial or microbial derivatives such as non-toxic derivatives of enterobacterial lipopolysaccharide (LPS), Lipid A derivatives, immunostimulatory oligonucleotides and ADP-ribosylating toxins and detoxified derivatives thereof.

Non-toxic derivatives of LPS include monophosphoryl lipid A (MPL) and 3-O-deacylated MPL (3dMPL). 3dMPL is a mixture of 3 de-O-acylated monophosphoryl lipid A with 4, 5 or 6 acylated chains. A preferred "small particle" form of 3 De-O-acylated monophosphoryl lipid A is disclosed in ref. 56. Such "small particles" of 3dMPL are small enough to be sterile filtered through a 0.22µm membrane [56]. Other non-toxic LPS derivatives include monophosphoryl lipid A mimics, such as aminoalkyl glucosaminide phosphate derivatives *e.g.* RC-529 [57,58].

Lipid A derivatives include derivatives of lipid A from *Escherichia coli* such as OM-174. OM-174 is described for example in refs. 59 & 60.

Immunostimulatory oligonucleotides suitable for use as adjuvants in the invention include nucleotide sequences containing a CpG motif (a dinucleotide sequence containing an unmethylated cytosine linked by a phosphate bond to a guanosine). Double-stranded RNAs and oligonucleotides containing palindromic or poly(dG) sequences have also been shown to be immunostimulatory.

The CpG's can include nucleotide modifications/analogs such as phosphorothioate modifications and can be double-stranded or single-stranded. References 61, 62 and 63 disclose possible analog substitutions *e.g.* replacement of guanosine with 2'-deoxy-7-deazaguanosine. The adjuvant effect of CpG oligonucleotides is further discussed in refs. 64-69.

The CpG sequence may be directed to TLR9, such as the motif GTCGTT or TTCGTT [70]. The CpG sequence may be specific for inducing a Th1 immune response, such as a CpG-A ODN, or it maybe more specific for inducing a B cell response, such a CpG-B ODN. CpG-A and CpG-B ODNs are discussed in refs. 71-73. Preferably, the CpG is a CpG-A ODN.

Preferably, the CpG oligonucleotide is constructed so that the 5' end is accessible for receptor recognition. Optionally, two CpG oligonucleotide sequences may be attached at their 3' ends to form "immunomers". See, for example, refs. 70 & 74-76.

A particularly useful adjuvant based around immunostimulatory oligonucleotides is known as IC31™ [77]. Thus an adjuvant used with the invention may comprise a mixture of (i) an oligonucleotide (*e.g*. between 15-40 nucleotides) including at least one (and preferably multiple) CpI motifs (*i.e.* a cytosine linked to an inosine to form a dinucleotide), and (ii) a polycationic polymer, such as an oligopeptide (*e.g*. between 5-20 amino acids) including at least one (and preferably multiple) Lys-Arg-Lys tripeptide sequence(s). The oligonucleotide may be a deoxynucleotide comprising 26-mer sequence 5'-(IC)₁₃-3' (SEQ ID NO: 79). The polycationic polymer may be a peptide comprising 11-mer amino acid sequence KLKLLLLLKLK (SEQ ID NO: 80).

Bacterial ADP-ribosylating toxins and detoxified derivatives thereof may be used as adjuvants in the invention. Preferably, the protein is derived from *E.coli* (*E.coli* heat labile enterotoxin "LT"), cholera ("CT"), or pertussis ("PT"). The use of detoxified ADP-ribosylating toxins as mucosal adjuvants is described in ref. 78 and as parenteral adjuvants in ref. 79. The toxin or toxoid is preferably in the form of a holotoxin, comprising both A and B subunits. Preferably, the A subunit contains a detoxifying mutation; preferably the B subunit is not mutated. Preferably, the adjuvant is a detoxified LT mutant such as LT-K63, LT-R72, and LT-G192. The use of ADP-ribosylating toxins and detoxified derivatives thereof, particularly LT-K63 and LT-R72, as adjuvants can be found in refs. 80-87. A useful CT mutant is or CT-E29H [88]. Numerical reference for amino acid substitutions is preferably based on the alignments of the A and B subunits of ADP-ribosylating toxins set forth in ref. 89.

### F. Human immunomodulators

Human immunomodulators suitable for use as adjuvants in the invention include cytokines, such as interleukins (*e.g.* IL-1, IL-2, IL-4, IL-5, IL-6, IL-7, IL-12 [90], *etc.*) [91], interferons (*e.g.* interferon-γ), macrophage colony stimulating factor, and tumor necrosis factor. A preferred immunomodulator is IL-12.

### G. Bioadhesives and Mucoadhesives

Bioadhesives and mucoadhesives may also be used as adjuvants in the invention. Suitable bioadhesives include esterified hyaluronic acid microspheres [92] or mucoadhesives such as cross-linked derivatives of poly(acrylic acid), polyvinyl alcohol, polyvinyl pyrollidone, polysaccharides and carboxymethylcellulose. Chitosan and derivatives thereof may also be used as adjuvants in the invention [93].

### H. Microparticles

Microparticles may also be used as adjuvants in the invention. Microparticles (*i.e.* a particle of ∼100nm to ∼150µm in diameter, more preferably ∼200nm to ∼30µm in diameter, and most preferably ∼500nm to ∼10µm in diameter) formed from materials that are biodegradable and non-toxic (*e.g.* a poly(α-hydroxy acid), a polyhydroxybutyric acid, a polyorthoester, a polyanhydride, a polycaprolactone, *etc.),* with poly(lactide-co-glycolide) are preferred, optionally treated to have a negatively-charged surface *(e.g.* with SDS) or a positively-charged surface *(e.g.* with a cationic detergent, such as CTAB).

### 1. Liposomes (Chapters 13 & 14 of ref. 34)

Examples of liposome formulations suitable for use as adjuvants are described in refs. 94-96.

### J. Polyoxyethylene ether and polyoxyethylene ester formulations

Adjuvants suitable for use in the invention include polyoxyethylene ethers and polyoxyethylene esters [97]. Such formulations further include polyoxyethylene sorbitan ester surfactants in combination with an octoxynol [98] as well as polyoxyethylene alkyl ethers or ester surfactants in combination with at least one additional non-ionic surfactant such as an octoxynol [99]. Preferred polyoxyethylene ethers are selected from the following group: polyoxyethylene-9-lauryl ether (laureth 9), polyoxyethylene-9-steoryl ether, polyoxytheylene-8-steoryl ether, polyoxyethylene-4-lauryl ether, polyoxyethylene-35-lauryl ether, and polyoxyethylene-23-lauryl ether.

### K. Polyphosphazene (PCPP)

PCPP formulations are described, for example, in refs. 100 and 101.

### L. Muramyl peptides

Examples of muramyl peptides suitable for use as adjuvants in the invention include N-acetyl-muramyl-L-threonyl-D-isoglutamine (thr-MDP), N-acetyl-normuramyl-L-alanyl-D-isoglutamine (nor-MDP), and N-acetylmuramyl-L-alanyl-D-isoglutaminyl-L-alanine-2-(1'-2'-dipalmitoyl-*sn-*glycero-3-hydroxyphosphoryloxy)-ethylamine MTP-PE).

### M. Imidazoquinolone Compounds.

Examples of imidazoquinolone compounds suitable for use adjuvants in the invention include Imiquamod and its homologues (*e.g.* "Resiquimod 3M"), described further in refs. 102 and 103.

The invention may also comprise combinations of aspects of one or more of the adjuvants identified above. For example, the following adjuvant compositions may be used in the invention: (1) a saponin and an oil-in-water emulsion [104]; (2) a saponin *(e.g.* QS21) + a non-toxic LPS derivative *(e.g.* 3dMPL) [105]; (3) a saponin *(e.g.* QS21) + a non-toxic LPS derivative *(e.g.* 3dMPL) + a cholesterol; (4) a saponin (*e.g.* QS21) + 3dMPL + IL-12 (optionally + a sterol) [106]; (5) combinations of 3dMPL with, for example, QS21 and/or oil-in-water emulsions [107]; (6) SAF, containing 10% squalane, 0.4% Tween 80™, 5% pluronic-block polymer L121, and thr-MDP, either microfluidized into a submicron emulsion or vortexed to generate a larger particle size emulsion. (7) Ribi™ adjuvant system (RAS), (Ribi Immunochem) containing 2% squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); and (8) one or more mineral salts (such as an aluminum salt) + a non-toxic derivative of LPS (such as 3dMPL).

Other substances that act as immunostimulating agents are disclosed in chapter 7 of ref. 34.

The use of an aluminium hydroxide and/or aluminium phosphate adjuvant is particularly preferred, and antigens are generally adsorbed to these salts. Other preferred adjuvant combinations include combinations of Th1 and Th2 adjuvants such as CpG & alum or resiquimod & alum. A combination of aluminium phosphate and 3dMPL may be used.

### Further antigenic components

Compositions of the invention include polypeptides comprising modified fHBP sequences. It is useful if the composition should not include complex or undefined mixtures of antigens *e.g.* it is preferred not to include outer membrane vesicles in the composition. Polypeptides of the invention are preferably expressed recombinantly in a heterologous host and then purified.

As well as including a fHBP-containing polypeptide, a composition of the invention may also include one or more further neisserial antigen(s), as a vaccine which targets more than one antigen per bacterium decreases the possibility of selecting escape mutants. Thus a composition can include a second polypeptide that, when administered to a mammal, elicits an antibody response that is bactericidal against meningococcus. The second polypeptide will not be a meningococcal fHBP, but it may be *e.g.* a 287 sequence, a NadA sequence, a 953 sequence, a 936 sequence, *etc.* A composition may include one or more of: a polypeptide comprising SEQ ID NO: 90; a polypeptide comprising SEQ ID NO: 91; and/or a polypeptide comprising SEQ ID NO: 92 or 139 (see refs. 108 & 109).

A composition comprising a first polypeptide comprising a fusion of a 936 antigen and at least one modified fHBP, a second polypeptide comprising amino acid sequence SEQ ID NO: 90 and a third polypeptide comprising amino acid sequence SEQ ID NO: 92 is useful. The first polypeptide may, for instance, comprise any one of amino acid sequences SEQ ID NOs: 124, 125, 126, 127, 128, 129.

A composition comprising a first polypeptide comprising a fusion of a 936 antigen and at least one modified fHBP, a second polypeptide comprising amino acid sequence SEQ ID NO: 90 and a third polypeptide comprising amino acid sequence SEQ ID NO: 139 is useful. The first polypeptide may, for instance, comprise any one of amino acid sequences SEQ ID NOs: 124, 125, 126, 127, 128, 129, and it preferably comprises SEQ ID NO: 126. This composition may include meningococcal outer membrane vesicles as described elsewhere herein, but preferably does not.

Antigens for inclusion in the compositions include polypeptides comprising one or more of:
(a) the 446 even SEQ IDs (*i.e.* 2, 4, 6, ... , 890, 892) disclosed in reference 110.
(b) the 45 even SEQ IDs (i.e. 2, 4, 6, ... , 88, 90) disclosed in reference 111;
(c) the 1674 even SEQ IDs 2-3020, even SEQ IDs 3040-3114, and all SEQ IDs 3115-3241, disclosed in reference 3;
(d) the 2160 amino acid sequences NMB0001 to NMB2160 from reference 2;
(e) a meningococcal PorA protein, of any subtype, preferably recombinantly expressed;
(f) a variant, homolog, ortholog, paralog, mutant *etc.* of (a) to (e); or
(g) an outer membrane vesicle preparation from *N.meningitidis [e.g.* see ref. 173], but preferably not.

In addition to Neisserial polypeptide antigens, the composition may include antigens for immunising against other diseases or infections. For example, the composition may include one or more of the following further antigens:
- a saccharide antigen from *N.meningitidis* serogroup A, C, W135 and/or Y, such as the saccharide disclosed in ref. 112 from serogroup C [see also ref. 113] or in ref. 114.
- a saccharide antigen from *Streptococcus pneumoniae* [*e.g.* 115, 116, 117].
- an antigen from hepatitis A virus, such as inactivated virus [*e.g.* 118, 119].
- an antigen from hepatitis B virus, such as the surface and/or core antigens [*e.g*. 119, 120].
- a diphtheria antigen, such as a diphtheria toxoid *[e.g.* chapter 3 of ref. 121] *e.g.* the CRM₁₉₇ mutant [*e.g*. 122].
- a tetanus antigen, such as a tetanus toxoid *[e.g.* chapter 4 of ref. 121].
- an antigen from *Bordetella pertussis,* such as pertussis holotoxin (PT) and filamentous haemagglutinin (FHA) from *B.pertussis,* optionally also in combination with pertactin and/or agglutinogens 2 and 3 *[e.g.* refs. 123 & 124].
- a saccharide antigen from *Haemophilus influenzae* B [*e.g.* 113].
- polio antigen(s) [*e.g*. 125, 126] such as IPV.
- measles, mumps and/or rubella antigens [*e.g*. chapters 9, 10 & 11 of ref. 121].
- influenza antigen(s) [*e.g.* chapter 19 of ref. 121], such as the haemagglutinin and/or neuraminidase surface proteins.
- an antigen from *Moraxella catarrhalis* [*e.g.* 127].
- an protein antigen from *Streptococcus agalactiae* (group B streptococcus) [*e.g.* 128, 129].
- a saccharide antigen from *Streptococcus agalactiae* (group B streptococcus).
- an antigen from *Streptococcus pyogenes* (group A streptococcus) [*e.g.* 129, 130, 131].
- an antigen from *Staphylococcus aureus* [*e.g.* 132].

The composition may comprise one or more of these further antigens.

Toxic protein antigens may be detoxified where necessary (*e.g.* detoxification of pertussis toxin by chemical and/or genetic means [124]).

Where a diphtheria antigen is included in the composition it is preferred also to include tetanus antigen and pertussis antigens. Similarly, where a tetanus antigen is included it is preferred also to include diphtheria and pertussis antigens. Similarly, where a pertussis antigen is included it is preferred also to include diphtheria and tetanus antigens. DTP combinations are thus preferred. Saccharide antigens are preferably in the form of conjugates. Carrier proteins for the conjugates are discussed in more detail below.

Antigens in the composition will typically be present at a concentration of at least 1µg/ml each. In general, the concentration of any given antigen will be sufficient to elicit an immune response against that antigen.

Immunogenic compositions of the invention may be used therapeutically (*i.e.* to treat an existing infection) or prophylactically *(i.e.* to prevent future infection).

As an alternative to using proteins antigens in the immunogenic compositions of the invention, nucleic acid (preferably DNA *e.g.* in the form of a plasmid) encoding the antigen may be used.

In some embodiments a composition of the invention comprises in addition to the fHBP sequence, conjugated capsular saccharide antigens from 1, 2, 3 or 4 of meningococcus serogroups A, C, W135 and Y. In other embodiments a composition of the invention comprises in addition to the fHBP sequence, at least one conjugated pneumococcal capsular saccharide antigen.

### Meningococcus serogroups Y, W135, C and A

Current serogroup C vaccines (Menjugate™ [133,112], Meningitec™ and NeisVac-C™) include conjugated saccharides. Menjugate™ and Meningitec™ have oligosaccharide antigens conjugated to a CRM₁₉₇ carrier, whereas NeisVac-C™ uses the complete polysaccharide (de-O-acetylated) conjugated to a tetanus toxoid carrier. The Menactra™ vaccine contains conjugated capsular saccharide antigens from each of serogroups Y, W135, C and A.

Compositions of the present invention may include capsular saccharide antigens from one or more of meningococcus serogroups Y, W135, C and A, wherein the antigens are conjugated to carrier protein(s) and/or are oligosaccharides. For example, the composition may include a capsular saccharide antigen from: serogroup C; serogroups A and C; serogroups A, C and W135; serogroups A, C and Y; serogroups C, W135 and Y; or from all four of serogroups A, C, W135 and Y.

A typical quantity of each meningococcal saccharide antigen per dose is between 1µg and 20µg e.g. about 1µg, about 2.5µg, about 4µg, about 5µg, or about 10µg (expressed as saccharide).

Where a mixture comprises capsular saccharides from both serogroups A and C, the ratio (w/w) of MenA saccharide:MenC saccharide may be greater than 1 *(e.g.* 2:1, 3:1, 4:1, 5:1, 10:1 or higher). Where a mixture comprises capsular saccharides from serogroup Y and one or both of serogroups C and W135, the ratio (w/w) of MenY saccharide:MenW135 saccharide may be greater than 1 *(e.g.* 2:1, 3:1, 4:1, 5:1, 10:1 or higher) and/or that the ratio (w/w) of MenY saccharide:MenC saccharide may be less than 1 *(e.g.* 1:2, 1:3, 1:4, 1:5, or lower). Preferred ratios (w/w) for saccharides from serogroups A:C:W135:Y are: 1:1:1:1; 1:1:1:2; 2:1:1:1; 4:2:1:1; 8:4:2:1; 4:2:1:2; 8:4:1:2; 4:2:2:1; 2:2:1:1; 4:4:2:1; 2:2:1:2; 4:4:1:2; and 2:2:2:1. Preferred ratios (w/w) for saccharides from serogroups C:W135:Y are: 1:1:1; 1:1:2; 1:1:1; 2:1:1; 4:2:1; 2:1:2; 4:1:2; 2:2:1; and 2:1:1. Using a substantially equal mass of each saccharide is preferred.

Capsular saccharides may be used in the form of oligosaccharides. These are conveniently formed by fragmentation of purified capsular polysaccharide (*e.g.* by hydrolysis), which will usually be followed by purification of the fragments of the desired size.

Fragmentation of polysaccharides is preferably performed to give a final average degree of polymerisation (DP) in the oligosaccharide of less than 30 (*e.g.* between 10 and 20, preferably around 10 for serogroup A; between 15 and 25 for serogroups W135 and Y, preferably around 15-20; between 12 and 22 for serogroup C; *etc.*). DP can conveniently be measured by ion exchange chromatography or by colorimetric assays [134].

If hydrolysis is performed, the hydrolysate will generally be sized in order to remove short-length oligosaccharides [113]. This can be achieved in various ways, such as ultrafiltration followed by ion-exchange chromatography. Oligosaccharides with a degree of polymerisation of less than or equal to about 6 are preferably removed for serogroup A, and those less than around 4 are preferably removed for serogroups W135 and Y.

Preferred MenC saccharide antigens are disclosed in reference 133, as used in Menjugate™.

The saccharide antigen may be chemically modified. This is particularly useful for reducing hydrolysis for serogroup A [135; see below]. De-O-acetylation of meningococcal saccharides can be performed. For oligosaccharides, modification may take place before or after depolymerisation.

Where a composition of the invention includes a MenA saccharide antigen, the antigen is preferably a modified saccharide in which one or more of the hydroxyl groups on the native saccharide has/have been replaced by a blocking group [135]. This modification improves resistance to hydrolysis.

### Covalent conjugation

Capsular saccharides in compositions of the invention will usually be conjugated to carrier protein(s). In general, conjugation enhances the immunogenicity of saccharides as it converts them from T-independent antigens to T-dependent antigens, thus allowing priming for immunological memory. Conjugation is particularly useful for paediatric vaccines and is a well known technique.

Typical carrier proteins are bacterial toxins, such as diphtheria or tetanus toxins, or toxoids or mutants thereof. The CRM₁₉₇ diphtheria toxin mutant [136] is useful, and is the carrier in the PREVNAR™ product. Other suitable carrier proteins include the *N.meningitidis* outer membrane protein complex [137], synthetic peptides [138,139], heat shock proteins [140,141], pertussis proteins [142,143], cytokines [144], lymphokines [144], hormones [144], growth factors [144], artificial proteins comprising multiple human CD4⁺ T cell epitopes from various pathogen-derived antigens [145] such as N19 [146], protein D from *H.influenzae* [147-149], pneumolysin [150] or its non-toxic derivatives [151], pneumococcal surface protein PspA [152], iron-uptake proteins [153], toxin A or B from *C.difficile* [154], recombinant *P.aeruginosa* exoprotein A (rEPA) [155], *etc.*

Any suitable conjugation reaction can be used, with any suitable linker where necessary.

The saccharide will typically be activated or functionalised prior to conjugation. Activation may involve, for example, cyanylating reagents such as CDAP (*e.g.* 1-cyano-4-dimethylamino pyridinium tetrafluoroborate [156,157,*etc*.]). Other suitable techniques use carbodiimides, hydrazides, active esters, norborane, p-nitrobenzoic acid, N-hydroxysuccinimide, S-NHS, EDC, TSTU, *etc.*

Linkages via a linker group may be made using any known procedure, for example, the procedures described in references 158 and 159. One type of linkage involves reductive amination of the polysaccharide, coupling the resulting amino group with one end of an adipic acid linker group, and then coupling a protein to the other end of the adipic acid linker group [160,161]. Other linkers include B-propionamido [162], nitrophenyl-ethylamine [163], haloacyl halides [164], glycosidic linkages [165], 6-aminocaproic acid [166], ADH [167], C₄ to C₁₂ moieties [168] *etc.* As an alternative to using a linker, direct linkage can be used. Direct linkages to the protein may comprise oxidation of the polysaccharide followed by reductive amination with the protein, as described in, for example, references 169 and 170.

A process involving the introduction of amino groups into the saccharide (*e.g.* by replacing terminal =O groups with -NH₂) followed by derivatisation with an adipic diester (*e.g.* adipic acid N-hydroxysuccinimido diester) and reaction with carrier protein is preferred. Another preferred reaction uses CDAP activation with a protein D carrier *e.g.* for MenA or MenC.

### Outer membrane vesicles

It is preferred that compositions of the invention should not include complex or undefined mixtures of antigens, which are typical characteristics of OMVs. However, the invention can be used in conjunction with OMVs, as fHBP has been found to enhance their efficacy [6], in particular by over-expressing the polypeptides of the invention in the strains used for OMV preparation, such that the polypeptide is displayed on the OMV surface.

This approach may be used in general to improve preparations of *N.meningitidis* serogroup B microvesicles [171], 'native OMVs' [172], blebs or outer membrane vesicles *[e.g.* refs. 173 to 178, *etc*.]*.* These may be prepared from bacteria which have been genetically manipulated [179-182] *e.g.* to increase immunogenicity (*e.g.* hyper-express immunogens), to reduce toxicity, to inhibit capsular polysaccharide synthesis, to down-regulate PorA expression, *etc.* They may be prepared from hyperblebbing strains [183-186]. Vesicles from a non-pathogenic *Neisseria* may be included [187]. OMVs may be prepared without the use of detergents [188,189]. They may express non-Neisserial proteins on their surface [190]. They may be LPS-depleted. They may be mixed with recombinant antigens [173,191]. Vesicles from bacteria with different class I outer membrane protein subtypes may be used *e.g.* six different subtypes [192,193] using two different genetically-engineered vesicle populations each displaying three subtypes, or nine different subtypes using three different genetically-engineered vesicle populations each displaying three subtypes, *etc.* Useful subtypes include: P1.7,16; P1.5-1,2-2; P1.19,15-1; P1.5-2,10; P1.12-1,13; P1.7-2,4; P1.22,14; P1.7-1,1; P1.18-1,3,6.

Where vesicles are present in a composition, the amount can be specified in terms of total protein in the vesicle. A composition can include between 1 and 100 µg/ml of vesicles *e.g.* between 15-30µg/ml, or preferably a lower dose *e.g.* between 2-10µg/ml.

Further details about vesicles are given below.

### Protein expression

Bacterial expression techniques are known in the art. A bacterial promoter is any DNA sequence capable of binding bacterial RNA polymerase and initiating the downstream (3') transcription of a coding sequence (*e.g*. structural gene) into mRNA. A promoter will have a transcription initiation region which is usually placed proximal to the 5' end of the coding sequence. This transcription initiation region usually includes an RNA polymerase binding site and a transcription initiation site. A bacterial promoter may also have a second domain called an operator, that may overlap an adjacent RNA polymerase binding site at which RNA synthesis begins. The operator permits negative regulated (inducible) transcription, as a gene repressor protein may bind the operator and thereby inhibit transcription of a specific gene. Constitutive expression may occur in the absence of negative regulatory elements, such as the operator. In addition, positive regulation may be achieved by a gene activator protein binding sequence, which, if present is usually proximal (5') to the RNA polymerase binding sequence. An example of a gene activator protein is the catabolite activator protein (CAP), which helps initiate transcription of the lac operon in Escherichia coli (E. coli) [Raibaud et al. (1984) Annu. Rev. Genet. 18:173]. Regulated expression may therefore be either positive or negative, thereby either enhancing or reducing transcription.

Sequences encoding metabolic pathway enzymes provide particularly useful promoter sequences. Examples include promoter sequences derived from sugar metabolizing enzymes, such as galactose, lactose (*lac*) [Chang et al. (1977) Nature 198:1056], and maltose. Additional examples include promoter sequences derived from biosynthetic enzymes such as tryptophan (*trp*) [Goeddel et al. (1980) Nuc. Acids Res. 8:4057; Yelverton et al. (1981) Nucl. Acids Res. 9:731; US patent 4,738,921; EP-A-0036776 and EP-A-0121775]. The β-lactamase (*bla*) promoter system [Weissmann (1981) "The cloning of interferon and other mistakes." In Interferon 3 (ed. I. Gresser)], bacteriophage lambda PL [Shimatake et al. (1981) Nature 292:128] and T5 [US patent 4,689,406] promoter systems also provide useful promoter sequences. Another promoter of interest is an inducible arabinose promoter (pBAD).

In addition, synthetic promoters which do not occur in nature also function as bacterial promoters. For example, transcription activation sequences of one bacterial or bacteriophage promoter may be joined with the operon sequences of another bacterial or bacteriophage promoter, creating a synthetic hybrid promoter [US patent 4,551,433]. For example, the *tac* promoter is a hybrid *trp-lac* promoter comprised of both *trp* promoter and *lac* operon sequences that is regulated by the *lac* repressor [Amann et al. (1983) Gene 25:167; de Boer et al. (1983) Proc. Natl. Acad. Sci. 80:21]. Furthermore, a bacterial promoter can include naturally occurring promoters of non-bacterial origin that have the ability to bind bacterial RNA polymerase and initiate transcription. A naturally occurring promoter of non-bacterial origin can also be coupled with a compatible RNA polymerase to produce high levels of expression of some genes in prokaryotes. The bacteriophage T7 RNA polymerase/promoter system is an example of a coupled promoter system [Studier et al. (1986) J. Mol. Biol. 189:113; Tabor et al. (1985) Proc Natl. Acad. Sci. 82:1074]. In addition, a hybrid promoter can also be comprised of a bacteriophage promoter and an *E. coli* operator region (EP-A-0 267 851).

In addition to a functioning promoter sequence, an efficient ribosome binding site is also useful for the expression of foreign genes in prokaryotes. In *E. coli,* the ribosome binding site is called the Shine-Dalgarno (SD) sequence and includes an initiation codon (ATG) and a sequence 3-9 nucleotides in length located 3-11 nucleotides upstream of the initiation codon. The SD sequence is thought to promote binding of mRNA to the ribosome by the pairing of bases between the SD sequence and the 3' and of *E*. *coli* 16S rRNA [Steitz et al. (1979) "Genetic signals and nucleotide sequences in messenger RNA." In Biological Regulation and Development: Gene Expression (ed. R.F. Goldberger)]. To express eukaryotic genes and prokaryotic genes with weak ribosome-binding site [Sambrook et al. (1989) "Expression of cloned genes in Escherichia coli." In Molecular Cloning: A Laboratory Manual].

A promoter sequence may be directly linked with the DNA molecule, in which case the first amino acid at the N-terminus will always be a methionine, which is encoded by the ATG start codon. If desired, methionine at the N-terminus may be cleaved from the protein by *in vitro* incubation with cyanogen bromide or by either *in vivo* on *in vitro* incubation with a bacterial methionine N-terminal peptidase (EP-A-0219237).

Usually, transcription termination sequences recognized by bacteria are regulatory regions located 3' to the translation stop codon, and thus together with the promoter flank the coding sequence. These sequences direct the transcription of an mRNA which can be translated into the polypeptide encoded by the DNA. Transcription termination sequences frequently include DNA sequences of about 50 nucleotides capable of forming stem loop structures that aid in terminating transcription. Examples include transcription termination sequences derived from genes with strong promoters, such as the *trp* gene in *E. coli* as well as other biosynthetic genes.

Usually, the above described components, comprising a promoter, signal sequence (if desired), coding sequence of interest, and transcription termination sequence, are put together into expression constructs. Expression constructs are often maintained in a replicon, such as an extrachromosomal element (e.g. plasmids) capable of stable maintenance in a host, such as bacteria. The replicon will have a replication system, thus allowing it to be maintained in a prokaryotic host either for expression or for cloning and amplification. In addition, a replicon may be either a high or low copy number plasmid. A high copy number plasmid will generally have a copy number ranging from about 5 to about 200, and usually about 10 to about 150. A host containing a high copy number plasmid will preferably contain at least about 10, and more preferably at least about 20 plasmids. Either a high or low copy number vector may be selected, depending upon the effect of the vector and the foreign protein on the host.

Alternatively, the expression constructs can be integrated into the bacterial genome with an integrating vector. Integrating vectors usually contain at least one sequence homologous to the bacterial chromosome that allows the vector to integrate. Integrations appear to result from recombinations between homologous DNA in the vector and the bacterial chromosome. For example, integrating vectors constructed with DNA from various Bacillus strains integrate into the Bacillus chromosome (EP-A-0127328). Integrating vectors may also be comprised of bacteriophage or transposon sequences.

Usually, extrachromosomal and integrating expression constructs may contain selectable markers to allow for the selection of bacterial strains that have been transformed. Selectable markers can be expressed in the bacterial host and may include genes which render bacteria resistant to drugs such as ampicillin, chloramphenicol, erythromycin, kanamycin (neomycin), and tetracycline [Davies et al. (1978) Annu. Rev. Microbiol. 32:469]. Selectable markers may also include biosynthetic genes, such as those in the histidine, tryptophan, and leucine biosynthetic pathways.

Alternatively, some of the above described components can be put together in transformation vectors. Transformation vectors are usually comprised of a selectable marker that is either maintained in a replicon or developed into an integrating vector, as described above.

Expression and transformation vectors, either extra-chromosomal replicons or integrating vectors, have been developed for transformation into many bacteria. For example, expression vectors have been developed for, *inter alia,* the following bacteria: *Bacillus subtilis* [Palva et al. (1982) Proc. Natl. Acad. Sci. USA 79:5582; EP-A-0036259 and EP-A-0063953; WO84/04541], *Escherichia coli* [Shimatake et al. (1981) Nature 292:128; Amann et al. (1985) Gene 40:183; Studier et al. (1986) J. Mol. Biol. 189:113; EP-A-0 036 776,EP-A-0 136 829 and EP-A-0 136 907], *Streptococcus cremoris* [Powell et al. (1988) Appl. Environ. Microbiol. 54:655]; *Streptococcus lividans* [Powell et al. (1988) Appl. Environ. Microbiol. 54:655], *Streptomyces lividans* [US patent 4,745,056].

Methods of introducing exogenous DNA into bacterial hosts are well-known in the art, and usually include either the transformation of bacteria treated with CaCl₂ or other agents, such as divalent cations and DMSO. DNA can also be introduced into bacterial cells by electroporation. Transformation procedures usually vary with the bacterial species to be transformed. See *e.g.* [Masson et al. (1989) FEMS Microbiol. Lett. 60:273; Palva et al. (1982) Proc. Natl. Acad. Sci. USA 79:5582; EP-A-0036259 and EP-A-0063953; WO84/04541, *Bacillus],* [Miller et al. (1988) Proc. Natl. Acad. Sci. 85:856; Wang et al. (1990) J. Bacteriol. 172:949*, Campylobacter*], [Cohen et al. (1973) Proc. Natl. Acad. Sci. 69:2110; Dower et al. (1988) Nucleic Acids Res. 16:6127; Kushner (1978) "An improved method for transformation of Escherichia coli with ColE1-derived plasmids. In Genetic Engineering: Proceedings of the International Symposium on Genetic Engineering (eds. H.W. Boyer and S. Nicosia); Mandel et al. (1970) J. Mol. Biol. 53:159; Taketo (1988) Biochim. Biophys. Acta 949:318; *Escherichia*], [Chassy et al. (1987) FEMS Microbiol. Lett. 44:173 *Lactobacillus*]; [Fiedler et al. (1988) Anal. Biochem 170:38*, Pseudomonas*]; [Augustin et al. (1990) FEMS Microbiol. Lett. 66:203, *Staphylococcus*], [Barany et al. (1980) J. Bacteriol. 144:698; Harlander (1987) "Transformation of Streptococcus lactis by electroporation, in: Streptococcal Genetics (ed. J. Ferretti and R. Curtiss III); Perry et al. (1981) Infect. Immun. 32:1295; Powell et al. (1988) Appl. Environ. Microbiol. 54:655; Somkuti et al. (1987) Proc. 4th Evr. Cong. Biotechnology 1:412, *Streptococcus].*

### Host cells

The invention provides a bacterium which expresses a polypeptide of the invention. The bacterium may be a meningococcus. The bacterium may constitutively express the polypeptide, but in some embodiments expression may be under the control of an inducible promoter. The bacterium may hyper-express the polypeptide (*cf.* ref.194). Expression of the polypeptide may not be phase variable.

The invention also provides outer membrane vesicles prepared from a bacterium of the invention. Also provided herein is a process for producing vesicles from a bacterium of the invention. Vesicles prepared from these strains preferably include the polypeptide of the invention, which should be in an immunoaccessible form in the vesicles *i.e.* an antibody which can bind to purified polypeptide of the invention should also be able to bind to the polypeptide which is present in the vesicles.

These outer membrane vesicles include any proteoliposomic vesicle obtained by disruption of or blebbling from a meningococcal outer membrane to form vesicles therefrom that include protein components of the outer membrane. Thus the term includes OMVs (sometimes referred to as 'blebs'), microvesicles (MVs [195]) and 'native OMVs' ('NOMVs' [196]).

MVs and NOMVs are naturally-occurring membrane vesicles that form spontaneously during bacterial growth and are released into culture medium. MVs can be obtained by culturing *Neisseria* in broth culture medium, separating whole cells from the smaller MVs in the broth culture medium *(e.g.* by filtration or by low-speed centrifugation to pellet only the cells and not the smaller vesicles), and then collecting the MVs from the cell-depleted medium (*e.g.* by filtration, by differential precipitation or aggregation of MVs, by high-speed centrifugation to pellet the MVs). Strains for use in production of MVs can generally be selected on the basis of the amount of MVs produced in culture *e.g.* refs. 197 & 198 describe *Neisseria* with high MV production.

OMVs are prepared artificially from bacteria, and may be prepared using detergent treatment (*e.g.* with deoxycholate), or by non-detergent means (*e.g.* see reference 199). Techniques for forming OMVs include treating bacteria with a bile acid salt detergent (*e.g.* salts of lithocholic acid, chenodeoxycholic acid, ursodeoxycholic acid, deoxycholic acid, cholic acid, ursocholic acid, *etc.,* with sodium deoxycholate [200 & 201] being preferred for treating *Neisseria*) at a pH sufficiently high not to precipitate the detergent [202]. Other techniques may be performed substantially in the absence of detergent [199] using techniques such as sonication, homogenisation, microfluidisation, cavitation, osmotic shock, grinding, French press, blending, *etc.* Methods using no or low detergent can retain useful antigens such as NspA [199]. Thus a method may use an OMV extraction buffer with about 0.5% deoxycholate or lower *e.g.* about 0.2%, about 0.1%, <0.05% or zero.

A useful process for OMV preparation is described in reference 203 and involves ultrafiltration on crude OMVs, rather than instead of high speed centrifugation. The process may involve a step of ultracentrifugation after the ultrafiltration takes place.

Vesicles for use with the invention can be prepared from any meningococcal strain. The vesicles will usually be from a serogroup B strain, but it is possible to prepare them from serogroups other than B (*e.g.* reference 202 discloses a process for serogroup A), such as A, C, W 135 or Y. The strain may be of any serotype (*e.g.* 1, 2a, 2b, 4, 14, 15, 16, *etc.*), any serosubtype, and any immunotype (*e.g.* L1; L2; L3; L3,3,7; L10; *etc.*)*.* The meningococci may be from any suitable lineage, including hyperinvasive and hypervirulent lineages *e.g.* any of the following seven hypervirulent lineages: subgroup I; subgroup III; subgroup IV-1; ET-5 complex; ET-37 complex; A4 cluster; lineage 3.

Bacteria of the invention may, in addition to encoding a polypeptide of the invention, have one or more further modifications. For instance, they may have a modified *fur* gene [204]. Reference 212 teaches that *nspA* expression should be up-regulated with concomitant *porA* and *cps* knockout, and these modifications may be used. Further knockout mutants of *N.meningitidis* for OMV production are disclosed in references 212 to 214. Reference 205 discloses the construction of vesicles from strains modified to express six different PorA subtypes. Mutant *Neisseria* with low endotoxin levels, achieved by knockout of enzymes involved in LPS biosynthesis, may also be used [206,207]. These or other mutants can all be used with the invention.

Thus a strain used with the invention may in some embodiments express more than one PorA subtype. 6-valent and 9-valent PorA strains have previously been constructed. The strain may express 2, 3, 4, 5, 6, 7, 8 or 9 of PorA subtypes: P1.7,16; P1.5-1,2-2; P1.19,15-1; P1.5-2,10; P1.12-1,13; P1.7-2,4; P1.22,14; P1.7-1,1 and/or P1.18-1,3,6. In other embodiments a strain may have been down-regulated for PorA expression e.g. in which the amount of PorA has been reduced by at least 20% *(e.g.* ≥30%, ≥40%, ≥50%, ≥60%, ≥70%, ≥80%, ≥90%, ≥95%, *etc.),* or even knocked out, relative to wild-type levels (*e.g.* relative to strain H44/76).

In some embodiments a strain may hyper-express (relative to the corresponding wild-type strain) certain proteins. For instance, strains may hyper-express NspA, protein 287 [208], fHBP [194], TbpA and/or TbpB [209], Cu,Zn-superoxide dismutase [209], HmbR, *etc.*

A gene encoding a polypeptide of the invention may be integrated into the bacterial chromosome or may be present in episomal form *e.g.* within a plasmid.

Advantageously for vesicle production, a meningococcus may be genetically engineered to ensure that expression of the polypeptide is not subject to phase variation. Methods for reducing or eliminating phase variability of gene expression in meningococcus are disclosed in reference 210. For example, a gene may be placed under the control of a constitutive or inducible promoter, or by removing or replacing the DNA motif which is responsible for its phase variability.

In some embodiments a strain may include one or more of the knockout and/or hyper-expression mutations disclosed in references 211 to 214. Preferred genes for down-regulation and/or knockout include: (a) Cps, CtrA, CtrB, CtrC, CtrD, FrpB, GalE, HtrB/MsbB, LbpA, LbpB, LpxK, Opa, Opc, PilC, PorB, SiaA, SiaB, SiaC, SiaD, TbpA, and/or TbpB [211]; (b) CtrA, CtrB, CtrC, CtrD, FrpB, GalE, HtrB/MsbB, LbpA, LbpB, LpxK, Opa, Opc, PhoP, PilC, PmrE, PmrF, SiaA, SiaB, SiaC, SiaD, TbpA, and/or TbpB [212]; (c) ExbB, ExbD, rmpM, CtrA, CtrB, CtrD, GalE, LbpA, LpbB, Opa, Opc, PilC, PorB, SiaA, SiaB, SiaC, SiaD, TbpA, and/or TbpB [213]; and (d) CtrA, CtrB, CtrD, FrpB, OpA, OpC, PilC, PorB, SiaD, SynA, SynB, and/or SynC [214].

Where a mutant strain is used, in some embodiments it may have one or more, or all, of the following characteristics: (i) down-regulated or knocked-out LgtB and/or GalE to truncate the meningococcal LOS; (ii) up-regulated TbpA; (iii) up-regulated NhhA; (iv) up-regulated Omp85; (v) up-regulated LbpA; (vi) up-regulated NspA; (vii) knocked-out PorA; (viii) down-regulated or knocked-out FrpB; (ix) down-regulated or knocked-out Opa; (x) down-regulated or knocked-out Opc; (xii) deleted *cps* gene complex. A truncated LOS can be one that does not include a sialyl-lacto-N-neotetraose epitope *e.g.* it might be a galactose-deficient LOS. The LOS may have no α chain.

Depending on the meningococcal strain used for preparing the vesicles, they may or may not include the strain's native fHBP antigen [215].

If LOS is present in a vesicle it is possible to treat the vesicle so as to link its LOS and protein components ("intra-bleb" conjugation [214]).

### General

The term "comprising" encompasses "including" as well as "consisting" *e.g.* a composition "comprising" X may consist exclusively of X or may include something additional *e.g.* X + Y.

The term "about" in relation to a numerical value *x* means, for example, *x*± 10%.

The word "substantially" does not exclude "completely" *e.g.* a composition which is "substantially free" from Y may be completely free from Y. Where necessary, the word "substantially" may be omitted from the definition of the invention.

"Sequence identity" is preferably determined by the Smith-Waterman homology search algorithm as implemented in the MPSRCH program (Oxford Molecular), using an affine gap search with parameters *gap open penalty=12* and *gap extension penalty=1.*

After serogroup, meningococcal classification includes serotype, serosubtype and then immunotype, and the standard nomenclature lists serogroup, serotype, serosubtype, and immunotype, each separated by a colon *e.g.* B:4:P1.15:L3,7,9. Within serogroup B, some lineages cause disease often (hyperinvasive), some lineages cause more severe forms of disease than others (hypervirulent), and others rarely cause disease at all. Seven hypervirulent lineages are recognised, namely subgroups I, III and IV-1, ET-5 complex, ET-37 complex, A4 cluster and lineage 3. These have been defined by multilocus enzyme electrophoresis (MLEE), but multilocus sequence typing (MLST) has also been used to classify meningococci [ref. 30]. The four main hypervirulent clusters are ST32, ST44, ST8 and ST 11 complexes.

In general, the invention does not encompass the various fHBP sequences specifically disclosed in references 4, 5, 7, 8, 9, 10, 11, 12, 13, 14 and 216.

### MODES FOR CARRYING OUT THE INVENTION

### Example 1

With the wild-type MC58 sequence (SEQ ID NO: 1) as a baseline, reference 15 prepared 72 modified fHBP sequences. These are SEQ ID NOs: 4 to 75. By a similar design route, the inventors now provide SEQ ID NOs: 77 and 78.

Polypeptides have been expressed in *E.coli* with an N-terminus methionine followed immediately by a SEQ ID NO amino acid sequence. The polypeptides have been combined with aluminium hydroxide adjuvant, sometimes with IC31™ included as well, and then used to immunise mice. Antisera from the mice have been tested in a bactericidal assay against a panel of meningococcal strains. The panel included strains from each of the three fHBP families. Wild-type MC58 polypeptide from families 1 and II were also used to immunise mice for comparison.

The polypeptide including SEQ ID NO: 78 gave particularly good results. Sera against this polypeptide were bactericidal against five different family I strains MC58, NM008, M4030, GB185, NZ) and four different family II strains (961-5945, M3153, C11, M2552). The titres were generally lower against the strains than when using a wild-type sequence from a particular family, but neither of the wild-type sequences showed good inter-family bactericidal activity. Thus the modifications effectively increase the cross-strain protection of fHBP.

### Example 2

Various of the modified fHBP sequences have been fused to: (i) each other; (ii) wild-type fHBP sequences; or (iii) other meningococcal antigens. These were expressed with or without a C-terminus poly-histidine tag and purified from *E.coli* using IMAC.

The fusion proteins fell into four main categories: (a) fusions of two modified fHBP sequences, which may be the same or different; (b) fusions of three modified fHBP sequences, which may be the same or different; (c) fusions of a wild-type fHBP sequence to one or two modified fHBP sequences; and (d) fusions of a modified fHBP sequence to a non-fHBP meningococcal antigen. These various fusion proteins comprise the following amino acid sequences:
*(a) fusions of two modified fHBP sequences, which may be the same or different*

| | |
|---|---|
| 9C-9C | SEQ ID NO: 132 |
| 10A-10A | SEQ ID NO: 134 |
| 10A-9C | SEQ ID NO: 135 |
| 8B-8B | SEQ ID NO: 138 |
| 9C-10A | SEQ ID NO: 133 |

*(b) fusions of three modified fHBP sequences, which may be the same or different*

| | |
|---|---|
| 10A-10A-10A | SEQ ID NO: 113 |
| 10A-10A-9C | SEQ ID NO: 117 |
| 10A-9C-10A | SEQ ID NO: 112 |
| 10A-9C-9C | SEQ ID NO: 118 |
| 8B-8B-8B | SEQ ID NO: 123 |
| 9C-10A-10A | SEQ ID NO: 105 |
| 9C-10A-9C | SEQ ID NO: 108 |
| 9C-9C-10A | SEQ ID NO: 104 |
| 9C-9C-9C | SEQ ID NO: 107 |

*(c) fusions of a wild-type fHBP sequence to one or two modified fHBP sequences*

| | |
|---|---|
| 10A-MC58 | SEQ ID NO: 131 |
| 10A-10A-MC58 | SEQ ID NO: 114 |
| 10A-MC58-10A | SEQ ID NO: 115 |
| 10A-MC58-9C | SEQ ID NO: 116 |
| 10A-9C-MC58 | SEQ ID NO: 111 |
| MC58-10A | SEQ ID NO: 137 |
| MC58-10A-10A | SEQ ID NO: 121 |
| MC58-10A-9C | SEQ ID NO: 119 |
| MC58-9C | SEQ ID NO: 136 |
| MC58-9C-10A | SEQ ID NO: 120 |
| MC58-9C-9C | SEQ ID NO: 122 |
| 9C-10A-MC58 | SEQ ID NO: 109 |
| 9C-MC58 | SEQ ID NO: 130 |
| 9C-MC58-10A | SEQ ID NO: 106 |
| 9C-MC58-9C | SEQ ID NO: 110 |
| 9C-9C-MC58 | SEQ ID NO: 103 |

*(d) fusions of a modified fHBP sequence to a non-fHBP meningococcal antigen. For example:*

| | |
|---|---|
| 936-10A-10A | SEQ ID NO: 126 |
| 936-10A-9C | SEQ ID NO: 125 |
| 936-9C-10A | SEQ ID NO: 124 |
| 936-9C-9C | SEQ ID NO: 127 |
| 936-10A | SEQ ID NO: 129 |
| 936-9C | SEQ ID NO: 128 |

*NB:* The 10A sequence is SEQ IS NO: 23; the 9C sequence is SEQ ID NO: 20; the 8B sequence is SEQ ID NO: 17; the MC58 sequence is SEQ ID NO: 97 (*i.e.* amino acids 27-274 of SEQ ID NO: 1); and the 936 sequence is SEQ ID NO: 98, including its own N-terminus methionine.

For example, to make the 9C-9C fusion the sequence encoding PATCH_9C (SEQ ID NO: 20) was linked via a *Bam*HI restriction site and a glycine linker (thus encoding SEQ ID NO: 81) to a second copy of the coding sequence, followed by a *Xho*I restriction site and a C-terminus hexa-histidine tag (SEQ ID NO: 95). An upstream sequence provided an N-terminus methionine, giving the following final expressed 511-mer sequence (SEQ ID NO: 99, comprising SEQ ID NO: 132):

MVAADIGAGLADALTAPLDHKDKGLQSLTLDQSVRKNEKLKLAAQGAEKTYGNGDSLNTGKLKNDK
VSRFDFIRQIEVDGQLITLESGEFQVYKQSHSALTAFQTEQIQDSEHSGKMVAKRQFRIGDIAGEH
TSFDKLPEGGRATYHGKAFGSDDPNGRLHYTIDFAAKQGYGRIEHLKTPEQNVDLAAADIKPDGKR
HAVISGSVLYNQAEKGSYSLGIFGGKAQEVAGSAEVKIGEGIRHIGLAAKQGSGGGGVAADIGAGL
ADALTAPLDHKDKGLQSLTLDQSVRKNEKLKLAAQGAEKTYGNGDSLNTGKLKNDKVSRFDFIRQI
EVDGQLITLESGEFQVYKQSHSALTAFQTEQIQDSEHSGKMVAKRQFRIGDIAGEHTSFDKLPEGG
RATYHGKAFGSDDPNGRLHYTIDFAAKQGYGRIEHLKTPEQNVDLAAADIKPDGKRHAVISGSVLY
NQAEKGSYSLGIFGGKAQEVAGSAEVKIGEGIRHIGLAAKQLEHHHHHH

Similarly, to make the 10A-10A-10A fusion, three sequences encoding PATCH_10A (SEQ ID NO: 23) were linked via a *Bam*HI restriction site and a glycine linker (thus encoding SEQ ID NO: 81), followed by a *Xho*I restriction site and a C-terminus hexa-histidine tag (SEQ ID NO: 95). An upstream sequence provided an N-terminus methionine, giving the following final expressed 762-mer sequence (SEQ ID NO: 100, comprising SEQ ID NO: 113):

MVAADIGAGLADALTAPLDHKDKGLQSLTLDQSVRKNEKLKLAAQGAEKTYGNGDSLNTGKLKNDK
VSRFDFIRQIEVDGQLITLESGEFQVYKQSHSALTAFQTEQIQDSEHSGKMVAKRQFRIGDLGGEH
TAFNQLPDGKAEYRGTAFGSDDAGGKLTYTIDFTKKQGNGKIEHLKSPELNVELASAEIKADGKSH
AVILGDVRYGSEEKGSYSLGIFGGRAQEVAGSAEVKTVNGIRHIGLAAKQGSGGGGVAADIGAGLA
DALTAPLDHKDKGLQSLTLDQSVRKNEKLKLAAQGAEKTYGNGDSLNTGKLKNDKVSRFDFIRQIE
VDGQLITLESGEFQVYKQSHSALTAFQTEQIQDSEHSGKMVAKRQFRIGDLGGEHTAFNQLPDGKA
EYRGTAFGSDDAGGKLTYTIDFTKKQGNGKIEHLKSPELNVELASAEIKADGKSHAVILGDVRYGS
EEKGSYSLGIFGGRAQEVAGSAEVKTVNGIRHIGLAAKQGSGGGGVAADIGAGLADALTAPLDHKD
KGLQSLTLDQSVRKNEKLKLAAQGAEKTYGNGDSLNTGKLKNDKVSRFDFIRQIEVDGQLITLESG
EFQVYKQSHSALTAFQTEQIQDSEHSGKMVAKRQFRIGDLGGEHTAFNQLPDGKAEYRGTAFGSDD
AGGKLTYTIDFTKKQGNGKIEHLKSPELNVELASAEIKADGKSHAVILGDVRYGSEEKGSYSLGIF
GGRAQEVAGSAEVKTVNGIRHIGLAAKQLEHHHHHH

Similarly, to make the 10A-MC58 fusion the sequence encoding PATCH_10A (SEQ ID NO: 23) was linked via a *Bam*HI restriction site and a glycine linker (thus encoding SEQ ID NO: 81) to the MC58 sequence (encoding SEQ ID NO: 97), followed by a *Xho*I restriction site and a C-terminus hexa-histidine tag (SEQ ID NO: 95). An upstream sequence provided an N-terminus methionine, giving an expressed 509-mer sequence (SEQ ID NO: 101, comprising SEQ ID NO: 131):

MVAADIGAGLADALTAPLDHKDKGLQSLTLDQSVRKNEKLKLAAQGAEKTYGNGDSLNTGKLKNDK
VSRFDFIRQIEVDGQLITLESGEFQVYKQSHSALTAFQTEQIQDSEHSGKMVAKRQFRIGDLGGEH
TAFNQLPDGKAEYRGTAFGSDDAGGKLTYTIDFTKKQGNGKIEHLKSPELNVELASAEIKADGKSH
AVILGDVRYGSEEKGSYSLGIFGGRAQEVAGSAEVKTVNGIRHIGLAAKQGSGGGVAADIGAGLA
DALTAPLDHKDKGLQSLTLDQSVRKNEKLKLAAQGAEKTYGNGDSLNTGKLKNDKVSRFDFIRQIE
VDGQLITLESGEFQVYKQSHSALTAFQTEQIQDSEHSGKMVAKRQFRIGDIAGEHTSFDKLPEGGR
ATYRGTAFGSDDAGGKLTYTIDFAAKQGNGKIEHLKSPELNVDLAAADIKPDGKRHAVISGSVLYN
QAEKGSYSLGIFGGKAQEVAGSAEVKTVNGIRHIGLAAKQLEHHHHHH

Similarly, to make the 936-9C fusion the sequence encoding 936 (SEQ ID NO: 98) was linked via a *Bam*HI restriction site and a glycine linker (thus encoding SEQ ID NO: 81) to the MC58 sequence (encoding SEQ ID NO: 97), followed by a *Xho*I restriction site and a C-terminus hexa-histidine tag (SEQ ID NO: 95). An upstream sequence provided an N-terminus methionine, giving the following final expressed 442-mer sequence (SEQ ID NO: 102, comprising SEQ ID NO: 128):

MVSAVIGSAAVGAKSAVDRRTTGAQTDDNVMALRIETTARSYLRQNNQTKGYTPQISVVGYDRHLL
LLGQVATEGEKQFVGQIARSEQAAEGVYNYITVASLPRTAGDIAGDTWNTSKVRATLLGISPATRA
RVKIVTYGNVTYVMGILTPEEQAQITQKVSTTVGVQKVITLYQNYVQRGSGGGGVAADIGAGLADA
LTAPLDHKDKGLQSLTLDQSVRKNEKLKLAAQGAEKTYGNGDSLNTGKLKNDKVSRFDFIRQIEVD
GQLITLESGEFQVYKQSHSALTAFQTEQIQDSEHSGKMVAKRQFRIGDIAGEHTSFDKLPEGGRAT
YHGKAFGSDDPNGRLHYTIDFAAKQGYGRIEHLKTPEQNVDLAAADIKPDGKRHAVISGSVLYNQA
EKGSYSLGIFGGKAQEVAGSAEVKIGEGIRHIGLAAKQLEHHHHHH

Fusion proteins were used to immunise mice. For comparison, the following antigens were also used: wild-type fHBP from strain MC58 or 2996; modified fHBP 9C or 10A; and a hybrid of families I, II and III as disclosed in reference 13 were also used, The resulting sera were tested for bactericidal activity against a panel of strains from fHBP families I, II and III. Titres were as follows after two immunisations using a mixture of alum+IC31™ as adjuvant:

| **fHBP family** | **I** | | | | | **II** | | | | **III** |
|---|---|---|---|---|---|---|---|---|---|---|
| | **MC58** | **NM008** | **M4030** | **GB195** | **NZ** | **5945** | **M3153** | **C11** | **M2552** | **M1239** |
| MC58 | >8192* | 512 | 2048 | <16 | 64 | <64 | <64 | <64 | <16 | 32 |
| 2996 | <64 | <16 | <16 | <16 | <64 | 4096 | 512 | 64 | 256 | 128 |
| 10A | 1024 | <16 | 1024 | <16 | 64 | 64 | <64 | <64 | <16 | <16 |
| 9C | 8192 | 256 | 2048 | <16 | <64 | 512 | <64 | <64 | <16 | <16 |
| 936-10A | 8192 | 256 | 4096 | 1024 | 512 | 8192 | 2048 | 128 | 512 | 64 |
| 10A-MC58 | >32768 | 2048 | >8192 | 2048 | 2048 | 8192 | 1024 | 256 | 512 | 512 |
| 936-9C-10A | 16384 | 256 | 8192 | 2048 | 4096 | 8192 | 1024 | 512 | 1024 | 256 |
| 9C-9C-MC58 | 16384 | 512 | 4096 | 64 | 64 | 256 | <64 | <64 | <16 | <16 |
| 9C-10A-9C | >32768 | 1024 | 8192 | 4096 | 4096 | 8192 | 2048 | 512 | 1024 | 256 |
| 10A-9C-10A | 8192* | 2048 | >8192 | 512 | 1024 | 8192 | 2048 | <32 | 512 | 1024 |
| 10A-10A-10A | 2048 | 16 | 2048 | 1024 | 1024 | 8192 | 2048 | 256 | 512 | 512 |
| II-III-I | >32768 | >16384 | >8192 | 2048 | 1024 | 16384 | 4096 | 512 | 512 | 1024 |

Whereas the MC58 (family I) and 2996 (family II) sequences show efficacy only against the strains with homologous fHBP, cross-strain efficacy is much improved with fusion proteins of the invention. Furthermore, the efficacy of the modified 9C and 10A sequences is improved by fusing them to 936 or to the wild-type MC58 fHBP sequence. The 9C-10A-9C fusion shows very good results across the complete panel.

Fusing the modified sequences to other antigens thus offers a general way of improving their ability to elicit anti-meningococcal immune responses.

### Adjuvant study

The PATCH_2S, PATCH_5bis, PATCH_5penta, PATCH_9C, PATCH_9F and PATCH_10A polypeptides, together with the wild-type fHBP sequence from strain 2996, were used to immunise mice with aluminium hydroxide (Al-H) and/or IC31™ adjuvant(s). Sera were tested against a panel of ten different meningococcal strains.

The combination of Al-H+IC31™ gave better results than Al-H alone when tested with fusion proteins containing PATCH_9C and/or PATCH_10A and/or the wild-type MC58 fHBP sequence and/or 936 antigen e.g. converting efficacy against only 1/10 strains with Al-H into efficacy against 9/10 strains when using a fusion protein containing 936 fused to two copies of PATCH_10A (SEQ ID NO: 18).

Using the 936-10A-10A sequence or a 936-9C-10A sequence bactericidal titers against a panel of 10 strains were:

| | **936-10A-10A** | | **936-9C-10A** | |
|---|---|---|---|---|
| | **Al-H** | **Al-H + IC31** | **Al-H** | **Al-H + IC31** |
| **A** | 8192 | ≥2768 | 8192 | ≥32768 |
| **B** | <16 | 4096 | 128 | 2048 |
| **C** | 512 | 8192 | 1024 | ≥8192 |
| **D** | 256 | 2048 | 512 | 4096 |
| **E** | 16 | 1024 | 256 | 4096 |
| **F** | 64 | 8192 | 2048 | 16384 |
| **G** | 128 | 2048 | 1024 | 4096 |
| **H** | 16 | 1024 | 1024 | 4096 |
| **I** | 64 | 4096 | 128 | 2048 |
| **J** | 64 | 32 | 256 | 1024 |

With one exception, therefore, the addition of IC31 improved titers.

### 936-10A-10A

The 936-10A-10A fusion was selected for further studies (*i.e*. SEQ ID NO: 126). This polypeptide was formulated with Al-H in a composition including 9mg/ml NaCl and 10mM histidine, pH 6.5. Water for injection and histidine buffer were mixed, and then NaCl was added to give a final osmolality of 308 mOsm/kg. Al-H was added to give a final Al⁺⁺⁺ concentration of 3mg/ml. The polypeptide was added to give a final concentration of 100µg/ml, left for 15 minutes under stirring at room temperature, and then stored overnight at 4°C. Just before administration, IC31™ (with a 25:1 molar ratio of peptide:DNA, 1µmol peptide) was added as an aqueous suspension, mixing equal volumes. The final mixture was isotonic and at physiological pH. Polypeptide adsorption was >90% (similar to the level seen with the Al-H alone).

Animals (6-week-old CD1 female mice), 8 per group, received 20µg adjuvanted polypeptide intraperitoneally at day 0, with booster doses at days 21 & 35. Blood samples for analysis were taken on day 49 and were analysed by bactericidal assay, in the presence of rabbit or human complement, against a panel of 11 meningococcal strains. Titers were as follows:

| | **Rabbit complement** | **Human complement** |
|---|---|---|
| **A** | 16384 | 1024 |
| **B** | 2048 | 512 |
| **C** | 4096 | >512 |
| **D** | 4096 | 256 |
| **E** | 4096 | 256 |
| **F** | 8192 | 64 |
| **G** | 4096 | 256 |
| **H** | 1024* | 256 |
| **I** | 4096 | - |
| **J** | 1024 | 256 |
| **K** | - | 512 |

| | | |
|---|---|---|
| * = bacteriostatic titer | | |

Similar results were seen whether the 936-10A-10A polypeptide did or did not have a C-terminus polyhistidine tag but, depending on the adjuvant which was used, a better titer was seen sometimes seen when using the 936-10A-10A polypeptide with a histidine tag.

The 936-10A-10A polypeptide was substituted for the '936-fHBP' polypeptide in the '5CVMB' vaccine disclosed in reference 108, to give a mixture of three polypeptides having amino acid sequences of SEQ ID NOs: 90, 139 and 126. This mixture is referred to below as '5CVMB*'. Bactericidal titers were similar but, as above, depending on the adjuvant which was used, a better titer was sometimes seen when using the 936-10A-10A polypeptide with a histidine tag.

A 5CVMB* mixture in which 936-10A-10A had a C-terminus histidine purification tag was combined with 2.5µg (measured as protein) of outer membrane vesicles from the MeNZB™ vaccine. This mixture ('5CVMB*+¼MV') was compared to 5CVMB* alone, to 5CVMB, or to 5CVMB in combination with 10µg of the OMVs. Sera obtained after immunisation with these four compositions were tested for bactericidal activity against a panel of 13 strains. the replacement of 5CVMB's 936-fHBP polypeptide with 936-10A-10A improved strain coverage, in the presence or absence of OMVs: the percentage of strains for which the bactericidal titer was ≥1024 was seven percentage points higher with 5CVMB* compared to 5CVMB, and was 15 percentage points higher with 5CVMB*+¼OMV compared to 5CVMB+OMV. Different results were seen using a different panel of strains.

### NL096 hybrids

Reference 15 discloses a fHBP sequence called 'NL096' (SEQ ID NO: 76 herein). This fHBP protein can provide sera which are bactericidal across almost all of a panel of 11 strains and, when using an aluminium hydroxide adjuvant, the strain coverage achieved by NL096 is superior to the coverage achieved by PATCH_10A.

Hybrids of the NL096 sequence have thus been designed, including:

| | |
|---|---|
| 936-10A-NL096 | SEQ ID NO: 140 |
| 936-NL096-P10A | SEQ ID NO: 141 |
| 936-NL096-NL096 | SEQ ID NO: 142 |

Each of these three sequences can be expressed with an N-terminus sequence (e.g. with a single methionine residue) and/or with a C-terminus histidine tag *e.g.* to add SEQ ID NO: 96 at the C-terminus.

### REFERENCES

[1] Jodar et al. (2002) Lancet 359(9316):1499-1508.
[2] Pizza et al. (2000) Science 287:1816-1820.
[3] WO99/57280.
[4] Masignani et al. (2003) J Exp Med 197:789-799.
[5] Welsch et al. (2004) J Immunol 172:5605-15.
[6] Hou et al. (2005) J Infect Dis 192(4):580-90.
[7] WO03/063766.
[8] Fletcher et al. (2004) Infect Immun 72:2088-2100.
[9] Zhu et al. (2005) Infect Immun 73(10):6838-45.
[10] WO01/64920.
[11] WO03/020756.
[12] WO2004/048404.
[13] WO2006/024954.
[14] WO2007/060548.
[15] WO2009/104097.
[16] Tettelin et al. (2000) Science 287:1809-1815.
[17] WO00/66741.
[18] WO99/57280
[19] Martin et al. (1997) J Exp Med 185(7): 1173-83.
[20] WO96/29412.
[21] Perkins-Balding et al. (2003) Microbiology 149:3423-35.
[22] WO01/55182.
[23] WO01/38350.
[24] WO00/23595.
[25] Tettelin et al. (2000) Science 287:1809-1815.
[26] Needleman & Wunsch (1970) J. Mol. Biol. 48, 443-453.
[27] Rice et al. (2000) Trends Genet 16:276-277.
[28] Achtman (1995) Global epidemiology of meningococcal disease. Pages 159-175 of Meningococcal disease (ed. Cartwight). ISBN: 0-471-95259-1.
[29] Caugant (1998) APMIS 106:505-525.
[30] Maiden et al. (1998) Proc. Natl. Acad. Sci. USA 95:3140-3145.
[31] WO01/30390.
[32] Gennaro (2000) Remington: The Science and Practice of Pharmacy. 20th edition, ISBN: 0683306472.
[33] WO03/009869.
*[34]* Vaccine Design... (1995) eds. Powell & Newman. ISBN: 030644867X. Plenum.
[35] WO00/23105.
[36] WO90/14837.
[37] WO90/14837.
[38] Podda & Del Giudice (2003) Expert Rev Vaccines 2:197-203.
[39] Podda (2001) Vaccine 19: 2673-2680.
[40] Vaccine Design: The Subunit and Adjuvant Approach (eds. Powell & Newman) Plenum Press 1995 (ISBN 0-306-44867-X).
[41] Vaccine Adjuvants: Preparation Methods and Research Protocols (Volume 42 of Methods in Molecular Medicine series). ISBN: 1-59259-083-7. Ed. O'Hagan.
[42] US 5,057,540.
[43] WO96/33739.
[44] EP-A-0109942.
[45] WO96/11711.
[46] WO00/07621.
[47] Barr et al. (1998) Advanced Drug Delivery Reviews 32:247-271.
[48] Sjolanderet et al. (1998) Advanced Drug Delivery Reviews 32:321-338.
[49] Niikura et al. (2002) Virology 293:273-280.
[50] Lenz et al. (2001) J Immunol 166:5346-5355.
[51] Pinto et al. (2003) J Infect Dis 188:327-338.
[52] Gerber et al. (2001) J Virol 75:4752-4760.
[53] WO03/024480.
[54] WO03/024481.
[55] Gluck et al. (2002) Vaccine 20:B10-B16.
[56] EP-A-0689454.
[57] Johnson et al. (1999) Bioorg Med Chem Lett 9:2273-2278.
[58] Evans et al. (2003) Expert Rev Vaccines 2:219-229.
[59] Meraldi et al. (2003) Vaccine 21:2485-2491.
[60] Pajak et al. (2003) Vaccine 21:836-842.
[61] Kandimalla et al. (2003) Nucleic Acids Research 31:2393-2400.
[62] WO02/26757.
[63] WO99/62923.
[64] Krieg (2003) Nature Medicine 9:831-835.
[65] McCluskie et al. (2002) FEMS Immunology and Medical Microbiology 32:179-185.
[66] WO98/40100.
[67] US 6,207,646.
[68] US 6,239,116.
[69] US 6,429,199.
[70] Kandimalla et al. (2003) Biochemical Society Transactions 31 (part 3):654-658.
[71] Blackwell et al. (2003) J Immunol 170:4061-4068.
[72] Krieg (2002) Trends Immunol 23:64-65.
[73] WO01/95935.
[74] Kandimalla et al. (2003) BBRC 306:948-953.
[75] Bhagat et al. (2003) BBRC 300:853-861.
[76] WO03/035836.
[77] Schellack et al. (2006) Vaccine 24:5461-72.
[78] WO95/17211.
[79] WO98/42375.
[80] Beignon et al. (2002) Infect Immun 70:3012-3019.
[81] Pizza et al. (2001) Vaccine 19:2534-2541.
[82] Pizza et al. (2000) Int J Med Microbiol 290:455-461.
[83] Scharton-Kersten et al. (2000) Infect Immun 68:5306-5313.
[84] Ryan et al. (1999) Inject Immun 67:6270-6280.
[85] Partidos et al. (1999) Immunol Lett 67:209-216.
[86] Peppoloni et al. (2003) Expert Rev Vaccines 2:285-293.
[87] Pine et al. (2002) J Control Release 85:263-270.
[88] Tebbey et al. (2000) Vaccine 18:2723-34.
[89] Domenighini et al. (1995) Mol Microbiol 15:1165-1167.
[90] WO99/40936.
[91] WO99/44636.
[92] Singh et al] (2001) J Cont Release 70:267-276.
[93] WO99/27960.
[94] US 6,090,406.
[95] US 5,916,588.
[96] EP-A-0626169.
[97] WO99/52549.
[98] WO01/21207.
[99] WO01/21152.
[100] Andrianov et al. (1998) Biomaterials 19:109-115.
[101] Payne et al. (1998) Adv Drug Delivery Review 31:185-196.
[102] Stanley (2002) Clin Exp Dermatol 27:571-577.
[103] Jones (2003) Curr Opin Investig Drugs 4:214-218.
[104] WO99/11241.
[105] WO94/00153.
[106] WO98/57659.
[107] European patent applications 0835318, 0735898 and 0761231.
[108] Giuliani et al. (2006) Proc Natl Acad Sci U S A. 103:10834-9.
[109] WO2004/032958.
[110] WO99/24578.
[111] WO99/36544.
[112] Costantino et al. (1992) Vaccine 10:691-698.
[113] Costantino et al. (1999) Vaccine 17:1251-1263.
[114] WO03/007985.
[115] Watson (2000) Pediatr Infect Dis J 19:331-332.
[116] Rubin (2000) Pediatr Clin North Am 47:269-285, v.
[117] Jedrzejas (2001) Microbiol Mol Biol Rev 65:187-207.
[118] Bell (2000) Pediatr Infect Dis J 19:1187-1188.
[119] Iwarson (1995) APMIS 103:321-326.
[120] Gerlich et al. (1990) Vaccine 8 Suppl:S63-68 & 79-80.
[121] Vaccines (1988) eds. Plotkin & Mortimer. ISBN 0-7216-1946-0.
[122] Del Guidice et al. (1998) Molecular Aspects of Medicine 19:1-70.
[123] Gustafsson et al. (1996) N. Engl. J. Med. 334:349-355.
[124] Rappuoli et al. (1991) TIBTECH 9:232-238.
[125] Sutter et al. (2000) Pediatr Clin North Am 47:287-308.
[126] Zimmerman & Spann (1999) Am Fam Physician 59:113-118, 125-126.
[127] McMichael (2000) Vaccine 19 Suppl 1:S101-107.
[128] Schuchat (1999) Lancet 353(9146):51-6.
[129] WO02/34771.
[130] Dale (1999) Infect Dis Clin North Am 13:227-43, viii.
[131] Ferretti et al. (2001) PNAS USA 98: 4658-4663.
[132] Kuroda et al. (2001) Lancet 357(9264):1225-1240; see also pages 1218-1219.
[133] Jones (2001) Curr Opin Investig Drugs 2:47-49.
[134] Ravenscroft et al. (1999) Vaccine 17:2802-2816.
[135] WO03/080678.
[136] Research Disclosure, 453077 (Jan 2002).
[137] EP-A-0372501.
[138] EP-A-0378881.
[139] EP-A-0427347.
[140] WO93/17712.
[141] WO94/03208.
[142] WO98/58668.
[143] EP-A-0471177.
[144] WO91/01146.
[145] Falugi et al. (2001) Eur J Immunol 31:3816-3824.
[146] Baraldo et al. (2004) Infect Immun 72(8):4884-7.
[147] EP-A-0594610.
[148] Ruan et al. (1990) J Immunol 145:3379-3384.
[149] WO00/56360.
[150] Kuo et al. (1995) Infect Immun 63:2706-13.
[151] Michon et al. (1998) Vaccine. 16:1732-41.
[152] WO02/091998.
[153] WO01/72337.
[154] WO00/61761.
[155] WO00/33882
[156] Lees et al. (1996) Vaccine 14:190-198.
[157] WO95/08348.
[158] US patent 4,882,317
[159] US patent 4,695,624
[160] Porro et al. (1985) Mol Immunol 22:907-919.s
[161] EP-A-0208375
[162] WO00/10599
[163] Gever et al. Med. Microbiol. Immunol, 165 : 171-288 (1979).
[164] US patent 4,057,685.
[165] US patents 4,673,574; 4,761,283; 4,808,700.
[166] US patent 4,459,286.
[167] US patent 4,965,338
[168] US patent 4,663,160.
[169] US patent 4,761,283
[170] US patent 4,356,170
[171] WO02/09643.
[172] Katial et al. (2002) Infect Immun 70:702-707.
[173] WO01/52885.
[174] European patent 0301992.
[175] Bjune et al. (1991) Lancet 338(8775):1093-1096.
[176] Fukasawa et al. (1999) Vaccine 17:2951-2958.
[177] WO02/09746.
[178] Rosenqvist et al. (1998) Dev. Biol. Stand. 92:323-333.
[179] WO01/09350.
[180] European patent 0449958.
[181] EP-A-0996712.
[182] EP-A-0680512.
[183] WO02/062378.
[184] WO99/59625.
[185] US patent 6,180,111.
[186] WO01/34642.
[187] WO03/051379.
[188] US patent 6,558,677.
[189] WO2004/019977.
[190] WO02/062380.
[191] WO00/25811.
[192] Peeters et al. (1996) Vaccine 14:1008-1015.
[193] Vermont et al. (2003) Infect Immun 71:1650-1655.
[194] WO2006/081259.
[195] WO02/09643.
[196] Katial et al. (2002) Infect. Immun. 70:702-707.
[197] US patent 6,180,111.
[198] WO01/34642.
[199] WO2004/019977.
[200] European patent 0011243.
[201] Fredriksen et al. (1991) NIPH Ann. 14(2):67-80.
[202] WO01/91788.
[203] WO2005/004908.
[204] WO98/56901.
[205] Claassen *et al.* (1996) 14(10):1001-8.
[206] WO99/10497.
[207] Steeghs et al. (2001) The EMBO Journal 20:6937-6945.
[208] WO01/52885.
[209] WO00/25811.
[210] WO2004/015099.
[211] WO01/09350.
[212] WO02/09746.
[213] WO02/062378.
[214] WO2004/014417.
[215] WO2004/046177.
[216] WO2004/094596

**ALTERNATIVE NAMES FOR SEQUENCES 1 to 78 IN THE SEQUENCE LISTING**

| **SEQ ID NO:** | **Description** | **SEQ ID NO:** | **Description** |
|---|---|---|---|
| 1 | fHBP, strain MC58-family I | 40 | PATCH_11L |
| 2 | fHBP, strain 961-5945 & 2996 - family II | 41 | PATCH_12 |
| 3 | fHBP, strain M1239-family III | 42 | PATCH_12B |
| 4 | LOOP2 | 43 | PATCH_12C |
| 5 | PATCH_1 | 44 | PATCH_12D |
| 6 | PATCH_2 | 45 | PATCH_12E |
| 7 | PATCH_2S | 46 | PATCH_12F |
| 8 | PATCH_2T | 47 | PATCH_12G |
| 9 | PATCH_2FAT | 48 | PATCH_12H |
| 10 | PATCH_3 | 49 | PATCH_12I |
| 11 | PATCH_5 | 50 | PATCH_12L |
| 12 | PATCH_5bis | 51 | PATCH_12M |
| 13 | PATCH_5tris | 52 | PATCH_12N |
| 14 | PATCH_5tetra | 53 | PATCH_13 |
| 15 | PATCH_5penta | 54 | PATCH_13B |
| 16 | PATCH_8 | 55 | PATCH_13C |
| 17 | PATCH_8B | 56 | PATCH_14 |
| 18 | PATCH_9 | 57 | PATCH_14B |
| 19 | PATCH_9B | 58 | PATCH_14C |
| 20 | PATCH_9C | 59 | PATCH_14D |
| 21 | PATCH_9D | 60 | PATCH_15A |
| 22 | PATCH_9E | 61 | PATCH_15B |
| 23 | PATCH_10A | 62 | PATCH_16A |
| 24 | PATCH_10B | 63 | PATCH_16B |
| 25 | PATCH_10C | 64 | PATCH_16C |
| 26 | PATCH_10D | 65 | PATCH_16D |
| 27 | PATCH_10E | 66 | PATCH_16E |
| 28 | PATCH_10F | 67 | PATCH_16F |
| 29 | PATCH_10G | 68 | PATCH_16G |
| 30 | PATCH_10H | 69 | PATCH_17A |
| 31 | PATCH_11 | 70 | PATCH_17B |
| 32 | PATCH_11B | 71 | PATCH_17C |
| 33 | PATCH_11C | 72 | PATCH_18A |
| 34 | PATCH_11D | 73 | PATCH_18B |
| 35 | PATCH_11E | 74 | PATCH_18C |
| 36 | PATCH_11F | 75 | PATCH_18D |
| 37 | PATCH_11G | 76 | NL096 |
| 38 | PATCH_11H | 77 | PATCH_19 |
| 39 | PATCH_11I | 78 | PATCH_20 |

### SEQUENCE LISTING

***SEQ ID NO: 1*** 
MNRTAFCCLSLTTALILTACSSGGGGVAADIGAGLADALTAPLDHKDKGLQSLTLDQSVRKNEKLKLAAQGAEKTYGNGD
SLNTGKLKNDKVSRFDFIRQIEVDGQLITLESGEFQVYKQSHSALTAFQTEQIQDSEHSGKMVAKRQFRIGDIAGEHTSF
DKLPEGGRATYRGTAFGSDDAGGKLTYTIDFAAKQGNGKIEHLKSPELNVDLAAADIKPDGKRHAVISGSVLYNQAEKGS
YSLGIFGGKAQEVAGSAEVKTVNGIRHIGLAAKQ
***SEQ ID NO: 2*** 
MNRTAFCCLSLTAALILTACSSGGGGVAADIGAGLADALTAPLDHKDKSLQSLTLDQSVRKNEKLKLAAQGAEKTYGNGD
SLNTGKLKNDKVSRFDFIRQIEVDGQLITLESGEFQIYKQDHSAVVALQIEKINNPDKIDSLINQRSFLVSGLGGEHTAF
NQLPDGKAEYHGKAFSSDDAGGKLTYTIDFAAKQGHGKIEHLKTPEQNVELAAAELKADEKSHAVILGDTRYGSEEKGTY
HLALFGDRAQEIAGSATVKIGEKVHEIGIAGKQ
***SEQ ID NO: 3*** 
MNRTAFCCLSLTTALILTACSSGGGGSGGGGVAADIGTGLADALTAPLDHKDKGLKSLTLEDSIPQNGTLTLSAQGAEKT
FKAGDKDNSLNTGKLKNDKISRFDFVQKIEVDGQTITLASGEFQIYKQNHSAVVALQIEKINNPDKTDSLINQRSFLVSG
LGGEHTAFNQLPGGKAEYHGKAFSSDDPNGRLHYSIDFTKKQGYGRIEHLKTLEQNVELAAAELKADEKSHAVILGDTRY
GSEEKGTYHLALFGDRAQEIAGSATVKIGEKVHEIGIAGKQ
***SEQ ID NO: 4*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQINNPDKIDSMVAKRQFRIGDIAGEHTSFDQLPDGKRATYRGTAFGSDDAGGKLT
YTIDFAAKQGNGKIEHLKSPELNVDLAAADIKPDGKRHAVISGSVLYNQAEKGSYSLGIFGGKAQEVAGSAEVKTVNGIR
HIGLAAKQLE
***SEQ ID NO: 5*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDQLPDGKRATYRGTAFGSDDAGGKLT
YTIDFAAKQGNGKIEHLKSPELNVDLAAADIKPDGKRHAVISGSVLYNQAEKGSYSLGIFGGKAQEVAGSAEVKTVNGIR
HIGLAAKQ
***SEQ ID NO: 6*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYRGTAFGSDDAGGKLT
YTIDFAAKQGNGRIEHLKSPELNVELAAADIKPDGKRHAVISGDVRYGGEEKGSYSLGIFGGKAQEVAGSAEVKIRNGIR
HIGLAAKQ
***SEQ ID NO: 7*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYRGTAFGSDDAGGKLT
YTIDFAAKQGNGRIEHLKSPELNVELASADIKPDGKRHAVISGDVRYGGEEKGSYSLGIFGGKAQEVAGSAEVKIRNGIR
HIGLAAKQLE
***SEQ ID NO: 8*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYRGTAFGSDDAGGKLT
YTIDFAAKQGNGRIEHLKSPELNVELATADIKPDGKRHAVISGDVRYGGEEKGSYSLGIFGGKAQEVAGSAEVKIRNGIR
HIGLAAKQ
***SEQ ID NO: 9*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYRGTAFGSDDAGGKLT
YTIDFAAKQGNGRIEHLKSPELNVELASADIKPDGKRHAVISGDVRYGGEEKGTYSLGIFGGKAQEVAGSATVKIRNGIH
EIGLAAKQ
***SEQ ID NO: 10*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQINNPDKIDSMVAKRQFRIGDIAGEHTSFDQLPDGKRATYRGTAFGSDDAGGKLT
YTIDFAAKQGNGRIEHLKSPELNVELASADIKPDGKRHAVISGDVRYGGEEKGTYSLGIFGGKAQEVAGSATVKIRNGIR
HIGLAAKQ
***SEQ ID NO: 11*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHIDKMVAKRQFRISGIAGEHTSFDKLPEGGKAEYHGKAFGSDDPNGRLH
 
YSIDFAAKQGNGRIEHLKSPELNVELASADIKPDGKRHAVISGSVLYNQAEKGSYSLGIFGGKAQEVAGSAEVKTVNGIR
HIGLAAKQ
***SEQ ID NO: 12*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHTDKMVAKRQFRISGIAGEHTSFDKLPEGGKAEYHGKAFGSDDPNGRLH
YTIDFAAKQGNGRIEHLKSPELNVELASADIKPDGKRHAVISGSVLYNQAEKGSYSLGIFGGKAQEVAGSAEVKTVNGIR
HIGLAAKQ
***SEQ ID NO: 13*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHTDKMVAKRQFRISGIAGEHTSFDKLPEGGKAEYHGKAFGSDDPNGRLH
YTIDFAAKQGYGRIEHLKSPELNVELASADIKPDGKRHAVISGSVLYNQAEKGSYSLGIFGGKAQEVAGSAEVKTVNGIR
HIGLAAKQ
***SEQ ID NO: 14*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHIDKMVAKRQFRISGIAGEHTSFDKLPEGGKAEYHGKAFGSDDPNGRLH
YSIDFAAKQGYGRIEHLKSPELNVELASADIKPDGKRHAVISGSVLYNQAEKGSYSLGIFGGKAQEVAGSAEVKTVNGIR
HIGLAAKQ
***SEQ ID NO: 15*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHIDKMVAKRQFRISGIAGEHTSFDKLPEGGKAEYHGKAFGSDDAGGKLT
YTIDFAAKQGHGRIEHLKSPELNVELAAADIKPDGKRHAVISGSVLYNQAEKGSYSLGIFGGKAQEVAGSAEVKTVNGIR
HIGLAAKQ
***SEQ ID NO: 16*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTSFDKLPEGGRATYRGTAFGSDDAGGKLT
YTIDFAAKQGNGKIEHLKSPELNVDLASAEIKADEKSHAVILGDVRYNQAEKGTYSLGIFGGKAQEVAGSAEVKTVNGIR
HIGLAAKQ
***SEQ ID NO:** 17* 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTSFDKLPEGGRATYRGTAFGSDDAGGKLT
YTIDFAAKQGNGKIEHLKSPELNVDLAAAEIKADEKSHAVILGDVRYNQAEKGTYSLGIFGGKAQEVAGSAEVKTVNGIR
HIGLAAKQ
***SEQ ID NO:** 18* 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYHGKAFGSDDPNGRLH
YSIDFAAKQGYGRIEHLKTPEQNVDLAAADIKPDGKRHAVISGSVLYNQAEKGSYSLGIFGGKAQEVAGSAEVKIREGIR
HIGLAAKQ
*SEO ID **NO:** 19* 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYHGKAFGSDDAGGKLT
YTIDFAAKQGHGRIEHLKTPEQNVDLAAADIKPDGKRHAVISGSVLYNQAEKGSYSLGIFGGKAQEVAGSAEVKIGEGIR
HIGLAAKQ
***SEQ ID** NO: **20*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYHGKAFGSDDPNGRLH
YTIDFAAKQGYGRIEHLKTPEQNVDLAAADIKPDGKRHAVISGSVLYNQAEKGSYSLGIFGGKAQEVAGSAEVKIGEGIR
HIGLAAKQ
***SEQ** ID NO: 21* 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYHGKAFGSDDAGGKLT
YTIDFAAKQGHGKIEHLKTPEQNVDLAAADIKPDGKRHAVISGSVLYNQAEKGSYSLGIFGGKAQEVAGSAEVKIREGIR
HIGLAAKQ
***SEQ ID NO: 22*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYHGKAFGSDDPNGRLH
 
YTIDFAAKQGYGRIEHLKTPEQNVDLAAADIKPDGKRHAVISGSVLYNQAEKGSYSLGIFGGKAQEVAGSAEVKIREGIR
HIGLAAKQ
***SEQ ID NO: 23*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTAFNQLPDGKAEYRGTAFGSDDAGGKLTY
TIDFTKKQGNGKIEHLKSPELNVELASAEIKADGKSHAVILGDVRYGSEEKGSYSLGIFGGRAQEVAGSAEVKTVNGIRH
IGLAAKQ
***SEQ ID NO: 24*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTAFNQLPSGKAEYRGTAFGSDDAGGKLTY
TIDFTKKQGNGKIEHLKSPELNVELASAEIKADGKSHAVILGDVRYGGEEKGSYSLGIFGGRAQEVAGSAEVKTVNGIRH
IGLAAKQ
***SEQ ID NO: 25*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTAFNQLPSGKAEYRGTAFGSDDAGGKLTY
TIDFAAKQGNGKIEHLKSPELNVELASAEIKADGKSHAVILGDVRYGGEEKGSYSLGIFGGRAQEVAGSAEVKTVNGIRH
IGLAAKQ
***SEQ ID NO: 26*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTAFNQLPDGKAEYRGTAFGSDDAGGKLTY
TIDFTKKQGNGKIEHLKSPELNVELAAAEIKADGKSHAVILGDVRYGSEEKGSYSLGIFGGRAQEVAGSAEVKTVNGIRH
IGLAAKQ
***SEQ ID NO: 27*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTAFNQLPDGKAEYRGTAFGSDDAGGKLTY
TIDFTKKQGNGKIEHLKSPELNVELASAEIKADGKSHAVILGDVRYGGEEKGSYSLGIFGGRAQEVAGSAEVKTVNGIRH
IGLAAKQ
***SEQ ID NO:** 28* 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTAFNQLPDGKAEYRGTAFGSDDAGGKLTY
TIDFTNKQGNGKIEHLKSPELNVELASAEIKADGKSHAVILGDVRYGSEEKGSYSLGIFGGRAQEVAGSAEVKTVNGIRH
IGLAAKQ
***SEQ ID NO:** 29* 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTAFNQLPGGKAEYRGTAFGSDDAGGKLTY
TIDFAAKQGNGKIEHLKSPELNVELAAAEIKADGKSHAVILGDVRYGSEEKGSYSLGIFGGRAQEVAGSAEVKTVNGIRH
IGLAAKQ
***SEQ ID NO*: *30*** 
SEQ ID NO: 30
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTAFNQLPGGKAEYRGTAFGSDDAGGKLTY
TIDFTKKQGNGKIEHLKSPELNVELASAEIKADGKSHAVILGDVRYGSEEKGSYSLGIFGGRAQEVAGSAEVKTVNGIRH
IGLAAKQ
*SEO ID NO: **31*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYRGTAFGSDDAGGKLT
YTIDFAAKQGNGKIEHLKTPELNVDLAAADIKADEKRHAVILGDVLYGSEEKGTYHLGIFGGKAQEVAGSATVKIGEKVH
HIGLAAKQ
***SEQ ID NO: 32*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYRGTAFGSDDAGGKLT
YTIDFAAKQGNGKIEHLKTPELNVDLAAADIKADEKRHAVILGDVLYGGEEKGTYHLGIFGGKAQEVAGSATVKIGEKVH
HIGLAAKQ
***SEQ** I**D NO: 33*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYRGTAFGSDDAGGKLT
 
YTIDFAAKQGNGKIEHLKTPELNVDLAAADIKADEKRHAVILGDVRYGGEEKGTYHLGIFGGKAQEVAGSATVKIREKVH
HIGLAAKQ
***SEQ ID NO: 34*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYRGTAFGFDDAGGKLT
YTIDFAAKQGNGKIEHLKTPELNVDLAAADIKADEKRHAVILGDVRYGGEEKGTYHLGIFGGKAQEVAGSATVKIREKVH
HIGLAAKQ
***SEQ ID NO:** 35* 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYRGTAFGSDDAGGKLT
YTIDFAAKQGNGKIEHLKTPELNVDLAAADIKADEKRHAVILGDVRYGSEEKGTYHLGIFGGKAQEVAGSATVKIREKVH
HIGLAAKQ
***SEQ ID NO: 36*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQINNPDKSGKMVAKRQFRIGDIAGEHTSFDKLPEGGRATYRGTAFGSDDAGGKLT
YTIDFAAKQGNGKIEHLKTPELNVDLAAADIKADEKRHAVILGDVRYGSEEKGTYHLGIFGGKAQEVAGSATVKIGEKVH
HIGLAAKQ
***SEQ ID NO: 37*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQINNPDKSGKMVAKRQFRIGDIAGEHTSFDKLPEGGRATYRGTAFGSDDAGGKLT
YTIDFAAKQGNGKIEHLKTPELNVDLAAADIKADEKRHAVILGDVRYGGEEKGTYHLGIFGGKAQEVAGSATVKIGEKVH
HIGLAAKQ
***SEQ ID NO: 38*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQINNPDKSGKMVAKRQFRIGDIAGEHTSFDKLPEGGRATYRGTAFGSDDAGGKLT
YTIDFAAKQGNGKIEHLKTPELNVDLAAADIKADEKRHAVILGDVRYGGEEKGTYHLGIFGGKAQEVAGSATVKIREKVH
HIGLAAKQ
***SEQ ID NO:** 39* 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQINNPDKSGKMVAKRQFRIGDIAGEHTSFDKLPEGGRATYRGTAFGFDDAGGKLT
YTIDFAAKQGNGKIEHLKTPELNVDLAAADIKADEKRHAVILGDVRYGGEEKGTYHLGIFGGKAQEVAGSATVKIREKVH
HIGLAAKQ
***SEQ ID NO: 40*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQINNPDKSGKMVAKRQFRIGDIAGEHTSFDKLPEGGRATYRGTAFGSDDAGGKLT
YTIDFAAKQGNGKIEHLKTPELNVDLAAADIKADEKRHAVILGDVRYGSEEKGTYHLGIFGGKAQEVAGSATVKIREKVH
HIGLAAKQ
*SEO ID **NO:** 41* 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTAFNQLPDGKAEYHGTAFGSDDAGGKLTY
SIDFTKKQGNGKIEHLKSPELNVDLAAAEIKADEKRHAVISGSVLYNQAEKGSYSLGIFGGRAQEVAGSAEVKTVNGIRH
IGLAAKQ
***SEQ ID** NO: **42*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQINNPDKIDSMVAKRQFRIGDLGGEHTAFNQLPDGKAEYHGTAFGSDDAGGKLTY
SIDFTKKQGNGKIEHLKSPELNVDLAAAEIKADEKRHAVISGSVLYNQAEKGSYSLGIFGGRAQEVAGSAEVKTVNGIRH
IGLAAKQ
*SEO ID **NO:** 43* 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTAFNQLPGGKAEYHGTAFGSDDAGGKLTY
SIDFTKKQGNGKIEHLKSPELNVDLAAAEIKADEKRHAVISGSVLYNQAEKGSYSLGIFGGRAQEVAGSAEVKTVNGIRH
IGLAAKQ
***SEQ ID NO:** 44* 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQINNPDKIDSMVAKRQFRIGDLGGEHTAFNQLPGGKAEYHGTAFGSDDAGGKLTY
 
SIDFTKKQGNGKIEHLKSPELNVDLAAAEIKADEKRHAVISGSVLYNQAEKGSYSLGIFGGRAQEVAGSAEVKTVNGIRH
IGLAAKQ
***SEQ ID NO: 45*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTAFNQLPGGKAEYHGTAFGSDDAGGKLTY
TIDFAAKQGNGKIEHLKSPELNVDLAAAEIKADEKRHAVISGSVLYNQAEKGSYSLGIFGGRAQEVAGSAEVKTVNGIRH
IGLAAKQ
***SEQ ID NO: 46*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQINNPDKIDSMVAKRQFRIGDLGGEHTAFNQLPGGKAEYHGTAFGSDDAGGKLTY
TIDFAAKQGNGKIEHLKSPELNVDLAAAEIKADEKRHAVISGSVLYNQAEKGSYSLGIFGGRAQEVAGSAEVKTVNGIRH
IGLAAKQ
***SEQ ID NO: 47*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTAFNQLPSGKAEYHGTAFGSDDAGGKLTY
SIDFTKKQGNGKIEHLKSPELNVDLAAAEIKADEKRHAVISGSVLYNQAEKGSYSLGIFGGRAQEVAGSAEVKTVNGIRH
IGLAAKQ
***SEQ ID NO: 48*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQINNPDKIDSMVAKRQFRIGDLGGEHTAFNQLPSGKAEYHGTAFGSDDAGGKLTY
SIDFTKKQGNGKIEHLKSPELNVDLAAAEIKADEKRHAVISGSVLYNQAEKGSYSLGIFGGRAQEVAGSAEVKTVNGIRH
IGLAAKQ
***SEQ ID NO: 49*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTAFNQLPSGKAEYHGTAFGSDDAGGKLTY
TIDFAAKQGNGKIEHLKSPELNVDLAAAEIKADEKRHAVISGSVLYNQAEKGSYSLGIFGGRAQEVAGSAEVKTVNGIRH
IGLAAKQ
***SEQ ID NO: 50*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQINNPDKIDSMVAKRQFRIGDLGGEHTAFNQLPSGKAEYHGTAFGSDDAGGKLTY
TIDFAAKQGNGKIEHLKSPELNVDLAAAEIKADEKRHAVISGSVLYNQAEKGSYSLGIFGGRAQEVAGSAEVKTVNGIRH
IGLAAKQ
*SEO ID **NO:** 51* 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQINNPDKIDSMVAKRQFRIGDLGGEHTAFNQLPGGKAEYHGTAFGSDDAGGKLTY
SIDFTNKQGNGKIEHLKSPELNVDLAAAEIKADEKRHAVISGSVLYNQAEKGSYSLGIFGGRAQEVAGSAEVKTVNGIRH
IGLAAKQ
*SEO **ID NO:** 52* 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTAFNQLPGGKAEYHGTAFGSDDAGGKLTY
SIDFTNKQGNGKIEHLKSPELNVDLAAAEIKADEKRHAVISGSVLYNQAEKGSYSLGIFGGRAQEVAGSAEVKTVNGIRH
IGLAAKQ
*SEO ID **NO:** 53* 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYRGTAFGSDDAGGKLH
YSIDFTKKQGYGRIEHLKTPELNVELASADIKPDGKRHAVISGSVRYNGSEKGSYSLGIFGGKAQEVAGSAEVKTGEGIR
HIGLAAKQ
*SEO ID **NO:** 54* 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYRGTAFGSDDAGGKLH
YSIDFAAKQGHGRIEHLKTPELNVELASADIKPDGKRHAVISGSVRYNGSEKGSYSLGIFGGKAQEVAGSAEVKTGEGIR
HIGLAAKQ
***SEQ ID NO: 55*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYRGTAFGSDDAGGKLH
 
YSIDFAAKQGHGRIEHLKTPELNVELASADIKPDGKRHAVISGSVRYNGGEKGSYSLGIFGGKAQEVAGSAEVKTREGIR
HIGLAAKQ
***SEQ ID NO: 56*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTSFDKLPEGGRATYRGTAFGSDDAGGKLT
YTIDFAAKQGNGKIEHLKSPELNVDLAAAELKADEKRHAVILGDVLYNQEEKGTYSLGIFGGKAQEVAGSATVKTVNGIH
EIGLAAKQ
***SEQ ID NO: 57*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQINNPDKIDSMVAKRQFRIGDLGGEHTSFDKLPEGGRATYRGTAFGSDDAGGKLT
YTIDFAAKQGNGKIEHLKSPELNVDLAAAELKADEKRHAVILGDVLYNQEEKGTYSLGIFGGKAQEVAGSATVKTVNGIH
EIGLAAKQLE
***SEQ ID NO: 58*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTSFDKLPEGGRATYRGTAFGSDDAGGKLT
YTIDFAAKQGNGKIEHLKSPELNVDLAAAELKADEKSHAVILGDVLYNQEEKGTYSLGIFGGKAQEVAGSATVKTVNGIH
EIGLAAKQ
***SEQ ID NO: 59*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQINNPDKIDSMVAKRQFRIGDLGGEHTSFDKLPEGGRATYRGTAFGSDDAGGKLT
YTIDFAAKQGNGKIEHLKSPELNVDLAAAELKADEKSHAVILGDVLYNQEEKGTYSLGIFGGKAQEVAGSATVKTVNGIH
EIGLAAKQ
***SEQ ID NO: 60*** 
VAADIGAGLADALTTPLDHKDKSLQSLTLDQSVRKNEKLKLAAQGAEKTYGNGDSLNTGKLKNDKVSRFDFIRQIEVDGQ
TITLASGEFQIYKQNHSAVVALQIEKINNPDKIDSLINQRSFLVSGLAGEHTSFDKLPEGGRATYRGTAFSSDDPNGRLT
YSIDFTAKQGNGRIEHLKTPEQNVDLASAELKPDGKRHAVISGSVRYGGEEKGTYSLGLFGGKAQEIAGSATVKIREKVH
EIGIAGKQ
***SEQ ID NO: 61*** 
VAADIGAGLADALTTPLDHKDKSLQSLTLDQSVRKNEKLKLAAQGAEKTYGNGDSLNTGKLKNDKVSRFDFIRQIEVDGQ
TITLASGEFQIYKQNHSAVVALQIEKINNPDKIDSLINQRSFLVSGLAGEHTSFDKLPEGGRATYRGTAFSSDDPNGRLT
YSIDFTAKQGHGRIEHLKTPEQNVELASAYLKPDEKHHAVISGSVRYGGEEKGTYSLGLFGGKAQEIAGSATVKIREKVH
EIGIAGKQ
*SEO ID **NO: 62*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYRGTAFGSDDAGGKLT
YTIDFAAKQGNGKIEHLKSPELNVELAAADIKADGKRHAVILGDTRYGSEEKGTYSLGIFGGKAQEVAGSAEVKTGNGIR
HIGLAAKQ
***SEQ ID NO:** 63* 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYRGTAFGSDDAGGKLT
YTIDFAAKQGNGKIEHLKSPELNVELAAADIKADGKRHAVILGDTRYGGEEKGTYSLGIFGGKAQEVAGSAEVKTRNGIR
HIGLAAKQ
***SEQ ID NO:** 64* 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYRGTAFGSDDAGGKLH
YTIDFAKKQGNGRIEHLKSPELNVELAAADIKADGKRHAVILGDTRYGGEEKGTYSLGIFGGKAQEVAGSAEVKTRNGIR
HIGLAAKQ
***SEQ ID NO:** 65* 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYRGTAFGSDDAGGKLH
YTIDFAKKQGNGRIEHLKSPELNVELAAADIKADGKRHAVILGDTRYGSEEKGTYSLGIFGGKAQEVAGSAEVKTRNGIR
HIGLAAKQ
*SEQ ID NO: 66* 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYRGTAFGSDDAGGKLH
 
YTIDFAKKQGNGRIEHLKSPELNVELAAADIKADGKRHAVILGDTRYGGEEKGTYSLGIFGGKAQEVAGSAEVKTGNGIR
HIGLAAKQ
***SEQ ID NO: 67*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYRGTAFGSDDAGGKLH
YTIDFAKKQGNGRIEHLKSPELNVELAAADIKADGKRHAVILGDTRYGSEEKGTYSLGIFGGKAQEVAGSAEVKTGNGIR
HIGLAAKQ
***SEQ ID NO: 68*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYRGTAFGSDDAGGKLT
YTIDFAAKQGNGKIEHLKSPELNVELAAADIKADGKRHAVILGDTRYGSEEKGTYSLGIFGGKAQEVAGSAEVKTRNGIR
HIGLAAKQ
***SEQ ID NO: 69*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYHGKAFGSDDPNGRLH
YTIDFAAKQGYGRIEHLKTPEQNVELASAEIKPDGKRHAVILGDVRYGGEEKGTYHLGIFGGKAQEVAGSAEVKIGEGIR
HIGLAAKQ
***SEQ ID NO: 70*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYHGKAFGSDDPNGRLH
YTIDFAAKQGYGRIEHLKTPEQNVELAAAEIKPDGKRHAVILGDVRYGSEEKGTYHLGIFGGKAQEVAGSAEVKIGEGIR
HIGLAAKQ
***SEQ ID NO: 71*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYHGKAFGSDDPNGRLH
YTIDFAAKQGYGRIEHLKTPEQNVELASAEIKPDGKRHAVILGDVRYGSEEKGTYHLGIFGGKAQEVAGSAEVKIGEGIR
HIGLAAKQ
***SEQ ID NO: 72*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTSFDKLPEGGRATYRGTAFGSDDAGGKLT
YTIDFAAKQGYGRIEHLKSPELNVELAAAEIKADEKSHAVILGDVRYGSEEKGTYSLGIFGGKAQEVAGSAEVKTVNGIR
HIGLAAKQ
***SEQ ID NO:** 73* 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTSFDKLPEGGRATYRGTAFGSDDAGGKLT
YTIDFAAKQGYGRIEHLKSPELNVELAAAEIKADEKSHAVILGDVRYGGEEKGTYSLGIFGGKAQEVAGSAEVKTVNGIR
HIGLAAKQ
***SEQ ID NO:** 74* 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTSFDKLPEGGRATYRGTAFGSDDAGGKLT
YTIDFAAKQGHGKIEHLKSPELNVELAAAEIKADEKSHAVILGDVRYGSEEKGTYSLGIFGGKAQEVAGSAEVKTVNGIR
HIGLAAKQ
***SEQ ID NO: 75*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTSFDKLPEGGRATYRGTAFGSDDAGGKLT
YTIDFAAKQGHGKIEHLKSPELNVELAAAEIKADEKSHAVILGDVRYGGEEKGTYSLGIFGGKAQEVAGSAEVKTVNGIR
HIGLAAKQ
***SEQ ID NO: 76*** 
VAADIGAGLADALTAPLDHKDKGLQSLTLDQSVRKNEKLKLAAQGAEKTYGNGDSLNTGKLKNDKVSRFDFIRQIEVDGK
LITLESGEFQVYKQSHSALTALQTEQVQDSEDSGKMVAKRQFRIGDIAGEHTSFDKLPKGGSATYRGTAFGSDDAGGKLT
YTIDFAAKQGHGKIEHLKSPELNVELATAELKADEKSHAVILGDTRYGGEEKGTYHLALFGDRAQEIAGSATVKIREKVH
EIGIAGKQ
***SEQ ID NO: 77*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYHGKAFGSDDPNGRLH
 
YTIDFAAKQGYGRIEHLKTPEQNVDLAAADIKPDGKRHAVISGSVLYNQAAKGSYSLGIFGGKAQEVAGSAAVKIGEGIR
HIGLAAKQ
***SEQ ID NO: 78*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTAFNQLPDGKAEYRGTAFGSDDAGGKLTY
TIDFTKKQGNGKIEHLKSPELNVELASAEIKADGKSHAVILGDVRYGSEAKGSYSLGIFGGRAQEVAGSAAVKTVNGIRH
IGLAAKQ
***SEQ ID NO: 79***
   ICICICICICICICICICICICICIC
***SEQ ID NO: 80***
   KLKLLLLLKLK
***SEQ ID NO: 81***
   GSGGGG
***SEQ ID NO: 82***
   GSGSGGGG
***SEQ ID NO: 83*** 
MFKRSVIAMACIFALSACGGGGGGSPDVKSADTLSKPAAPVVSEKETEAKEDAPQAGSQGQGAPSAQGSQDMAAVSEENT
GNGGAVTADNPKNEDEVAQNDMPQNAAGTDSSTPNHTPDPNMLAGNMENQATDAGESSQPANQPDMANAADGMQGDDPSA
GGQNAGNTAAQGANQAGNNQAAGSSDPIPASNPAPANGGSNFGRVDLANGVLIDGPSQNITLTHCKGDSCSGNNFLDEEV
QLKSEFEKLSDADKISNYKKDGKNDKFVGLVADSVQMKGINQYIIFYKPKPTSFARFRRSARSRRSLPAEMPLIPVNQAD
TLIVDGEAVSLTGHSGNIFAPEGNYRYLTYGAEKLPGGSYALRVQGEPAKGEMLAGAAVYNGEVLHFHTENGRPYPTRGR
FAAKVDFGSKSVDGIIDSGDDLHMGTQKFKAAIDGNGFKGTWTENGSGDVSGKFYGPAGEEVAGKYSYRPTDAEKGGFGV
FAGKKEQD
***SEQ ID NO: 84*** 
MSMKHFPSKVLTTAILATFCSGALAATSDDDVKKAATVAIVAAYNNGQEINGFKAGETIYDIGEDGTITQKDATAADVEA
DDFKGLGLKKVVTNLTKTVNENKQNVDAKVKAAESEIEKLTTKLADTDAALADTDAALDETTNALNKLGENITTFAEETK
TNIVKIDEKLEAVADTVDKHAEAFNDIADSLDETNTKADEAVKTANEAKQTAEETKQNVDAKVKAAETAAGKAEAAAGTA
NTAADKAEAVAAKVTDIKADIATNKADIAKNSARIDSLDKNVANLRKETRQGLAEQAALSGLFQPYNVGRFNVTAAVGGY
KSESAVAIGTGFRFTENFAAKAGVAVGTSSGSSAAYHVGVNYEW
***SEQ ID NO: 85*** 
MKKALATLIALALPAAALAEGASGFYVQADAAHAKASSSLGSAKGFSPRISAGYRINDLRFAVDYTRYKNYKAPSTDFKL
YSIGASAIYDFDTQSPVKPYLGARLSLNRASVDLGGSDSFSQTSIGLGVLTGVSYAVTPNVDLDAGYRYNYIGKVNTVKN
VRSGELSAGVRVKF
***SEQ ID NO: 86*** 
MKPLQMLPIAALVGSIFGNPVLAADEAATETTPVKAEIKAVRVKGQRNAPAAVERVNLNRIKQEMIRDNKDLVRYSTDVG
LSDSGRHQKGFAVRGVEGNRVGVSIDGVNLPDSEENSLYARYGNFNSSRLSIDPELVRNIEIVKGADSFNTGSGALGGGV
NYQTLQGRDLLLDDRQFGVMMKNGYSTRNREWTNTLGFGVSNDRVDAALLYSQRRGHETESAGNRGYAVEGEGSGANIRG
SARGIPDSSKHKYNHHALGKIAYQINDNHRIGASLNGQQGHNYTVEESYNLTASSWREADDVNRRRNANLFYEWMPDSNW
LSSLKADFDYQKTKVAAVNNKGSFPMDYSTWTRNYNQKDLDEIYNRSMDTRFKRFTLRLDSHPLQLGGGRHRLSFKTFVS
RRDFENLNRDDYYFSGRVVRTTSSIQHPVKTTNYGFSLSDQIQWNDVFSSRAGIRYDHTKMTPQELNAECHACDKTPPAA
NTYKGWSGFVGLAAQLNQAWRVGYDITSGYRVPNASEVYFTYNHGSGNWLPNPNLKAERSTTHTLSLQGRSEKGMLDANL
YQSNYRNFLSEEQKLTTSGTPGCTEENAYYGICSDPYKEKLDWQMKNIDKARIRGIELTGRLNVDKVASFVPEGWKLFGS
LGYAKSKLSGDNSLLSTQPLKVIAGIDYESPSEKWGVFSRLTYLGAKKVKDAQYTVYENKGWGTPLQKKVKDYPWLNKSA
YVFDMYGFYKPAKNLTLRAGVYNLFNRKYTTWDSLRGLYSYSTTNAVDRDGKGLDRYRAPGRNYAVSLEWKF
***SEQ ID NO: 87*** 
MNKIYRIIWNSALNAWVVVSELTRNHTKRASATVKTAVLATLLFATVQASANNEEQEEDLYLDPVQRTVAVLIVNSDKEG
TGEKEKVEENSDWAVYFNEKGVLTAREITLKAGDNLKIKQNGTNFTYSLKKDLTDLTSVGTEKLSFSANGNKVNITSDTK
GLNFAKETAGTNGDTTVHLNGIGSTLTDTLLNTGATTNVTNDNVTDDEKKRAASVKDVLNAGWNIKGVKPGTTASDNVDF
VRTYDTVEFLSADTKTTTVNVESKDNGKKTEVKIGAKTSVIKEKDGKLVTGKDKGENGSSTDEGEGLVTAKEVIDAVNKA
GWRMKTTTANGQTGQADKFETVTSGTNVTFASGKGTTATVSKDDQGNITVMYDVNVGDALNVNQLQNSGWNLDSKAVAGS
SGKVISGNVSPSKGKMDETVNINAGNNIEITRNGKNIDIATSMTPQFSSVSLGAGADAPTLSVDGDALNVGSKKDNKPVR
ITNVAPGVKEGDVTNVAQLKGVAQNLNNRIDNVDGNARAGIAQAIATAGLVQAYLPGKSMMAIGGGTYRGEAGYAIGYSS
ISDGGNWIIKGTASGNSRGHFGASASVGYQW
***SEQ ID NO: 88*** 
MKTTDKRTTETHRKAPKTGRIRFSPAYLAICLSFGILPQAWAGHTYFGINYQYYRDFAENKGKFAVGAKDIEVYNKKGEL
VGKSMTKAPMIDFSVVSRNGVAALVGDQYIVSVAHNGGYNNVDFGAEGRNPDQHRFTYKIVKRNNYKAGTKGHPYGGDYH
MPRLHKFVTDAEPVEMTSYMDGRKYIDQNNYPDRVRIGAGRQYWRSDEDEPNNRESSYHIASAYSWLVGGNTFAQNGSGG
GTVNLGSEKIKHSPYGFLPTGGSFGDSGSPMFIYDAQKQKWLINGVLQTGNPYIGKSNGFQLVRKDWFYDEIFAGDTHSV
FYEPRQNGKYSFNDDNNGTGKINAKHEHNSLPNRLKTRTVQLFNVSLSETAREPVYHAAGGVNSYRPRLNNGENISFIDE
GKGELILTSNINQGAGGLYFQGDFTVSPENNETWQGAGVHISEDSTVTWKVNGVANDRLSKIGKGTLHVQAKGENQGSIS
VGDGTVILDQQADDKGKKQAFSEIGLVSGRGTVQLNADNQFNPDKLYFGFRGGRLDLNGHSLSFHRIQNTDEGAMIVNHN
QDKESTVTITGNKDIATTGNNNSLDSKKEIAYNGWFGEKDTTKTNGRLNLVYQPAAEDRTLLLSGGTNLNGNITQTNGKL
FFSGRPTPHAYNHLNDHWSQKEGIPRGEIVWDNDWINRTFKAENFQIKGGQAVVSRNVAKVKGDWHLSNHAQAVFGVAPH
QSHTICTRSDWTGLTNCVEKTITDDKVIASLTKTDISGNVDLADHAHLNLTGLATLNGNLSANGDTRYTVSHNATQNGNL
SLVGNAQATFNQATLNGNTSASGNASFNLSDHAVQNGSLTLSGNAKANVSHSALNGNVSLADKAVFHFESSRFTGQISGG
KDTALHLKDSEWTLPSGTELGNLNLDNATITLNSAYRHDAAGAQTGSATDAPRRRSRRSRRSLLSVTPPTSVESRFNTLT
VNGKLNGQGTFRFMSELFGYRSDKLKLAESSEGTYTLAVNNTGNEPASLEQLTVVEGKDNKPLSENLNFTLQNEHVDAGA
WRYQLIRKDGEFRLHNPVKEQELSDKLGKAEAKKQAEKDNAQSLDALIAAGRDAVEKTESVAEPARQAGGENVGIMQAEE
EKKRVQADKDTALAKQREAETRPATTAFPRARRARRDLPQLQPQPQPQPQRDLISRYANSGLSEFSATLNSVFAVQDELD
RVFAEDRRNAVWTSGIRDTKHYRSQDFRAYRQQTDLRQIGMQKNLGSGRVGILFSHNRTENTFDDGIGNSARLAHGAVFG
QYGIDRFYIGISAGAGFSSGSLSDGIGGKIRRRVLHYGIQARYRAGFGGFGIEPHIGATRYFVQKADYRYENVNIATPGL
AFNRYRAGIKADYSFKPAQHISITPYLSLSYTDAASGKVRTRVNTAVLAQDFGKTRSAEWGVNAEIKGFTLSLHAAAAKG
PQLEAQHSAGIKLGYRW
***SEQ ID NO: 89*** 
MKLKQIASALMMLGISPLALADFTIQDIRVEGLQRTEPSTVFNYLPVKVGDTYNDTHGSAIIKSLYATGFFDDVRVETAD
GQLLLTVIERPTIGSLNITGAKMLQNDAIKKNLESFGLAQSQYFNQATLNQAVAGLKEEYLGRGKLNIQITPKVTKLARN
RVDIDITIDEGKSAKITDIEFEGNQVYSDRKLMRQMSLTEGGIWTWLTRSNQFNEQKFAQDMEKVTDFYQNNGYFDFRIL
DTDIQTNEDKTKQTIKITVHEGGRFRWGKVSIEGDTNEVPKAELEKLLTMKPGKWYERQQMTAVLGEIQNRMGSAGYAYS
EISVQPLPNAETKTVDFVLHIEPGRKIYVNEIHITGNNKTRDEVVRRELRQMESAPYDTSKLQRSKERVELLGYFDNVQF
DAVPLAGTPDKVDLNMSLTERSTGSLDLSAGWVQDTGLVMSAGVSQDNLFGTGKSAALRASRSKTTLNGSLSFTDPYFTA
DGVSLGYDVYGKAFDPRKASTSIKQYKTTTAGAGIRMSVPVTEYDRVNFGLVAEHLTVNTYNKAPKHYADFIKKYGKTDG
TDGSFKGWLYKGTVGWGRNKTDSALWPTRGYLTGVNAEIALPGSKLQYYSATHNQTWFFPLSKTFTLMLGGEVGIAGGYG
RTKEIPFFENFYGGGLGSVRGYESGTLGPKVYDEYGEKISYGGNKKANVSAELLFPMPGAKDARTVRLSLFADAGSVWDG
KTYDDNSSSATGGRVQNIYGAGNTHKSTFTNELRYSAGGAVTWLSPLGPMKFSYAYPLKKKPEDEIQRFQFQLGTTF
***SEQ ID NO: 90*** 
MASPDVKSADTLSKPAAPVVSEKETEAKEDAPQAGSQGQGAPSAQGGQDMAAVSEENTGNGGAAATDKPKNEDEGAQNDM
PQNAADTDSLTPNHTPASNMPAGNMENQAPDAGESEQPANQPDMANTADGMQGDDPSAGGENAGNTAAQGTNQAENNQTA
GSQNPASSTNPSATNSGGDFGRTNVGNSVVIDGPSQNITLTHCKGDSCSGNNFLDEEVQLKSEFEKLSDADKISNYKKDG
KNDGKNDKFVGLVADSVQMKGINQYIIFYKPKPTSFARFRRSARSRRSLPAEMPLIPVNQADTLIVDGEAVSLTGHSGNI
FAPEGNYRYLTYGAEKLPGGSYALRVQGEPSKGEMLAGTAVYNGEVLHFHTENGRPSPSRGRFAAKVDFGSKSVDGIIDS
GDGLHMGTQKFKAAIDGNGFKGTWTENGGGDVSGKFYGPAGEEVAGKYSYRPTDAEKGGFGVFAGKKEQDGSGGGATYK
VDEYHANARFAIDHFNTSTNVGGFYGLTGSVEFDQAKRDGKIDITIPVANLQSGSQHFTDHLKSADIFDAAQYPDIRFVS
TKFNFNGKKLVSVDGNLTMHGKTAPVKLKAEKFNCYQSPMAKTEVCGGDFSTTIDRTKWGVDYLVNVGMTKSVRIDIQIE
AAKQ
***SEQ ID NO: 91*** 
MVSAVIGSAAVGAKSAVDRRTTGAQTDDNVMALRIETTARSYLRQNNQTKGYTPQISVVGYDRHLLLLGQVATEGEKQFV
GQIARSEQAAEGVYNYITVASLPRTAGDIAGDTWNTSKVRATLLGISPATRARVKIVTYGNVTYVMGILTPEEQAQITQK
VSTTVGVQKVITLYQNYVQRGSGGGGVAADIGAGLADALTAPLDHKDKGLQSLTLDQSVRKNEKLKLAAQGAEKTYGNGD
SLNTGKLKNDKVSRFDFIRQIEVDGQLITLESGEFQVYKQSHSALTAFQTEQIQDSEHSGKMVAKRQFRIGDIAGEHTSF
DKLPEGGRATYRGTAFGSDDAGGKLTYTIDFAAKQGNGKIEHLKSPELNVDLAAADIKPDGKRHAVISGSVLYNQAEKGS
YSLGIFGGKAQEVAGSAEVKTVNGIRHIGLAAKQ
***SEQ ID NO: 92*** 
MKHFPSKVLTTAILATFCSGALAATNDDDVKKAATVAIAAAYNNGQEINGFKAGETIYDIDEDGTITKKDATAADVEADD
FKGLGLKKVVTNLTKTVNENKQNVDAKVKAAESEIEKLTTKLADTDAALADTDAALDATTNALNKLGENITTFAEETKTN
IVKIDEKLEAVADTVDKHAEAFNDIADSLDETNTKADEAVKTANEAKQTAEETKQNVDAKVKAAETAAGKAEAAAGTANT
AADKAEAVAAKVTDIKADIATNKDNIAKKANSADVYTREESDSKFVRIDGLNATTEKLDTRLASAEKSIADHDTRLNGLD
KTVSDLRKETRQGLAEQAALSGLFQPYNVG
 *SEQ ID **NO:** 93*
   ASGGGS
***SEQ** ID **NO:** 94*
   GCTAGCGGTGGCGGATCC
***SEQ ID NO: 95***
   HHHHHH
***SEQ ID NO: 96***
   LEHHHHHH
***SEQ ID NO: 97*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYRGTAFGSDDAGGKLT
YTIDFAAKQGNGKIEHLKSPELNVDLAAADIKPDGKRHAVISGSVLYNQAEKGSYSLGIFGGKAQEVAGSAEVKTVNGIR
HIGLAAKQ
***SEQ ID NO: 98*** 
MVSAVIGSAAVGAKSAVDRRTTGAQTDDNVMALRIETTARSYLRQNNQTKGYTPQISVVGYDRHLLLLGQVATEGEKQFV
GQIARSEQAAEGVYNYITVASLPRTAGDIAGDTWNTSKVRATLLGISPATRARVKIVTYGNVTYVMGILTPEEQAQITQK
VSTTVGVQKVITLYQNYVQR
***SEQ ID NO: 99*** 
MVAADIGAGLADALTAPLDHKDKGLQSLTLDQSVRKNEKLKLAAQGAEKTYGNGDSLNTGKLKNDKVSRFDFIRQIEVDG
QLITLESGEFQVYKQSHSALTAFQTEQIQDSEHSGKMVAKRQFRIGDIAGEHTSFDKLPEGGRATYHGKAFGSDDPNGRL
HYTIDFAAKQGYGRIEHLKTPEQNVDLAAADIKPDGKRHAVISGSVLYNQAEKGSYSLGIFGGKAQEVAGSAEVKIGEGI
RHIGLAAKQGSGGGGVAADIGAGLADALTAPLDHKDKGLQSLTLDQSVRKNEKLKLAAQGAEKTYGNGDSLNTGKLKNDK
VSRFDFIRQIEVDGQLITLESGEFQVYKQSHSALTAFQTEQIQDSEHSGKMVAKRQFRIGDIAGEHTSFDKLPEGGRATY
HGKAFGSDDPNGRLHYTIDFAAKQGYGRIEHLKTPEQNVDLAAADIKPDGKRHAVISGSVLYNQAEKGSYSLGIFGGKAQ
EVAGSAEVKIGEGIRHIGLAAKQLEHHHHHH
***SEQ ID NO: 100*** 
MVAADIGAGLADALTAPLDHKDKGLQSLTLDQSVRKNEKLKLAAQGAEKTYGNGDSLNTGKLKNDKVSRFDFIRQIEVDG
QLITLESGEFQVYKQSHSALTAFQTEQIQDSEHSGKMVAKRQFRIGDLGGEHTAFNQLPDGKAEYRGTAFGSDDAGGKLT
YTIDFTKKQGNGKIEHLKSPELNVELASAEIKADGKSHAVILGDVRYGSEEKGSYSLGIFGGRAQEVAGSAEVKTVNGIR
HIGLAAKQGSGGGGVAADIGAGLADALTAPLDHKDKGLQSLTLDQSVRKNEKLKLAAQGAEKTYGNGDSLNTGKLKNDKV
SRFDFIRQIEVDGQLITLESGEFQVYKQSHSALTAFQTEQIQDSEHSGKMVAKRQFRIGDLGGEHTAFNQLPDGKAEYRG
TAFGSDDAGGKLTYTIDFTKKQGNGKIEHLKSPELNVELASAEIKADGKSHAVILGDVRYGSEEKGSYSLGIFGGRAQEV
AGSAEVKTVNGIRHIGLAAKQGSGGGGVAADIGAGLADALTAPLDHKDKGLQSLTLDQSVRKNEKLKLAAQGAEKTYGNG
DSLNTGKLKNDKVSRFDFIRQIEVDGQLITLESGEFQVYKQSHSALTAFQTEQIQDSEHSGKMVAKRQFRIGDLGGEHTA
FNQLPDGKAEYRGTAFGSDDAGGKLTYTIDFTKKQGNGKIEHLKSPELNVELASAEIKADGKSHAVILGDVRYGSEEKGS
YSLGIFGGRAQEVAGSAEVKTVNGIRHIGLAAKQLEHHHHHH
***SEQ ID NO: 101*** 
MVAADIGAGLADALTAPLDHKDKGLQSLTLDQSVRKNEKLKLAAQGAEKTYGNGDSLNTGKLKNDKVSRFDFIRQIEVDG
QLITLESGEFQVYKQSHSALTAFQTEQIQDSEHSGKMVAKRQFRIGDLGGEHTAFNQLPDGKAEYRGTAFGSDDAGGKLT
YTIDFTKKQGNGKIEHLKSPELNVELASAEIKADGKSHAVILGDVRYGSEEKGSYSLGIFGGRAQEVAGSAEVKTVNGIR
HIGLAAKQGSGGGGVAADIGAGLADALTAPLDHKDKGLQSLTLDQSVRKNEKLKLAAQGAEKTYGNGDSLNTGKLKNDKV
SRFDFIRQIEVDGQLITLESGEFQVYKQSHSALTAFQTEQIQDSEHSGKMVAKRQFRIGDIAGEHTSFDKLPEGGRATYR
GTAFGSDDAGGKLTYTIDFAAKQGNGKIEHLKSPELNVDLAAADIKPDGKRHAVISGSVLYNQAEKGSYSLGIFGGKAQE
VAGSAEVKTVNGIRHIGLAAKQLEHHHHHH
***SEQ ID NO: 102*** 
MVSAVIGSAAVGAKSAVDRRTTGAQTDDNVMALRIETTARSYLRQNNQTKGYTPQISVVGYDRHLLLLGQVATEGEKQFV
GQIARSEQAAEGVYNYITVASLPRTAGDIAGDTWNTSKVRATLLGISPATRARVKIVTYGNVTYVMGILTPEEQAQITQK
VSTTVGVQKVITLYQNYVQRGSGGGGVAADIGAGLADALTAPLDHKDKGLQSLTLDQSVRKNEKLKLAAQGAEKTYGNGD
SLNTGKLKNDKVSRFDFIRQIEVDGQLITLESGEFQVYKQSHSALTAFQTEQIQDSEHSGKMVAKRQFRIGDIAGEHTSF
DKLPEGGRATYHGKAFGSDDPNGRLHYTIDFAAKQGYGRIEHLKTPEQNVDLAAADIKPDGKRHAVISGSVLYNQAEKGS
YSLGIFGGKAQEVAGSAEVKIGEGIRHIGLAAKQLEHHHHHH
***SEQ ID NO: 103*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYHGKAFGSDDPNGRLH
YTIDFAAKQGYGRIEHLKTPEQNVDLAAADIKPDGKRHAVISGSVLYNQAEKGSYSLGIFGGKAQEVAGSAEVKIGEGIR
HIGLAAKQGSGGGGVAADIGAGLADALTAPLDHKDKGLQSLTLDQSVRKNEKLKLAAQGAEKTYGNGDSLNTGKLKNDKV
SRFDFIRQIEVDGQLITLESGEFQVYKQSHSALTAFQTEQIQDSEHSGKMVAKRQFRIGDIAGEHTSFDKLPEGGRATYH
GKAFGSDDPNGRLHYTIDFAAKQGYGRIEHLKTPEQNVDLAAADIKPDGKRHAVISGSVLYNQAEKGSYSLGIFGGKAQE
VAGSAEVKIGEGIRHIGLAAKQGSGGGGVAADIGAGLADALTAPLDHKDKGLQSLTLDQSVRKNEKLKLAAQGAEKTYGN
GDSLNTGKLKNDKVSRFDFIRQIEVDGQLITLESGEFQVYKQSHSALTAFQTEQIQDSEHSGKMVAKRQFRIGDIAGEHT
 
SFDKLPEGGRATYRGTAFGSDDAGGKLTYTIDFAAKQGNGKIEHLKSPELNVDLAADIKPDGKRHAVISGSVLYNQAEK
GSYSLGIFGGKAQEVAGSAEVKTVNGIRHIGLAAKQ
***SEQ ID NO: 104*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYHGKAFGSDDPNGRLH
YTIDFAAKQGYGRIEHLKTPEQNVDLAAADIKPDGKRHAVISGSVLYNQAEKGSYSLGIFGGKAQEVAGSAEVKIGEGIR
HIGLAAKQGSGGGVAADIGAGLADALTAPLDHKDKGLQSLTLDQSVRKNEKLKLAAQGAEKTYGNGDSLNTGKLKNDKV
SRFDFIRQIEVDGQLITLESGEFQVYKQSHSALTAFQTEQIQDSEHSGKMVAKRQFRIGDIAGEHTSFDKLPEGGRATYH
GKAFGSDDPNGRLHYTIDFAAKQGYGRIEHLKTPEQNVDLAAADIKPDGKRHAVISGSVLYNQAEKGSYSLGIFGGKAQE
VAGSAEVKIGEGIRHIGLAAKQGSGGGVAADIGAGLADALTAPLDHKDKGLQSLTLDQSVRKNEKLKLAAQGAEKTYGN
GDSLNTGKLKNDKVSRFDFIRQIEVDGQLITLESGEFQVYKQSHSALTAFQTEQIQDSEHSGKMVAKRQFRIGDLGGEHT
AFNQLPDGKAEYRGTAFGSDDAGGKLTYTIDFTKKQGNGKIEHLKSPELNVELASAEIKADGKSHAVILGDVRYGSEEKG
SYSLGIFGGRAQEVAGSAEVKTVNGIRHIGLAAKQ
***SEQ ID NO: 105*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYHGKAFGSDDPNGRLH
YTIDFAAKQGYGRIEHLKTPEQNVDLAAADIKPDGKRHAVISGSVLYNQAEKGSYSLGIFGGKAQEVAGSAEVKIGEGIR
HIGLAAKQGSGGGVAADIGAGLADALTAPLDHKDKGLQSLTLDQSVRKNEKLKLAAQGAEKTYGNGDSLNTGKLKNDKV
SRFDFIRQIEVDGQLITLESGEFQVYKQSHSALTAFQTEQIQDSEHSGKMVAKRQFRIGDLGGEHTAFNQLPDGKAEYRG
TAFGSDDAGGKLTYTIDFTKKQGNGKIEHLKSPELNVELASAEIKADGKSHAVILGDVRYGSEEKGSYSLGIFGGRAQEV
AGSAEVKTVNGIRHIGLAAKQGSGGGVAADIGAGLADALTAPLDHKDKGLQSLTLDQSVRKNEKLKLAAQGAEKTYGNG
DSLNTGKLKNDKVSRFDFIRQIEVDGQLITLESGEFQVYKQSHSALTAFQTEQIQDSEHSGKMVAKRQFRIGDLGGEHTA
FNQLPDGKAEYRGTAFGSDDAGGKLTYTIDFTKKQGNGKIEHLKSPELNVELASAEIKADGKSHAVILGDVRYGSEEKGS
YSLGIFGGRAQEVAGSAEVKTVNGIRHIGLAAKQ
***SEQ ID NO: 106*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYHGKAFGSDDPNGRLH
YTIDFAAKQGYGRIEHLKTPEQNVDLAAADIKPDGKRHAVISGSVLYNQAEKGSYSLGIFGGKAQEVAGSAEVKIGEGIR
HIGLAAKQGSGGGGVAADIGAGLADALTAPLDHKDKGLQSLTLDQSVRKNEKLKLAAQGAEKTYGNGDSLNTGKLKNDKV
SRFDFIRQIEVDGQLITLESGEFQVYKQSHSALTAFQTEQIQDSEHSGKMVAKRQFRIGDIAGEHTSFDKLPEGGRATYR
GTAFGSDDAGGKLTYTIDFAAKQGNGKIEHLKSPELNVDLAAADIKPDGKRHAVISGSVLYNQAEKGSYSLGIFGGKAQE
VAGSAEVKTVNGIRHIGLAAKQGSGGGGVAADIGAGLADALTAPLDHKDKGLQSLTLDQSVRKNEKLKLAAQGAEKTYGN
GDSLNTGKLKNDKVSRFDFIRQIEVDGQLITLESGEFQVYKQSHSALTAFQTEQIQDSEHSGKMVAKRQFRIGDLGGEHT
AFNQLPDGKAEYRGTAFGSDDAGGKLTYTIDFTKKQGNGKIEHLKSPELNVELASAEIKADGKSHAVILGDVRYGSEEKG
SYSLGIFGGRAQEVAGSAEVKTVNGIRHIGLAAKQ
***SEQ ID NO: 107*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYHGKAFGSDDPNGRLH
YTIDFAAKQGYGRIEHLKTPEQNVDLAAADIKPDGKRHAVISGSVLYNQAEKGSYSLGIFGGKAQEVAGSAEVKIGEGIR
HIGLAAKQGSGGGVAADIGAGLADALTAPLDHKDKGLQSLTLDQSVRKNEKLKLAAQGAEKTYGNGDSLNTGKLKNDKV
SRFDFIRQIEVDGQLITLESGEFQVYKQSHSALTAFQTEQIQDSEHSGKMVAKRQFRIGDIAGEHTSFDKLPEGGRATYH
GKAFGSDDPNGRLHYTIDFAAKQGYGRIEHLKTPEQNVDLAAADIKPDGKRHAVISGSVLYNQAEKGSYSLGIFGGKAQE
VAGSAEVKIGEGIRHIGLAAKQGSGGGVAADIGAGLADALTAPLDHKDKGLQSLTLDQSVRKNEKLKLAAQGAEKTYGN
GDSLNTGKLKNDKVSRFDFIRQIEVDGQLITLESGEFQVYKQSHSALTAFQTEQIQDSEHSGKMVAKRQFRIGDIAGEHT
SFDKLPEGGRATYHGKAFGSDDPNGRLHYTIDFAAKQGYGRIEHLKTPEQNVDLAAADIKPDGKRHAVISGSVLYNQAEK
GSYSLGIFGGKAQEVAGSAEVKIGEGIRHIGLAAKQ
*SEO **ID NO:** 108* 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYHGKAFGSDDPNGRLH
YTIDFAAKQGYGRIEHLKTPEQNVDLAAADIKPDGKRHAVISGSVLYNQAEKGSYSLGIFGGKAQEVAGSAEVKIGEGIR
HIGLAAKQGSGGGVAADIGAGLADALTAPLDHKDKGLQSLTLDQSVRKNEKLKLAAQGAEKTYGNGDSLNTGKLKNDKV
SRFDFIRQIEVDGQLITLESGEFQVYKQSHSALTAFQTEQIQDSEHSGKMVAKRQFRIGDLGGEHTAFNQLPDGKAEYRG
TAFGSDDAGGKLTYTIDFTKKQGNGKIEHLKSPELNVELASAEIKADGKSHAVILGDVRYGSEEKGSYSLGIFGGRAQEV
AGSAEVKTVNGIRHIGLAAKQGSGGGVAADIGAGLADALTAPLDHKDKGLQSLTLDQSVRKNEKLKLAAQGAEKTYGNG
DSLNTGKLKNDKVSRFDFIRQIEVDGQLITLESGEFQVYKQSHSALTAFQTEQIQDSEHSGKMVAKRQFRIGDIAGEHTS
FDKLPEGGRATYHGKAFGSDDPNGRLHYTIDFAAKQGYGRIEHLKTPEQNVDLAAADIKPDGKRHAVISGSVLYNQAEKG
SYSLGIFGGKAQEVAGSAEVKIGEGIRHIGLAAKQ
***SEQ ID NO: 109*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYHGKAFGSDDPNGRLH
YTIDFAAKQGYGRIEHLKTPEQNVDLAAADIKPDGKRHAVISGSVLYNQAEKGSYSLGIFGGKAQEVAGSAEVKIGEGIR
HIGLAAKQGSGGGGVAADIGAGLADALTAPLDHKDKGLQSLTLDQSVRKNEKLKLAAQGAEKTYGNGDSLNTGKLKNDKV
SRFDFIRQIEVDGQLITLESGEFQVYKQSHSALTAFQTEQIQDSEHSGKMVAKRQFRIGDLGGEHTAFNQLPDGKAEYRG
TAFGSDDAGGKLTYTIDFTKKQGNGKIEHLKSPELNVELASAEIKADGKSHAVILGDVRYGSEEKGSYSLGIFGGRAQEV
AGSAEVKTVNGIRHIGLAAKQGSGGGGVAADIGAGLADALTAPLDHKDKGLQSLTLDQSVRKNEKLKLAAQGAEKTYGNG
DSLNTGKLKNDKVSRFDFIRQIEVDGQLITLESGEFQVYKQSHSALTAFQTEQIQDSEHSGKMVAKRQFRIGDIAGEHTS
FDKLPEGGRATYRGTAFGSDDAGGKLTYTIDFAAKQGNGKIEHLKSPELNVDLAAADIKPDGKRHAVISGSVLYNQAEKG
SYSLGIFGGKAQEVAGSAEVKTVNGIRHIGLAAKQ
***SEQ ID NO: 110*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYHGKAFGSDDPNGRLH
YTIDFAAKQGYGRIEHLKTPEQNVDLAAADIKPDGKRHAVISGSVLYNQAEKGSYSLGIFGGKAQEVAGSAEVKIGEGIR
HIGLAAKQGSGGGGVAADIGAGLADALTAPLDHKDKGLQSLTLDQSVRKNEKLKLAAQGAEKTYGNGDSLNTGKLKNDKV
SRFDFIRQIEVDGQLITLESGEFQVYKQSHSALTAFQTEQIQDSEHSGKMVAKRQFRIGDIAGEHTSFDKLPEGGRATYR
GTAFGSDDAGGKLTYTIDFAAKQGNGKIEHLKSPELNVDLAAADIKPDGKRHAVISGSVLYNQAEKGSYSLGIFGGKAQE
VAGSAEVKTVNGIRHIGLAAKQGSGGGGVAADIGAGLADALTAPLDHKDKGLQSLTLDQSVRKNEKLKLAAQGAEKTYGN
GDSLNTGKLKNDKVSRFDFIRQIEVDGQLITLESGEFQVYKQSHSALTAFQTEQIQDSEHSGKMVAKRQFRIGDIAGEHT
SFDKLPEGGRATYHGKAFGSDDPNGRLHYTIDFAAKQGYGRIEHLKTPEQNVDLAAADIKPDGKRHAVISGSVLYNQAEK
GSYSLGIFGGKAQEVAGSAEVKIGEGIRHIGLAAKQ
***SEQ ID NO: 111*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTAFNQLPDGKAEYRGTAFGSDDAGGKLTY
TIDFTKKQGNGKIEHLKSPELNVELASAEIKADGKSHAVILGDVRYGSEEKGSYSLGIFGGRAQEVAGSAEVKTVNGIRH
IGLAAKQGSGGGVAADIGAGLADALTAPLDHKDKGLQSLTLDQSVRKNEKLKLAAQGAEKTYGNGDSLNTGKLKNDKVS
RFDFIRQIEVDGQLITLESGEFQVYKQSHSALTAFQTEQIQDSEHSGKMVAKRQFRIGDIAGEHTSFDKLPEGGRATYHG
KAFGSDDPNGRLHYTIDFAAKQGYGRIEHLKTPEQNVDLAAADIKPDGKRHAVISGSVLYNQAEKGSYSLGIFGGKAQEV
AGSAEVKIGEGIRHIGLAAKQGSGGGVAADIGAGLADALTAPLDHKDKGLQSLTLDQSVRKNEKLKLAAQGAEKTYGNG
DSLNTGKLKNDKVSRFDFIRQIEVDGQLITLESGEFQVYKQSHSALTAFQTEQIQDSEHSGKMVAKRQFRIGDIAGEHTS
FDKLPEGGRATYRGTAFGSDDAGGKLTYTIDFAAKQGNGKIEHLKSPELNVDLAAADIKPDGKRHAVISGSVLYNQAEKG
SYSLGIFGGKAQEVAGSAEVKTVNGIRHIGLAAKQ
***SEQ ID NO: 112*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTAFNQLPDGKAEYRGTAFGSDDAGGKLTY
TIDFTKKQGNGKIEHLKSPELNVELASAEIKADGKSHAVILGDVRYGSEEKGSYSLGIFGGRAQEVAGSAEVKTVNGIRH
IGLAAKQGSGGGGVAADIGAGLADALTAPLDHKDKGLQSLTLDQSVRKNEKLKLAAQGAEKTYGNGDSLNTGKLKNDKVS
RFDFIRQIEVDGQLITLESGEFQVYKQSHSALTAFQTEQIQDSEHSGKMVAKRQFRIGDIAGEHTSFDKLPEGGRATYHG
KAFGSDDPNGRLHYTIDFAAKQGYGRIEHLKTPEQNVDLAAADIKPDGKRHAVISGSVLYNQAEKGSYSLGIFGGKAQEV
AGSAEVKIGEGIRHIGLAAKQGSGGGGVAADIGAGLADALTAPLDHKDKGLQSLTLDQSVRKNEKLKLAAQGAEKTYGNG
DSLNTGKLKNDKVSRFDFIRQIEVDGQLITLESGEFQVYKQSHSALTAFQTEQIQDSEHSGKMVAKRQFRIGDLGGEHTA
FNQLPDGKAEYRGTAFGSDDAGGKLTYTIDFTKKQGNGKIEHLKSPELNVELASAEIKADGKSHAVILGDVRYGSEEKGS
YSLGIFGGRAQEVAGSAEVKTVNGIRHIGLAAKQ
***SEQ ID NO: 113*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTAFNQLPDGKAEYRGTAFGSDDAGGKLTY
TIDFTKKQGNGKIEHLKSPELNVELASAEIKADGKSHAVILGDVRYGSEEKGSYSLGIFGGRAQEVAGSAEVKTVNGIRH
IGLAAKQGSGGGGVAADIGAGLADALTAPLDHKDKGLQSLTLDQSVRKNEKLKLAAQGAEKTYGNGDSLNTGKLKNDKVS
RFDFIRQIEVDGQLITLESGEFQVYKQSHSALTAFQTEQIQDSEHSGKMVAKRQFRIGDLGGEHTAFNQLPDGKAEYRGT
AFGSDDAGGKLTYTIDFTKKQGNGKIEHLKSPELNVELASAEIKADGKSHAVILGDVRYGSEEKGSYSLGIFGGRAQEVA
GSAEVKTVNGIRHIGLAAKQGSGGGGVAADIGAGLADALTAPLDHKDKGLQSLTLDQSVRKNEKLKLAAQGAEKTYGNGD
SLNTGKLKNDKVSRFDFIRQIEVDGQLITLESGEFQVYKQSHSALTAFQTEQIQDSEHSGKMVAKRQFRIGDLGGEHTAF
NQLPDGKAEYRGTAFGSDDAGGKLTYTIDFTKKQGNGKIEHLKSPELNVELASAEIKADGKSHAVILGDVRYGSEEKGSY
SLGIFGGRAQEVAGSAEVKTVNGIRHIGLAAKQ
***SEQ ID NO: 114*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTAFNQLPDGKAEYRGTAFGSDDAGGKLTY
TIDFTKKQGNGKIEHLKSPELNVELASAEIKADGKSHAVILGDVRYGSEEKGSYSLGIFGGRAQEVAGSAEVKTVNGIRH
 
IGLAAKQGSGGGVAADIGAGLADALTAPLDHKDKGLQSLTLDQSVRKNEKLKLAAQGAEKTYGNGDSLNTGKLKNDKVS
RFDFIRQIEVDGQLITLESGEFQVYKQSHSALTAFQTEQIQDSEHSGKMVAKRQFRIGDLGGEHTAFNQLPDGKAEYRGT
AFGSDDAGGKLTYTIDFTKKQGNGKIEHLKSPELNVELASAEIKADGKSHAVILGDVRYGSEEKGSYSLGIFGGRAQEVA
GSAEVKTVNGIRHIGLAAKQGSGGGVAADIGAGLADALTAPLDHKDKGLQSLTLDQSVRKNEKLKLAAQGAEKTYGNGD
SLNTGKLKNDKVSRFDFIRQIEVDGQLITLESGEFQVYKQSHSALTAFQTEQIQDSEHSGKMVAKRQFRIGDIAGEHTSF
DKLPEGGRATYRGTAFGSDDAGGKLTYTIDFAAKQGNGKIEHLKSPELNVDLAAADIKPDGKRHAVISGSVLYNQAEKGS
YSLGIFGGKAQEVAGSAEVKTVNGIRHIGLAAKQ
***SEQ ID NO: 115*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTAFNQLPDGKAEYRGTAFGSDDAGGKLTY
TIDFTKKQGNGKIEHLKSPELNVELASAEIKADGKSHAVILGDVRYGSEEKGSYSLGIFGGRAQEVAGSAEVKTVNGIRH
IGLAAKQGSGGGGVAADIGAGLADALTAPLDHKDKGLQSLTLDQSVRKNEKLKLAAQGAEKTYGNGDSLNTGKLKNDKVS
RFDFIRQIEVDGQLITLESGEFQVYKQSHSALTAFQTEQIQDSEHSGKMVAKRQFRIGDIAGEHTSFDKLPEGGRATYRG
TAFGSDDAGGKLTYTIDFAAKQGNGKIEHLKSPELNVDLAAADIKPDGKRHAVISGSVLYNQAEKGSYSLGIFGGKAQEV
AGSAEVKTVNGIRHIGLAAKQGSGGGGVAADIGAGLADALTAPLDHKDKGLQSLTLDQSVRKNEKLKLAAQGAEKTYGNG
DSLNTGKLKNDKVSRFDFIRQIEVDGQLITLESGEFQVYKQSHSALTAFQTEQIQDSEHSGKMVAKRQFRIGDLGGEHTA
FNQLPDGKAEYRGTAFGSDDAGGKLTYTIDFTKKQGNGKIEHLKSPELNVELASAEIKADGKSHAVILGDVRYGSEEKGS
YSLGIFGGRAQEVAGSAEVKTVNGIRHIGLAAKQ
***SEQ ID NO: 116*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTAFNQLPDGKAEYRGTAFGSDDAGGKLTY
TIDFTKKQGNGKIEHLKSPELNVELASAEIKADGKSHAVILGDVRYGSEEKGSYSLGIFGGRAQEVAGSAEVKTVNGIRH
IGLAAKQGSGGGVAADIGAGLADALTAPLDHKDKGLQSLTLDQSVRKNEKLKLAAQGAEKTYGNGDSLNTGKLKNDKVS
RFDFIRQIEVDGQLITLESGEFQVYKQSHSALTAFQTEQIQDSEHSGKMVAKRQFRIGDIAGEHTSFDKLPEGGRATYRG
TAFGSDDAGGKLTYTIDFAAKQGNGKIEHLKSPELNVDLAAADIKPDGKRHAVISGSVLYNQAEKGSYSLGIFGGKAQEV
AGSAEVKTVNGIRHIGLAAKQGSGGGVAADIGAGLADALTAPLDHKDKGLQSLTLDQSVRKNEKLKLAAQGAEKTYGNG
DSLNTGKLKNDKVSRFDFIRQIEVDGQLITLESGEFQVYKQSHSALTAFQTEQIQDSEHSGKMVAKRQFRIGDIAGEHTS
FDKLPEGGRATYHGKAFGSDDPNGRLHYTIDFAAKQGYGRIEHLKTPEQNVDLAAADIKPDGKRHAVISGSVLYNQAEKG
SYSLGIFGGKAQEVAGSAEVKIGEGIRHIGLAAKQ
***SEQ ID NO: 117*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTAFNQLPDGKAEYRGTAFGSDDAGGKLTY
TIDFTKKQGNGKIEHLKSPELNVELASAEIKADGKSHAVILGDVRYGSEEKGSYSLGIFGGRAQEVAGSAEVKTVNGIRH
IGLAAKQGSGGGGVAADIGAGLADALTAPLDHKDKGLQSLTLDQSVRKNEKLKLAAQGAEKTYGNGDSLNTGKLKNDKVS
RFDFIRQIEVDGQLITLESGEFQVYKQSHSALTAFQTEQIQDSEHSGKMVAKRQFRIGDLGGEHTAFNQLPDGKAEYRGT
AFGSDDAGGKLTYTIDFTKKQGNGKIEHLKSPELNVELASAEIKADGKSHAVILGDVRYGSEEKGSYSLGIFGGRAQEVA
GSAEVKTVNGIRHIGLAAKQGSGGGGVAADIGAGLADALTAPLDHKDKGLQSLTLDQSVRKNEKLKLAAQGAEKTYGNGD
SLNTGKLKNDKVSRFDFIRQIEVDGQLITLESGEFQVYKQSHSALTAFQTEQIQDSEHSGKMVAKRQFRIGDIAGEHTSF
DKLPEGGRATYHGKAFGSDDPNGRLHYTIDFAAKQGYGRIEHLKTPEQNVDLAAADIKPDGKRHAVISGSVLYNQAEKGS
YSLGIFGGKAQEVAGSAEVKIGEGIRHIGLAAKQ
***SEQ ID NO: 118*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTAFNQLPDGKAEYRGTAFGSDDAGGKLTY
TIDFTKKQGNGKIEHLKSPELNVELASAEIKADGKSHAVILGDVRYGSEEKGSYSLGIFGGRAQEVAGSAEVKTVNGIRH
IGLAAKQGSGGGGVAADIGAGLADALTAPLDHKDKGLQSLTLDQSVRKNEKLKLAAQGAEKTYGNGDSLNTGKLKNDKVS
RFDFIRQIEVDGQLITLESGEFQVYKQSHSALTAFQTEQIQDSEHSGKMVAKRQFRIGDIAGEHTSFDKLPEGGRATYHG
KAFGSDDPNGRLHYTIDFAAKQGYGRIEHLKTPEQNVDLAAADIKPDGKRHAVISGSVLYNQAEKGSYSLGIFGGKAQEV
AGSAEVKIGEGIRHIGLAAKQGSGGGGVAADIGAGLADALTAPLDHKDKGLQSLTLDQSVRKNEKLKLAAQGAEKTYGNG
DSLNTGKLKNDKVSRFDFIRQIEVDGQLITLESGEFQVYKQSHSALTAFQTEQIQDSEHSGKMVAKRQFRIGDIAGEHTS
FDKLPEGGRATYHGKAFGSDDPNGRLHYTIDFAAKQGYGRIEHLKTPEQNVDLAAADIKPDGKRHAVISGSVLYNQAEKG
SYSLGIFGGKAQEVAGSAEVKIGEGIRHIGLAAKQ
***SEQ ID NO: 119*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYRGTAFGSDDAGGKLT
YTIDFAAKQGNGKIEHLKSPELNVDLAAADIKPDGKRHAVISGSVLYNQAEKGSYSLGIFGGKAQEVAGSAEVKTVNGIR
HIGLAAKQGSGGGGVAADIGAGLADALTAPLDHKDKGLQSLTLDQSVRKNEKLKLAAQGAEKTYGNGDSLNTGKLKNDKV
SRFDFIRQIEVDGQLITLESGEFQVYKQSHSALTAFQTEQIQDSEHSGKMVAKRQFRIGDLGGEHTAFNQLPDGKAEYRG
TAFGSDDAGGKLTYTIDFTKKQGNGKIEHLKSPELNVELASAEIKADGKSHAVILGDVRYGSEEKGSYSLGIFGGRAQEV
AGSAEVKTVNGIRHIGLAAKQGSGGGGVAADIGAGLADALTAPLDHKDKGLQSLTLDQSVRKNEKLKLAAQGAEKTYGNG
 
DSLNTGKLKNDKVSRFDFIRQIEVDGQLITLESGEFQVYKQSHSALTAFQTEQIQDSEHSGKMVAKRQFRIGDIAGEHTS
FDKLPEGGRATYHGKAFGSDDPNGRLHYTIDFAAKQGYGRIEHLKTPEQNVDLAAADIKPDGKRHAVISGSVLYNQAEKG
SYSLGIFGGKAQEVAGSAEVKIGEGIRHIGLAAKQ
***SEQ ID NO: 120*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYRGTAFGSDDAGGKLT
YTIDFAAKQGNGKIEHLKSPELNVDLAAADIKPDGKRHAVISGSVLYNQAEKGSYSLGIFGGKAQEVAGSAEVKTVNGIR
HIGLAAKQGSGGGGVAADIGAGLADALTAPLDHKDKGLQSLTLDQSVRKNEKLKLAAQGAEKTYGNGDSLNTGKLKNDKV
SRFDFIRQIEVDGQLITLESGEFQVYKQSHSALTAFQTEQIQDSEHSGKMVAKRQFRIGDIAGEHTSFDKLPEGGRATYH
GKAFGSDDPNGRLHYTIDFAAKQGYGRIEHLKTPEQNVDLAAADIKPDGKRHAVISGSVLYNQAEKGSYSLGIFGGKAQE
VAGSAEVKIGEGIRHIGLAAKQGSGGGGVAADIGAGLADALTAPLDHKDKGLQSLTLDQSVRKNEKLKLAAQGAEKTYGN
GDSLNTGKLKNDKVSRFDFIRQIEVDGQLITLESGEFQVYKQSHSALTAFQTEQIQDSEHSGKMVAKRQFRIGDLGGEHT
AFNQLPDGKAEYRGTAFGSDDAGGKLTYTIDFTKKQGNGKIEHLKSPELNVELASAEIKADGKSHAVILGDVRYGSEEKG
SYSLGIFGGRAQEVAGSAEVKTVNGIRHIGLAAKQ
***SEQ ID NO: 121*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYRGTAFGSDDAGGKLT
YTIDFAAKQGNGKIEHLKSPELNVDLAAADIKPDGKRHAVISGSVLYNQAEKGSYSLGIFGGKAQEVAGSAEVKTVNGIR
HIGLAAKQGSGGGGVAADIGAGLADALTAPLDHKDKGLQSLTLDQSVRKNEKLKLAAQGAEKTYGNGDSLNTGKLKNDKV
SRFDFIRQIEVDGQLITLESGEFQVYKQSHSALTAFQTEQIQDSEHSGKMVAKRQFRIGDLGGEHTAFNQLPDGKAEYRG
TAFGSDDAGGKLTYTIDFTKKQGNGKIEHLKSPELNVELASAEIKADGKSHAVILGDVRYGSEEKGSYSLGIFGGRAQEV
AGSAEVKTVNGIRHIGLAAKQGSGGGGVAADIGAGLADALTAPLDHKDKGLQSLTLDQSVRKNEKLKLAAQGAEKTYGNG
DSLNTGKLKNDKVSRFDFIRQIEVDGQLITLESGEFQVYKQSHSALTAFQTEQIQDSEHSGKMVAKRQFRIGDLGGEHTA
FNQLPDGKAEYRGTAFGSDDAGGKLTYTIDFTKKQGNGKIEHLKSPELNVELASAEIKADGKSHAVILGDVRYGSEEKGS
YSLGIFGGRAQEVAGSAEVKTVNGIRHIGLAAKQ
***SEQ ID NO: 122*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYRGTAFGSDDAGGKLT
YTIDFAAKQGNGKIEHLKSPELNVDLAAADIKPDGKRHAVISGSVLYNQAEKGSYSLGIFGGKAQEVAGSAEVKTVNGIR
HIGLAAKQGSGGGGVAADIGAGLADALTAPLDHKDKGLQSLTLDQSVRKNEKLKLAAQGAEKTYGNGDSLNTGKLKNDKV
SRFDFIRQIEVDGQLITLESGEFQVYKQSHSALTAFQTEQIQDSEHSGKMVAKRQFRIGDIAGEHTSFDKLPEGGRATYH
GKAFGSDDPNGRLHYTIDFAAKQGYGRIEHLKTPEQNVDLAAADIKPDGKRHAVISGSVLYNQAEKGSYSLGIFGGKAQE
VAGSAEVKIGEGIRHIGLAAKQGSGGGGVAADIGAGLADALTAPLDHKDKGLQSLTLDQSVRKNEKLKLAAQGAEKTYGN
GDSLNTGKLKNDKVSRFDFIRQIEVDGQLITLESGEFQVYKQSHSALTAFQTEQIQDSEHSGKMVAKRQFRIGDIAGEHT
SFDKLPEGGRATYHGKAFGSDDPNGRLHYTIDFAAKQGYGRIEHLKTPEQNVDLAAADIKPDGKRHAVISGSVLYNQAEK
GSYSLGIFGGKAQEVAGSAEVKIGEGIRHIGLAAKQ
***SEQ ID NO: 123*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTSFDKLPEGGRATYRGTAFGSDDAGGKLT
YTIDFAAKQGNGKIEHLKSPELNVDLAAAEIKADEKSHAVILGDVRYNQAEKGTYSLGIFGGKAQEVAGSAEVKTVNGIR
HIGLAAKQGSGGGGVAADIGAGLADALTAPLDHKDKGLQSLTLDQSVRKNEKLKLAAQGAEKTYGNGDSLNTGKLKNDKV
SRFDFIRQIEVDGQLITLESGEFQVYKQSHSALTAFQTEQIQDSEHSGKMVAKRQFRIGDLGGEHTSFDKLPEGGRATYR
GTAFGSDDAGGKLTYTIDFAAKQGNGKIEHLKSPELNVDLAAAEIKADEKSHAVILGDVRYNQAEKGTYSLGIFGGKAQE
VAGSAEVKTVNGIRHIGLAAKQGSGGGGVAADIGAGLADALTAPLDHKDKGLQSLTLDQSVRKNEKLKLAAQGAEKTYGN
GDSLNTGKLKNDKVSRFDFIRQIEVDGQLITLESGEFQVYKQSHSALTAFQTEQIQDSEHSGKMVAKRQFRIGDLGGEHT
SFDKLPEGGRATYRGTAFGSDDAGGKLTYTIDFAAKQGNGKIEHLKSPELNVDLAAAEIKADEKSHAVILGDVRYNQAEK
GTYSLGIFGGKAQEVAGSAEVKTVNGIRHIGLAAKQ
***SEQ ID NO: 124*** 
MVSAVIGSAAVGAKSAVDRRTTGAQTDDNVMALRIETTARSYLRQNNQTKGYTPQISVVGYDRHLLLLGQVATEGEKQFV
GQIARSEQAAEGVYNYITVASLPRTAGDIAGDTWNTSKVRATLLGISPATRARVKIVTYGNVTYVMGILTPEEQAQITQK
VSTTVGVQKVITLYQNYVQRGSGGGVAADIGAGLADALTAPLDHKDKGLQSLTLDQSVRKNEKLKLAAQGAEKTYGNGD
SLNTGKLKNDKVSRFDFIRQIEVDGQLITLESGEFQVYKQSHSALTAFQTEQIQDSEHSGKMVAKRQFRIGDIAGEHTSF
DKLPEGGRATYHGKAFGSDDPNGRLHYTIDFAAKQGYGRIEHLKTPEQNVDLAAADIKPDGKRHAVISGSVLYNQAEKGS
YSLGIFGGKAQEVAGSAEVKIGEGIRHIGLAAKQGSGGGVAADIGAGLADALTAPLDHKDKGLQSLTLDQSVRKNEKLK
LAAQGAEKTYGNGDSLNTGKLKNDKVSRFDFIRQIEVDGQLITLESGEFQVYKQSHSALTAFQTEQIQDSEHSGKMVAKR
QFRIGDLGGEHTAFNQLPDGKAEYRGTAFGSDDAGGKLTYTIDFTKKQGNGKIEHLKSPELNVELASAEIKADGKSHAVI
LGDVRYGSEEKGSYSLGIFGGRAQEVAGSAEVKTVNGIRHIGLAAKQ
***SEQ ID NO: 125*** 
MVSAVIGSAAVGAKSAVDRRTTGAQTDDNVMALRIETTARSYLRQNNQTKGYTPQISVVGYDRHLLLLGQVATEGEKQFV
GQIARSEQAAEGVYNYITVASLPRTAGDIAGDTWNTSKVRATLLGISPATRARVKIVTYGNVTYVMGILTPEEQAQITQK
VSTTVGVQKVITLYQNYVQRGSGGGGVAADIGAGLADALTAPLDHKDKGLQSLTLDQSVRKNEKLKLAAQGAEKTYGNGD
SLNTGKLKNDKVSRFDFIRQIEVDGQLITLESGEFQVYKQSHSALTAFQTEQIQDSEHSGKMVAKRQFRIGDLGGEHTAF
NQLPDGKAEYRGTAFGSDDAGGKLTYTIDFTKKQGNGKIEHLKSPELNVELASAEIKADGKSHAVILGDVRYGSEEKGSY
SLGIFGGRAQEVAGSAEVKTVNGIRHIGLAAKQGSGGGGVAADIGAGLADALTAPLDHKDKGLQSLTLDQSVRKNEKLKL
AAQGAEKTYGNGDSLNTGKLKNDKVSRFDFIRQIEVDGQLITLESGEFQVYKQSHSALTAFQTEQIQDSEHSGKMVAKRQ
FRIGDIAGEHTSFDKLPEGGRATYHGKAFGSDDPNGRLHYTIDFAAKQGYGRIEHLKTPEQNVDLAAADIKPDGKRHAVI
SGSVLYNQAEKGSYSLGIFGGKAQEVAGSAEVKIGEGIRHIGLAAKQ
***SEQ ID NO: 126*** 
MVSAVIGSAAVGAKSAVDRRTTGAQTDDNVMALRIETTARSYLRQNNQTKGYTPQISVVGYDRHLLLLGQVATEGEKQFV
GQIARSEQAAEGVYNYITVASLPRTAGDIAGDTWNTSKVRATLLGISPATRARVKIVTYGNVTYVMGILTPEEQAQITQK
VSTTVGVQKVITLYQNYVQRGSGGGGVAADIGAGLADALTAPLDHKDKGLQSLTLDQSVRKNEKLKLAAQGAEKTYGNGD
SLNTGKLKNDKVSRFDFIRQIEVDGQLITLESGEFQVYKQSHSALTAFQTEQIQDSEHSGKMVAKRQFRIGDLGGEHTAF
NQLPDGKAEYRGTAFGSDDAGGKLTYTIDFTKKQGNGKIEHLKSPELNVELASAEIKADGKSHAVILGDVRYGSEEKGSY
SLGIFGGRAQEVAGSAEVKTVNGIRHIGLAAKQGSGGGGVAADIGAGLADALTAPLDHKDKGLQSLTLDQSVRKNEKLKL
AAQGAEKTYGNGDSLNTGKLKNDKVSRFDFIRQIEVDGQLITLESGEFQVYKQSHSALTAFQTEQIQDSEHSGKMVAKRQ
FRIGDLGGEHTAFNQLPDGKAEYRGTAFGSDDAGGKLTYTIDFTKKQGNGKIEHLKSPELNVELASAEIKADGKSHAVIL
GDVRYGSEEKGSYSLGIFGGRAQEVAGSAEVKTVNGIRHIGLAAKQ
***SEQ ID NO: 127*** 
MVSAVIGSAAVGAKSAVDRRTTGAQTDDNVMALRIETTARSYLRQNNQTKGYTPQISVVGYDRHLLLLGQVATEGEKQFV
GQIARSEQAAEGVYNYITVASLPRTAGDIAGDTWNTSKVRATLLGISPATRARVKIVTYGNVTYVMGILTPEEQAQITQK
VSTTVGVQKVITLYQNYVQRGSGGGGVAADIGAGLADALTAPLDHKDKGLQSLTLDQSVRKNEKLKLAAQGAEKTYGNGD
SLNTGKLKNDKVSRFDFIRQIEVDGQLITLESGEFQVYKQSHSALTAFQTEQIQDSEHSGKMVAKRQFRIGDIAGEHTSF
DKLPEGGRATYHGKAFGSDDPNGRLHYTIDFAAKQGYGRIEHLKTPEQNVDLAAADIKPDGKRHAVISGSVLYNQAEKGS
YSLGIFGGKAQEVAGSAEVKIGEGIRHIGLAAKQGSGGGGVAADIGAGLADALTAPLDHKDKGLQSLTLDQSVRKNEKLK
LAAQGAEKTYGNGDSLNTGKLKNDKVSRFDFIRQIEVDGQLITLESGEFQVYKQSHSALTAFQTEQIQDSEHSGKMVAKR
QFRIGDIAGEHTSFDKLPEGGRATYHGKAFGSDDPNGRLHYTIDFAAKQGYGRIEHLKTPEQNVDLAAADIKPDGKRHAV
ISGSVLYNQAEKGSYSLGIFGGKAQEVAGSAEVKIGEGIRHIGLAAKQ
***SEQ ID NO: 128*** 
MVSAVIGSAAVGAKSAVDRRTTGAQTDDNVMALRIETTARSYLRQNNQTKGYTPQISVVGYDRHLLLLGQVATEGEKQFV
GQIARSEQAAEGVYNYITVASLPRTAGDIAGDTWNTSKVRATLLGISPATRARVKIVTYGNVTYVMGILTPEEQAQITQK
VSTTVGVQKVITLYQNYVQRGSGGGVAADIGAGLADALTAPLDHKDKGLQSLTLDQSVRKNEKLKLAAQGAEKTYGNGD
SLNTGKLKNDKVSRFDFIRQIEVDGQLITLESGEFQVYKQSHSALTAFQTEQIQDSEHSGKMVAKRQFRIGDIAGEHTSF
DKLPEGGRATYHGKAFGSDDPNGRLHYTIDFAAKQGYGRIEHLKTPEQNVDLAAADIKPDGKRHAVISGSVLYNQAEKGS
YSLGIFGGKAQEVAGSAEVKIGEGIRHIGLAAKQ
***SEQ ID NO:** 129* 
MVSAVIGSAAVGAKSAVDRRTTGAQTDDNVMALRIETTARSYLRQNNQTKGYTPQISVVGYDRHLLLLGQVATEGEKQFV
GQIARSEQAAEGVYNYITVASLPRTAGDIAGDTWNTSKVRATLLGISPATRARVKIVTYGNVTYVMGILTPEEQAQITQK
VSTTVGVQKVITLYQNYVQRGSGGGVAADIGAGLADALTAPLDHKDKGLQSLTLDQSVRKNEKLKLAAQGAEKTYGNGD
SLNTGKLKNDKVSRFDFIRQIEVDGQLITLESGEFQVYKQSHSALTAFQTEQIQDSEHSGKMVAKRQFRIGDLGGEHTAF
NQLPDGKAEYRGTAFGSDDAGGKLTYTIDFTKKQGNGKIEHLKSPELNVELASAEIKADGKSHAVILGDVRYGSEEKGSY
SLGIFGGRAQEVAGSAEVKTVNGIRHIGLAAKQ
*SEO **ID NO: 130*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYHGKAFGSDDPNGRLH
YTIDFAAKQGYGRIEHLKTPEQNVDLAAADIKPDGKRHAVISGSVLYNQAEKGSYSLGIFGGKAQEVAGSAEVKIGEGIR
HIGLAAKQGSGGGGVAADIGAGLADALTAPLDHKDKGLQSLTLDQSVRKNEKLKLAAQGAEKTYGNGDSLNTGKLKNDKV
SRFDFIRQIEVDGQLITLESGEFQVYKQSHSALTAFQTEQIQDSEHSGKMVAKRQFRIGDIAGEHTSFDKLPEGGRATYR
GTAFGSDDAGGKLTYTIDFAAKQGNGKIEHLKSPELNVDLAAADIKPDGKRHAVISGSVLYNQAEKGSYSLGIFGGKAQE
VAGSAEVKTVNGIRHIGLAAKQ
***SEQ ID NO: 131*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTAFNQLPDGKAEYRGTAFGSDDAGGKLTY
TIDFTKKQGNGKIEHLKSPELNVELASAEIKADGKSHAVILGDVRYGSEEKGSYSLGIFGGRAQEVAGSAEVKTVNGIRH
IGLAAKQGSGGGGVAADIGAGLADALTAPLDHKDKGLQSLTLDQSVRKNEKLKLAAQGAEKTYGNGDSLNTGKLKNDKVS
RFDFIRQIEVDGQLITLESGEFQVYKQSHSALTAFQTEQIQDSEHSGKMVAKRQFRIGDIAGEHTSFDKLPEGGRATYRG
 
TAFGSDDAGGKLTYTIDFAAKQGNGKIEHLKSPELNVDLAAADIKPDGKRHAVISGSVLYNQAEKGSYSLGIFGGKAQEV
AGSAEVKTVNGIRHIGLAAKQ
***SEQ ID NO: 132*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYHGKAFGSDDPNGRLH
YTIDFAAKQGYGRIEHLKTPEQNVDLAAADIKPDGKRHAVISGSVLYNQAEKGSYSLGIFGGKAQEVAGSAEVKIGEGIR
HIGLAAKQGSGGGGVAADIGAGLADALTAPLDHKDKGLQSLTLDQSVRKNEKLKLAAQGAEKTYGNGDSLNTGKLKNDKV
SRFDFIRQIEVDGQLITLESGEFQVYKQSHSALTAFQTEQIQDSEHSGKMVAKRQFRIGDIAGEHTSFDKLPEGGRATYH
GKAFGSDDPNGRLHYTIDFAAKQGYGRIEHLKTPEQNVDLAAADIKPDGKRHAVISGSVLYNQAEKGSYSLGIFGGKAQE
VAGSAEVKIGEGIRHIGLAAKQ
***SEQ ID NO: 133*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYHGKAFGSDDPNGRLH
YTIDFAAKQGYGRIEHLKTPEQNVDLAAADIKPDGKRHAVISGSVLYNQAEKGSYSLGIFGGKAQEVAGSAEVKIGEGIR
HIGLAAKQGSGGGGVAADIGAGLADALTAPLDHKDKGLQSLTLDQSVRKNEKLKLAAQGAEKTYGNGDSLNTGKLKNDKV
SRFDFIRQIEVDGQLITLESGEFQVYKQSHSALTAFQTEQIQDSEHSGKMVAKRQFRIGDLGGEHTAFNQLPDGKAEYRG
TAFGSDDAGGKLTYTIDFTKKQGNGKIEHLKSPELNVELASAEIKADGKSHAVILGDVRYGSEEKGSYSLGIFGGRAQEV
AGSAEVKTVNGIRHIGLAAKQ
***SEQ ID NO: 134*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTAFNQLPDGKAEYRGTAFGSDDAGGKLTY
TIDFTKKQGNGKIEHLKSPELNVELASAEIKADGKSHAVILGDVRYGSEEKGSYSLGIFGGRAQEVAGSAEVKTVNGIRH
IGLAAKQGSGGGVAADIGAGLADALTAPLDHKDKGLQSLTLDQSVRKNEKLKLAAQGAEKTYGNGDSLNTGKLKNDKVS
RFDFIRQIEVDGQLITLESGEFQVYKQSHSALTAFQTEQIQDSEHSGKMVAKRQFRIGDLGGEHTAFNQLPDGKAEYRGT
AFGSDDAGGKLTYTIDFTKKQGNGKIEHLKSPELNVELASAEIKADGKSHAVILGDVRYGSEEKGSYSLGIFGGRAQEVA
GSAEVKTVNGIRHIGLAAKQ
***SEQ ID NO: 135*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTAFNQLPDGKAEYRGTAFGSDDAGGKLTY
TIDFTKKQGNGKIEHLKSPELNVELASAEIKADGKSHAVILGDVRYGSEEKGSYSLGIFGGRAQEVAGSAEVKTVNGIRH
IGLAAKQGSGGGGVAADIGAGLADALTAPLDHKDKGLQSLTLDQSVRKNEKLKLAAQGAEKTYGNGDSLNTGKLKNDKVS
RFDFIRQIEVDGQLITLESGEFQVYKQSHSALTAFQTEQIQDSEHSGKMVAKRQFRIGDIAGEHTSFDKLPEGGRATYHG
KAFGSDDPNGRLHYTIDFAAKQGYGRIEHLKTPEQNVDLAAADIKPDGKRHAVISGSVLYNQAEKGSYSLGIFGGKAQEV
AGSAEVKIGEGIRHIGLAAKQ
***SEQ ID NO: 136*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYRGTAFGSDDAGGKLT
YTIDFAAKQGNGKIEHLKSPELNVDLAAADIKPDGKRHAVISGSVLYNQAEKGSYSLGIFGGKAQEVAGSAEVKTVNGIR
HIGLAAKQGSGGGGVAADIGAGLADALTAPLDHKDKGLQSLTLDQSVRKNEKLKLAAQGAEKTYGNGDSLNTGKLKNDKV
SRFDFIRQIEVDGQLITLESGEFQVYKQSHSALTAFQTEQIQDSEHSGKMVAKRQFRIGDIAGEHTSFDKLPEGGRATYH
GKAFGSDDPNGRLHYTIDFAAKQGYGRIEHLKTPEQNVDLAAADIKPDGKRHAVISGSVLYNQAEKGSYSLGIFGGKAQE
VAGSAEVKIGEGIRHIGLAAKQ
***SEQ ID NO: 137*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDIAGEHTSFDKLPEGGRATYRGTAFGSDDAGGKLT
YTIDFAAKQGNGKIEHLKSPELNVDLAAADIKPDGKRHAVISGSVLYNQAEKGSYSLGIFGGKAQEVAGSAEVKTVNGIR
HIGLAAKQGSGGGGVAADIGAGLADALTAPLDHKDKGLQSLTLDQSVRKNEKLKLAAQGAEKTYGNGDSLNTGKLKNDKV
SRFDFIRQIEVDGQLITLESGEFQVYKQSHSALTAFQTEQIQDSEHSGKMVAKRQFRIGDLGGEHTAFNQLPDGKAEYRG
TAFGSDDAGGKLTYTIDFTKKQGNGKIEHLKSPELNVELASAEIKADGKSHAVILGDVRYGSEEKGSYSLGIFGGRAQEV
AGSAEVKTVNGIRHIGLAAKQ
***SEQ ID NO: 138*** 
VAADIGAGLADALTAPLDHKDKGLQSLTLDQSVRKNEKLKLAAQGAEKTYGNGDSLNTGKLKNDKVSRFDFIRQIEVDGQ
LITLESGEFQVYKQSHSALTAFQTEQIQDSEHSGKMVAKRQFRIGDLGGEHTSFDKLPEGGRATYRGTAFGSDDAGGKLT
YTIDFAAKQGNGKIEHLKSPELNVDLAAAEIKADEKSHAVILGDVRYNQAEKGTYSLGIFGGKAQEVAGSAEVKTVNGIR
HIGLAAKQGSGGGGVAADIGAGLADALTAPLDHKDKGLQSLTLDQSVRKNEKLKLAAQGAEKTYGNGDSLNTGKLKNDKV
SRFDFIRQIEVDGQLITLESGEFQVYKQSHSALTAFQTEQIQDSEHSGKMVAKRQFRIGDLGGEHTSFDKLPEGGRATYR
GTAFGSDDAGGKLTYTIDFAAKQGNGKIEHLKSPELNVDLAAAEIKADEKSHAVILGDVRYNQAEKGTYSLGIFGGKAQE
VAGSAEVKTVNGIRHIGLAAKQ
***SEQ ID NO: 139*** 
ATNDDVKKAATVAIAAAYNNGQEINGFKAGETIYDIDEDGTITKKDATAADVEADDFKGLGLKKVVTNLTKTVNENKQN
VDAKVKAAESEIEKLTTKLADTDAALADTDAALDATTNALNKLGENITTFAEETKTNIVKIDEKLEAVADTVDKHAEAFN
DIADSLDETNTKADEAVKTANEAKQTAEETKQNVDAKVKAAETAAGKAEAAAGTANTAADKAEAVAAKVTDIKADIATNK
DNIAKKANSADVYTREESDSKFVRIDGLNATTEKLDTRLASAEKSIADHDTRLNGLDKTVSDLRKETRQGLAEQAALSGL
FQPYNVG
***SEQ ID NO: 140*** 
VSAVIGSAAVGAKSAVDRRTTGAQTDDNVMALRIETTARSYLRQNNQTKGYTPQISVVGYDRHLLLLGQVATEGEKQFVG
QIARSEQAAEGVYNYITVASLPRTAGDIAGDTWNTSKVRATLLGISPATRARVKIVTYGNVTYVMGILTPEEQAQITQKV
STTVGVQKVITLYQNYVQRGSGGGVAADIGAGLADALTAPLDHKDKGLQSLTLDQSVRKNEKLKLAAQGAEKTYGNGDS
LNTGKLKNDKVSRFDFIRQIEVDGQLITLESGEFQVYKQSHSALTAFQTEQIQDSEHSGKMVAKRQFRIGDLGGEHTAFN
QLPDGKAEYRGTAFGSDDAGGKLTYTIDFTKKQGNGKIEHLKSPELNVELASAEIKADGKSHAVILGDVRYGSEEKGSYS
LGIFGGRAQEVAGSAEVKTVNGIRHIGLAAKQGSGGGVAADIGAGLADALTAPLDHKDKGLQSLTLDQSVRKNEKLKLA
AQGAEKTYGNGDSLNTGKLKNDKVSRFDFIRQIEVDGKLITLESGEFQVYKQSHSALTALQTEQVQDSEDSGKMVAKRQF
RIGDIAGEHTSFDKLPKGGSATYRGTAFGSDDAGGKLTYTIDFAAKQGHGKIEHLKSPELNVELATAELKADEKSHAVIL
GDTRYGGEEKGTYHLALFGDRAQEIAGSATVKIREKVHEIGIAGKQ
***SEQ ID NO: 141*** 
VSAVIGSAAVGAKSAVDRRTTGAQTDDNVMALRIETTARSYLRQNNQTKGYTPQISVVGYDRHLLLLGQVATEGEKQFVG
QIARSEQAAEGVYNYITVASLPRTAGDIAGDTWNTSKVRATLLGISPATRARVKIVTYGNVTYVMGILTPEEQAQITQKV
STTVGVQKVITLYQNYVQRGSGGGVAADIGAGLADALTAPLDHKDKGLQSLTLDQSVRKNEKLKLAAQGAEKTYGNGDS
LNTGKLKNDKVSRFDFIRQIEVDGKLITLESGEFQVYKQSHSALTALQTEQVQDSEDSGKMVAKRQFRIGDIAGEHTSFD
KLPKGGSATYRGTAFGSDDAGGKLTYTIDFAAKQGHGKIEHLKSPELNVELATAELKADEKSHAVILGDTRYGGEEKGTY
HLALFGDRAQEIAGSATVKIREKVHEIGIAGKQGSGGGVAADIGAGLADALTAPLDHKDKGLQSLTLDQSVRKNEKLKL
AAQGAEKTYGNGDSLNTGKLKNDKVSRFDFIRQIEVDGQLITLESGEFQVYKQSHSALTAFQTEQIQDSEHSGKMVAKRQ
FRIGDLGGEHTAFNQLPDGKAEYRGTAFGSDDAGGKLTYTIDFTKKQGNGKIEHLKSPELNVELASAEIKADGKSHAVIL
GDVRYGSEEKGSYSLGIFGGRAQEVAGSAEVKTVNGIRHIGLAAKQ
***SEQ ID NO: 142***

VSAVIGSAAVGAKSAVDRRTTGAQTDDNVMALRIETTARSYLRQNNQTKGYTPQISVVGYDRHLLLLGQVATEGEKQFVG
QIARSEQAAEGVYNYITVASLPRTAGDIAGDTWNTSKVRATLLGISPATRARVKIVTYGNVTYVMGILTPEEQAQITQKV
STTVGVQKVITLYQNYVQRGSGGGGVAADIGAGLADALTAPLDHKDKGLQSLTLDQSVRKNEKLKLAAQGAEKTYGNGDS
LNTGKLKNDKVSRFDFIRQIEVDGKLITLESGEFQVYKQSHSALTALQTEQVQDSEDSGKMVAKRQFRIGDIAGEHTSFD
KLPKGGSATYRGTAFGSDDAGGKLTYTIDFAAKQGHGKIEHLKSPELNVELATAELKADEKSHAVILGDTRYGGEEKGTY
HLALFGDRAQEIAGSATVKIREKVHEIGIAGKQGSGGGGVAADIGAGLADALTAPLDHKDKGLQSLTLDQSVRKNEKLKL
AAQGAEKTYGNGDSLNTGKLKNDKVSRFDFIRQIEVDGKLITLESGEFQVYKQSHSALTALQTEQVQDSEDSGKMVAKRQ
FRIGDIAGEHTSFDKLPKGGSATYRGTAFGSDDAGGKLTYTIDFAAKQGHGKIEHLKSPELNVELATAELKADEKSHAVI
LGDTRYGGEEKGTYHLALFGDRAQEIAGSATVKIREKVHEIGIAGKQ

## Claims

1. A polypeptide comprising a first immunogenic amino acid sequence and a second immunogenic amino acid sequence, wherein the first immunogenic amino acid sequence is SEQ ID NO 23.

2. The polypeptide of claim 1, wherein the second immunogenic amino acid sequence is (a) a non-meningococcal antigen; (b) a meningococcal non-fHBP antigen; (c) a wild-type meningococcal fHBP antigen; or (d) an amino acid sequence selected from the group consisting of SEQ ID NOs 23, 20, 4, 5, 6, 7, 8, 9, 10, 11, 12, 13, 14, 15, 16, 17, 18, 19, 21, 22, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77 and 78.

3. The polypeptide of claim 1, comprising an amino acid sequence selected from the group consisting of SEQ ID NOs 126, 124, 125, 133, 134, 135, 112, 113, 117, 118, 104, 105, 108, 111, 114, 115, 116, 131, 137, 119, 121, 120, 109, 106, 129, 100, 101, 99, 102, 103, 107, 110, 122, 123, 127, 128, 130, 132, 136, 138, 140, 141, 142, 77 and 78.

4. A polypeptide comprising a first immunogenic amino acid sequence, a second immunogenic amino acid sequence and a third immunogenic amino acid sequence, wherein:
the first immunogenic amino acid sequence is SEQ ID NO 23;
the second immunogenic amino acid sequence is (a) a non-meningococcal antigen; (b) a meningococcal non-fHBP antigen; (c) a wild-type meningococcal fHBP antigen; or (d) an amino acid sequence selected from the group consisting of SEQ ID NOs 23, 20, 4, 5, 6, 7, 8, 9, 10, 11, 12, 13, 14, 15, 16, 17, 18, 19, 21, 22, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77 and 78; and
the third immunogenic amino acid sequence is selected from the group consisting of SEQ ID NOs 23, 20, 4, 5, 6, 7, 8, 9, 10, 11, 12, 13, 14, 15, 16, 17, 18, 19, 21, 22, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77 and 78.

5. Nucleic acid encoding the polypeptide of any preceding claim.

6. A plasmid comprising a nucleotide sequence encoding the polypeptide of any of claims 1 to 4.

7. A non-human host cell transformed with the plasmid of claim 6.

8. The host cell of claim 7, wherein the cell is a meningococcal bacterium.

9. Membrane vesicles prepared from the host cell of claim 8, wherein the vesicles include a polypeptide of any one of claims 1 to 4.

10. An immunogenic composition comprising a polypeptide of any one of claims 1 to 4 or a vesicle of claim 9.

11. The composition of claim 10, comprising a first polypeptide comprising amino acid sequence SEQ ID NO: 90, a second polypeptide comprising amino acid sequence SEQ ID NO: 139, and a third polypeptide comprising amino acid sequence SEQ ID NO: 126.

12. The composition of claim 11, including meningococcal outer membrane vesicles.

13. The composition of claim 11, which does not include meningococcal outer membrane vesicles.

14. The composition of any one of claims 10 to 13, including an adjuvant, preferably wherein the adjuvant comprises an aluminium salt.

15. The composition of any of claims 10 to 14, further comprising a second polypeptide that, when administered to a mammal, elicits an antibody response that is bactericidal against meningococcus, provided that the second polypeptide is not a meningococcal fHBP.

16. The composition of any of claims 10 to 15, further comprising a conjugated capsular saccharide from *N.meningitidis* serogroup A, C, W135 and/or Y.

17. The composition of any of claims 10 to 16, further comprising a conjugated pneumococcal capsular saccharide.

18. The immunogenic composition of any of claims 10 to 17 for use in a method for raising an antibody response in a mammal.

19. The immunogenic composition of any of claims 10 to 17 for use in a method of protecting a mammal against a Neisserial infection and/or disease.

## Patentansprüche

1. Polypeptid, das eine erste immunogene Aminosäuresequenz und eine zweite immunogene Aminosäuresequenz umfasst, wobei die erste immunogene Aminosäuresequenz SEQ ID NO: 23 ist.

2. Polypeptid gemäss Anspruch 1, wobei die zweite immunogene Aminosäuresequenz (a) ein Nicht-Meningokokken-Antigen; (b) ein Meningokokken-Nicht-fHBP-Antigen; (c) ein Meningokokken-fHBP-Antigen vom Wildtyp oder (d) eine Aminosäuresequenz, ausgewählt aus der Gruppe bestehend aus den SEQ ID NOs: 23, 20, 4, 5, 6, 7, 8, 9, 10, 11, 12, 13, 14, 15, 16, 17, 18, 19, 21, 22, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77 und 78, ist.

3. Polypeptid gemäss Anspruch 1, das eine Aminosäuresequenz, ausgewählt aus der Gruppe bestehend aus den SEQ ID NOs: 126, 124, 125, 133, 134, 135, 112, 113, 117, 118, 104, 105, 108, 111, 114, 115, 116, 131, 137, 119, 121, 120, 109, 106, 129, 100, 101, 99, 102, 103, 107, 110, 122, 123, 127, 128, 130, 132, 136, 138, 140, 141, 142, 77 and 78, umfasst.

4. Polypeptid, das eine erste immunogene Aminosäuresequenz, eine zweite immunogene Aminosäuresequenz und eine dritte immunogene Aminosäuresequenz umfasst, wobei:
die erste immunogene Aminosäuresequenz SEQ ID NO: 23 ist;
die zweite immunogene Aminosäuresequenz (a) ein Nicht-Meningokokken-Antigen; (b) ein Meningokokken-Nicht-fHBP-Antigen; (c) ein Meningokokken-fHBP-Antigen vom Wildtyp oder (d) eine Aminosäuresequenz, ausgewählt aus der Gruppe bestehend aus den SEQ ID NOs: 23, 20, 4, 5, 6, 7, 8, 9, 10, 11, 12, 13, 14, 15, 16, 17, 18, 19, 21, 22, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77 und 78, ist; und
die dritte immunogene Aminosäuresequenz aus der Gruppe bestehend aus den SEQ ID NOs: 23, 20, 4, 5, 6, 7, 8, 9, 10, 11, 12, 13, 14, 15, 16, 17, 18, 19, 21, 22, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77 und 78 ausgewählt ist.

5. Nukleinsäure, die das Polypeptid gemäss einem der vorhergehenden Ansprüche codiert.

6. Plasmid, das eine Nukleotidsequenz umfasst, die das Polypeptid gemäss einem der Ansprüche 1 bis 4 codiert.

7. Nicht-menschliche Wirtszelle, die mit dem Plasmid gemäss Anspruch 6 transformiert ist.

8. Wirtszelle gemäss Anspruch 7, wobei die Zelle ein Meningokokken-Bakterium ist.

9. Membranvesikel, die aus der Wirtszelle gemäss Anspruch 8 hergestellt sind, wobei die Vesikel ein Polypeptid gemäss irgendeinem der Ansprüche 1 bis 4 einschliessen.

10. Immunogene Zusammensetzung, die ein Polypeptid gemäss irgendeinem der Ansprüche 1 bis 4 oder ein Vesikel gemäss Anspruch 9 umfasst.

11. Zusammensetzung gemäss Anspruch 10, umfassend ein erstes Polypeptid, das die Aminosäuresequenz SEQ ID NO: 90 umfasst, ein zweites Polypeptid, das die Aminosäuresequenz SEQ ID NO: 139 umfasst, und ein drittes Polypeptid, dass die Aminosäuresequenz SEQ ID NO: 126 umfasst.

12. Zusammensetzung gemäss Anspruch 11, die Vesikel der äusseren Meningokokken-Membran einschliesst.

13. Zusammensetzung gemäss Anspruch 11, die keine Vesikel der äusseren Meningokokken-Membran einschliesst.

14. Zusammensetzung gemäss irgendeinem der Ansprüche 10 bis 13, die ein Adjuvans einschliesst, wobei das Adjuvans vorzugsweise ein Aluminiumsalz umfasst.

15. Zusammensetzung gemäss einem der Ansprüche 10 bis 14, die ferner ein zweites Polypeptid umfasst, das, wenn es an einen Säuger verabreicht wird, eine Antikörper-Reaktion hervorruft, die gegen Meningokokkus bakterizid ist, vorausgesetzt, dass das zweite Polypeptid nicht Meningokokken-fHBP ist.

16. Zusammensetzung gemäss einem der Ansprüche 10 bis 15, die ferner ein konjugiertes Kapselsaccharid der N. Meningitidis-Serogruppe A, C, W135 und/oder Y umfasst.

17. Zusammensetzung gemäss einem der Ansprüche 10 bis 16, das ferner ein konjugiertes Pneumokokken-Kapselsaccharid umfasst.

18. Immunogene Zusammensetzung gemäss einem der Ansprüche 10 bis 17 zur Verwendung in einem Verfahren zum Auslösen einer Antikörper-Antwort in einem Säuger.

19. Immunogene Zusammensetzung gemäss einem der Ansprüche 10 bis 17 zur Verwendung in einem Verfahren zum Schutz eines Säugers gegen eine Neisseria-Infektion und/oder -Erkrankung.

## Revendications

1. Polypeptide comprenant une première séquence d'acides aminés immunogène et une deuxième séquence d'acides aminés immunogène, où la première séquence d'acides aminés immunogène est SEQ ID NO : 23.

2. Polypeptide selon la revendication 1, dans lequel la deuxième séquence d'acides aminés immunogène est (a) un antigène non méningococcique ; (b) un antigène méningococcique non-fHBP ; (c) un antigène méningococcique fHBP de type sauvage ; ou (d) une séquence d'acides aminés choisie dans le groupe constitué de SEQ ID NO : 23, 20, 4, 5, 6, 7, 8, 9, 10, 11, 12, 13, 14, 15, 16, 17, 18, 19, 21, 22, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77 et 78.

3. Polypeptide selon la revendication 1, comprenant une séquence d'acides aminés choisie dans le groupe constitué de SEQ ID NO : 126, 124, 125, 133, 134, 135, 112, 113, 117, 118, 104, 105, 108, 111, 114, 115, 116, 131, 137, 119, 121, 120, 109, 106, 129, 100, 101, 99, 102, 103, 107, 110, 122, 123, 127, 128, 130, 132, 136, 138, 140, 141, 142, 77 et 78.

4. Polypeptide comprenant une première séquence d'acides aminés immunogène, une deuxième séquence d'acides aminés immunogène et une troisième séquence d'acides aminés immunogène, où :
la première séquence d'acides aminés immunogène est SEQ ID NO : 23 ;
la deuxième séquence d'acides aminés immunogène est (a) un antigène non méningococcique ; (b) un antigène méningococcique non-fHBP ; (c) un antigène méningococcique fHBP de type sauvage ; ou (d) une séquence d'acides aminés choisie dans le groupe constitué de SEQ ID NO : 23, 20, 4, 5, 6, 7, 8, 9, 10, 11, 12, 13, 14, 15, 16, 17, 18, 19, 21, 22, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77 et 78 ; et
la troisième séquence d'acides aminés est choisie dans le groupe constitué de SEQ ID NO : 23, 20, 4, 5, 6, 7, 8, 9, 10, 11, 12, 13, 14, 15, 16, 17, 18, 19, 21, 22, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77 et 78.

5. Acide nucléique codant pour le polypeptide selon l'une quelconque des revendications précédentes.

6. Plasmide comprenant une séquence nucléotidique codant pour le polypeptide selon l'une quelconque des revendications 1 à 4.

7. Cellule hôte non humaine transformée avec le plasmide selon la revendication 6.

8. Cellule hôte selon la revendication 7, où la cellule est une bactérie méningococcique.

9. Vésicules membranaires préparées à partir de la cellule hôte selon la revendication 8, où les vésicules comprennent un polypeptide selon l'une quelconque des revendications 1 à 4.

10. Composition immunogène comprenant un polypeptide selon l'une quelconque des revendications 1 à 4 ou une vésicule selon la revendication 9.

11. Composition selon la revendication 10, comprenant un premier polypeptide comprenant la séquence d'acides aminés SEQ ID NO : 90, un deuxième polypeptide comprenant la séquence d'acides aminés SEQ ID NO : 139, et un troisième polypeptide comprenant la séquence d'acides aminés SEQ ID NO : 126.

12. Composition selon la revendication 11, comprenant des vésicules de la membrane externe méningococciques.

13. Composition selon la revendication 11, qui ne comprend pas des vésicules de la membrane externe méningococciques.

14. Composition selon l'une quelconque des revendications 10 à 13, comprenant un adjuvant, de préférence où l'adjuvant comprend un sel d'aluminium.

15. Composition selon l'une quelconque des revendications 10 à 14, comprenant en outre un deuxième polypeptide qui, lorsqu'elle est administrée à un mammifère, déclenche une réponse en anticorps qui est bactéricide contre le méningocoque, à condition que le deuxième polypeptide ne soit pas la fHBP méningococcique.

16. Composition selon l'une quelconque des revendications 10 à 15, comprenant en outre un saccharide capsulaire conjugué de *N. meningitidis* du sérogroupe A, C, W135 et/ou Y.

17. Composition selon l'une quelconque des revendications 10 à 16, comprenant en outre un saccharide capsulaire pneumococcique conjugué.

18. Composition immunogène selon l'une quelconque des revendications 10 à 17, pour une utilisation dans un procédé permettant de soulever une réponse en anticorps chez un mammifère.

19. Composition immunogène selon l'une quelconque des revendications 10 à 17, pour une utilisation dans un procédé de protection d'un mammifère contre une infection et/ou une maladie neissérienne.
